# EUROPEAN PATENT APPLICATION

(11) **EP 4 461 750 A2**
(43) Date of publication of application: **13.11.2024**
(21) Application number: 24191000.9
(22) Date of filing: 11.12.2020
(51) Int. Cl.: C07K 16/40

(54) **NOVEL INTERLEUKIN-15 (IL-15) FUSION PROTEINS AND USES THEREOF**

(30) Priority: 13.12.2019 US 201962947686 P
(62) Divisional of application: 20899197.6
(71) Applicant: Cugene Inc., Waltham MA 02452 (US)
(72) Inventor: LI, Yue-Sheng, Thousand Oaks, California, 91360 (US); RUI, Lingyun, Weston, Massachusetts, 02493 (US); XU, Jing, Waltham, Massachusetts, 02452 (US)
(74) Representative: Schlich

(57) **Abstract**

The present disclosure provides novel and improved IL-15 fusion proteins for use in the treatment of cancer and other disorders. In various embodiments, the fusion proteins of the invention have two functional domains: an IL-15/IL-15RαSushi domain (also referred to herein as an "IL-15/IL-15RαSushi complex") and an antibody domain, each of which can take different forms, and configured such that the IL-15 is fused to the C-terminal of the antibody domain and co-expressed and non-covalently complexed with IL-15RαSushi. Importantly, the fusions proteins of the present invention address several of the limitations observed with the IL-15 therapeutics evaluated to date; specifically, the fusion proteins demonstrate extended the half-life of IL-15 *in vivo,* and demonstrate optimized preclinical activity compared to rIL-15 or related cytokine therapeutics.

## Description

### Background

While cancer has been traditionally treated by chemotherapy, radiation, targeted therapies and surgery, a fifth pillar of cancer treatment, immunotherapy, has emerged over the past 10 years and revolutionized the war on cancer. The benchmark for the immunotherapy drugs has been established by the development of T cell checkpoint (CTLA-4 and PD-1/PD-L1) inhibitors. It has been demonstrated that these therapies effectively expand and reactivate the pool of tumor-specific T cells leading to objective response rates of up to 50% in patients with certain cancers.

Recently, interleukin-15 (IL-15), a member of the four α-helix bundle family of cytokines, has emerged as a candidate immunomodulator for the treatment of cancer. IL-15 binds to its specific receptor, IL-15Rα, which is expressed on antigen-presenting dendritic cells, monocytes and macrophages, and trans-activates a heterodimeric receptor complex composed of IL-15Rβ and the common cytokine receptor γ chain (γ_{c}) on the responding cells, including T and natural killer (NK) cells, to initiate signaling. IL-15 exhibits broad activity and induces the differentiation and proliferation of T, B and natural killer (NK) cells. It also enhances the cytolytic activity of CD8⁺ T cells and induces long-lasting antigen experienced CD8⁺CD44^{hi} memory T cells. IL-15 stimulates differentiation and immunoglobulin synthesis by B cells and induces maturation of dendritic cells. It does not stimulate immunosuppressive T regulatory cells (Tregs). As such, it was hypothesized that boosting IL-15 activity could enhance innate and adaptive immunity and fight tumors, making it a promising agent for anticancer therapy (Steel et al., Trends in Pharmacological Sciences, 33 (1): 35-41, 2012).

In a first-in-human phase I clinical trial of intravenous infusions of recombinant human IL-15 in patients with metastatic malignant melanoma, it was reported that IL-15 could be safely administered to patients with metastatic malignancy and that IL-15 administration markedly altered homeostasis of lymphocyte subsets in blood, with NK cells and γδ cells most dramatically affected, followed by CD8 memory T cells (Conlon et al, J Clin Oncol., 33(1), 74-82).

Despite these new advancements using IL-15 as a cancer immunotherapeutic to augment immune responses, there remain limitations to the effective use of IL-15 as a therapeutic. For example, IL-15 has a short half-life (<40 minutes) resulting in 1) low bioavailability that impedes its *in vivo* antitumor effects and 2) the requirement for administration of a high dose to achieve therapeutic relevant exposure, which results in toxicity. In addition, it is understood that IL-15 has poor expression levels in standard mammalian cell systems.

There remains a critical need to provide novel therapeutics which are both highly effective and safe for the treatment of cancer.

### Disclosure of the Invention

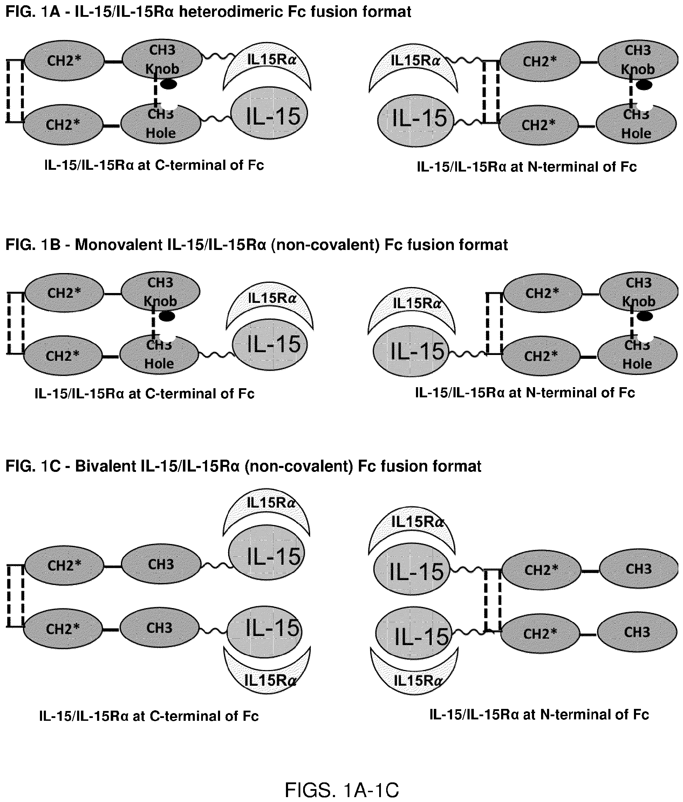

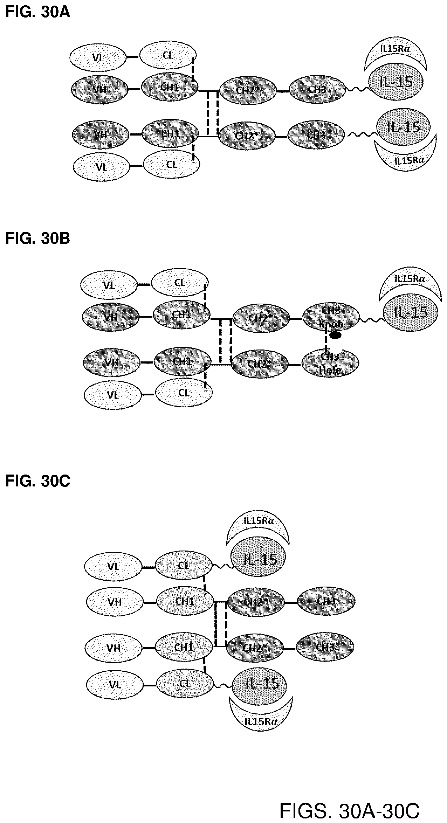

In one aspect, the present invention provides novel and improved IL-15 fusion proteins for use in the treatment of cancer. In various embodiments, the fusion proteins of the present invention have two functional domains: an IL-15/IL-15Receptor α (IL-15Rα) component (also referred to herein as an "IL-15/IL-15Rα complex") and a heterologous protein, each of which can take different forms. In various embodiments, the fusion proteins are configured such that the IL-15 is fused to either the C-terminal of the heterologous protein or to the N-terminal of the heterologous protein and co-expressed and non-covalently complexed with an IL-15Rα domain (see FIGS. 1B and 1C wherein the heterologous protein is an Fc domain, and FIGS. 30A and 30B wherein the heterologous protein is an antibody).

In various embodiments, the IL-15 fusion proteins of the present invention comprise an IL-15/IL-15Rα complex wherein the IL-15 domain comprises the sequence of the mature human IL-15 polypeptide (also referred to herein as huIL-15 or IL-15 wild type (wt)) as set forth in SEQ ID NO: 2. In various embodiments, the IL-15 domain will be an IL-15 variant (or mutant) comprising a sequence derived from the sequence of the mature human IL-15 polypeptide as set forth in SEQ ID NO: 2. Variants (or mutants) of IL-15 are referred to herein using the native amino acid, its position in the mature sequence and the variant amino acid. For example, hulL-15 "S58D" refers to human IL-15 comprising a substitution of S to D at position 58 of SEQ ID NO: 2. In various embodiments, the IL-15 variant functions as an IL-15 super-agonist as demonstrated by, e.g., increased binding activity for the IL-15Rβ and increased functional activity compared to the native IL-15 polypeptide. In various embodiments, the IL-15 variant functions as an IL-15 antagonist as demonstrated by e.g., binding activity for the IL-15Rβ but reduced or no functional activity compared to the native IL-15 polypeptide. In various embodiments, the IL-15 variant has increased binding affinity or decreased binding activity for the IL-15Rβγc receptors compared to the native IL-15 polypeptide. In various embodiments, the sequence of the IL-15 variant has at least one (i.e., 1, 2, 3, 4, 5, 6, 7, 8, 9, 10, or more) amino acid change compared to the native IL-15 sequence. The amino acid change can include one or more of amino acid substitution, deletion, or insertion in the domain of IL-15 that interacts with IL-15Rβ and/or IL-15Rγc. In various embodiments, the amino acid change is one or more amino acid substitutions at position 30, 31, 32, 58, 62, 63, 67, 68, or 108 of SEQ ID NO: 2. In various embodiments, the amino acid change is the substitution of D to T at position 30, V to Y at position 31, H to E at position 32, S to D or H or R or Q or I or P at position 58, T to D at position 62, V to F or A or R or K at position 63, I to V at position 67, I to F or H or D or K or Q or G at position 68, or Q to A or M or S or E or K at position 108 of the mature human IL-15 sequence, or any combination of these substitutions. In various embodiments, the IL-15 variant comprises 1, or 2, or 3, or 4, 5, or 6 amino acid deletion at the N-terminus of SEQ ID NO: 2. In various embodiments, the IL-15 variant comprises 1, or 2, or 3, or 4, or 5, or 6, or 7, or 8, or 9, or 10 amino acid deletion at the C-terminus of SEQ ID NO: 2. In various embodiments, the IL-2 variant comprises a `GS' (SEQ ID NO: 12), or `GGSGG' (SEQ ID NO: 153), or 'GSSGGSGGS' (SEQ ID NO: 154) amino acids insertion after position N95 of SEQ ID NO: 2. In various embodiments, the IL-15 variants comprises combination of amino acid substitution, deletion, or insertion. In various embodiments, the IL-15 variant polypeptides comprise sequences set forth in SEQ ID NOS: 24-45, 56-63, and 66-81.

In various embodiments, the IL-15 fusion proteins of the present invention comprise an IL-15/IL-15Rα complex wherein the IL-15Rα comprises either IL-15RαSushi domain (SEQ ID NO: 5) or IL-15Rα extracellular domain (SEQ ID NO: 4) or any functional domain thereof. In various embodiments, the IL-15Rα domain comprises a sequence that is at least 90% to the sequence set forth in SEQ ID NO: 4. In various embodiments the IL-15Rα domain comprises a sequence that is at least 95% to the sequence set forth in SEQ ID NO: 4. In various embodiments, the IL-15Rα domain is an IL-15RαSushi domain which comprises a sequence that is at least 90% to the sequence set forth in SEQ ID NO: 5. In various embodiments the IL-15RαSushi domain comprises a sequence that is at least 95% to the sequence set forth in SEQ ID NO: 5.

In various embodiments, the IL-15 fusion proteins of the present invention comprise an IL-15/IL-15RαSushi complex and at least one heterologous protein.

In various embodiments, the IL-15 fusion proteins of the present invention comprise an IL-15/IL-15Rα complex wherein the IL-15 is fused to either the C-terminus, or N-terminus of the heterologous protein.

In various embodiments, the IL-15 fusion proteins of the present invention contain an IL-15/IL-15Rα-heterologous protein complex either in dimeric or monomeric format.

In various embodiments, the heterologous protein is an Fc domain (or functional fragment thereof). In various embodiments, the Fc domain is selected from the group consisting of human IgG1 Fc domain, human IgG2 Fc domain, human IgG3 Fc domain, human IgG4 Fc domain, IgA Fc domain, IgD Fc domain, IgE Fc domain, IgG Fc domain and IgM Fc domain; or any combination thereof. In various embodiments, the Fc domain includes an amino acid change that results in an Fc domain having altered complement or Fc receptor binding properties. Amino acid changes to produce an Fc domain with altered complement or Fc receptor binding properties are known in the art. In various embodiments, the Fc domain sequence used to make dimeric IL-15/IL-15Rα complex-Fc fusion proteins is the human IgG1-Fc domain sequence set forth in SEQ ID NO: 6. SEQ ID NO: 6 contains amino acid substitutions that ablate FcγR and C1q binding. In various embodiments, the heterodimeric Fc domain sequence used to make monovalent IL-15/1L-15Rα complex-Fc fusion proteins is the Knob-Fc domain sequence set forth in SEQ ID NO: 7. SEQ ID NO: 7 contains amino acid substitutions that ablate FcγR and C1q binding. In various embodiments, the heterodimeric Fc domain sequence used to make monovalent IL-15/IL-15Rα complex-Fc fusion proteins is the Hole-Fc domain sequence set forth in SEQ ID NO: 8. SEQ ID NO: 8 contains amino acid substitutions that ablate FcγR and C1q binding.

In various embodiments, the IL-15 fusion proteins of the present invention comprise an IL-15/IL-15Rα complex and the heterologous protein is a full-length non-binding antibody for half-life extension or is a specific antibody or fragment used for targeting, multifunction, and half-life extension.

In various embodiments, the IL-15 fusion proteins of the present invention comprise an IL-15/IL-15Rα complex and the heterologous protein is an antibody either in full-length IgG or antibody fragment format (monospecific or bispecific) and provides additive or synergistic effect with IL-15/IL-15RαSushi complex.

In various embodiments, the IL-15 fusion proteins of the present invention comprise an IL-15/IL-15Rα complex and the heterologous protein provides tissue- or tumor-specific targeting to increase IL-15 local concentration and penetration into the tumor microenvironment and to increase tumor cell-killing efficacy and reduce systemic toxicity.

In various embodiments, the heterologous protein is an antibody, or an antibody fragment, or a ligand or its variant, or a receptor or its variant capable of binding to a tumor associated antigen (TAA) or a tissue-specific antigen or target; a cell surface molecule or extracellular matrix protein; protease(s) and any post-translational modification residue(s). In various embodiments, the antibody is an anti-Fibroblast Activation Protein (FAP) antibody or antibody fragment. In various embodiments, the antibody is a humanized anti-FAP antibody comprising the heavy chain and light chain sequence set forth in SEQ ID NOS: 109-110. In various embodiments, the heterologous protein is an antibody or an antibody fragment to an immune checkpoint modulator. In various embodiments, the antibody is an antagonistic Programmed Death-1 (PD-1) antibody or antibody fragment. In various embodiments, the antibody is an antagonistic humanized PD-1 antibody comprising the heavy chain and light chain amino acid sequences set forth in SEQ ID NOs: 111-112. In various embodiments, the antibody is an antagonistic human PD-1 antibody comprising the heavy chain and light chain amino acid sequences set forth in SEQ ID NO: 113-114. In various embodiments, the antibody is an antagonistic Programmed Death Ligand-1 (PD-L1) antibody or antibody fragment.

In various embodiments, the heterologous protein is covalently linked to IL-15 polypeptide of the IL-15/IL-15RαSushi complex by polypeptide linker sequence. In various embodiments, the linker may be an artificial sequence of between 5, 10, 15, 20, 30, 40 or more amino acids that are relatively free of secondary structure. In various embodiments, the linker is rich in G/S content (e.g., at least about 60%, 70%, 80%, 90%, or more of the amino acids in the linker are G or S). In various embodiments, the linker is selected from the group of sequences set forth in SEQ ID NOs: 9-12, 47, and 153-154. Each peptide linker sequence can be selected independently.

In another aspect, the present disclosure provides a pharmaceutical composition comprising the isolated IL-15 fusion proteins of the present invention in admixture with a pharmaceutically acceptable carrier.

In another aspect, the present disclosure provides a method for treating cancer or cancer metastasis in a subject, comprising administering a therapeutically effective amount of the pharmaceutical compositions of the invention to a subject in need thereof. In one embodiment, the subject is a human subject. In various embodiments, the cancer is selected from pancreatic cancer, gastric cancer, liver cancer, breast cancer, ovarian cancer, colorectal cancer, melanoma, leukemia, myelodysplastic syndrome, lung cancer, prostate cancer, brain cancer, bladder cancer, head-neck cancer, or rhabdomyosarcoma.

In another aspect, the present disclosure provides a method for treating cancer or cancer metastasis in a subject, comprising administering a therapeutically effective amount of the pharmaceutical compositions of the invention in combination with a second therapy selected from the group consisting of: cytotoxic chemotherapy, immunotherapy, small molecule kinase inhibitor targeted therapy, surgery, radiation therapy, stem cell transplantation, cell therapies including CAR-T cell, CAR-NK cell, iPS-induced NK cell, iPS-induced CAR-NK cell, iPS-induced T cell, iPS-induced CAR-T cell or TCR-T cell, and vaccine such as Bacille Calmette-Guerine (BCG). In various embodiments, the combination therapy may comprise administering to the subject a therapeutically effective amount of immunotherapy, including, but are not limited to, treatment using depleting antibodies to specific tumor antigens; treatment using antibody-drug conjugates; treatment using agonistic, antagonistic, or blocking antibodies to co-stimulatory or co-inhibitory molecules (immune checkpoints) such as CD276, CD272, CTLA-4, PD-1, PD-L1, CD40, SIRPα, CD47, OX-40, CD137, GITR, LAG3, ICOS, CD27, 4-1BB, TIM-3, B7-H4, Siglec-7, Siglec-8, Siglec-9, Siglec-15 and VISTA; treatment using bispecific T cell engaging antibodies (BiTE^{®}) such as blinatumomab; treatment involving administration of biological response modifiers such as IL-2, IL-7, IL-10, IL-12, IL-21, G-CSF, GM-CSF, IFN-α, IFN-β and IFN-γ; treatment using therapeutic vaccines such as sipuleucel-T; treatment using dendritic cell vaccines, or tumor antigen peptide vaccines; treatment using chimeric antigen receptor (CAR)-T cells; treatment using CAR-NK cells; treatment using tumor infiltrating lymphocytes (TILs); treatment using adoptively transferred anti-tumor T cells (ex vivo expanded and/or TCR transgenic T-cells); treatment using TALL-104 cells; and treatment using immunostimulatory agents such as Toll-like receptor (TLR) agents such as TLR4, TLR7, TLR8, TLR9 agonists CpG and imiquimod; and treatment using vaccine such as Bacille Calmette-Guerine (BCG); wherein the combination therapy provides increased effector cell killing of tumor cells, i.e., a synergy exists between the IL-15/IL-15RαSushi-Fc fusion proteins and the immunotherapy when co-administered.

In another aspect, the present disclosure provides a method to expand and renew NK cells and T cells in vitro and in vivo and in combination with any adoptive transfer NK and T cell therapy or CAR-NK and CAR-T therapy to sustain cell survival and half-life.

In another aspect, the present disclosure provides a method for treating a viral infection in a subject, comprising administering a therapeutically effective amount of the pharmaceutical compositions of the invention to a subject in need thereof. In one embodiment, the subject is a human subject.

In another aspect, the disclosure provides uses of the IL-15 fusion proteins for the preparation of a medicament for the treatment of cancer.

In another aspect, the disclosure provides uses of the IL-15 fusion proteins for the preparation of a medicament for the treatment of a viral infection.

In another aspect, the present disclosure provides isolated nucleic acid molecules comprising a polynucleotide encoding an IL-15 fusion protein of the present disclosure. In various embodiments, the isolated nucleic acid molecules comprise the polynucleotides described herein, and further comprise a polynucleotide encoding at least one heterologous protein described herein. In various embodiments, the nucleic acid molecules further comprise polynucleotides encoding the linkers described herein.

In another aspect, the present disclosure provides vectors comprising the nucleic acids described herein. In various embodiments, the vector is an expression vector. In another aspect, the present disclosure provides isolated cells comprising the nucleic acids of the disclosure. In various embodiments, the cell is a host cell comprising the expression vector of the disclosure. In another aspect, methods of making the IL-15 fusion proteins are provided by culturing the host cells under conditions promoting expression of the proteins or polypeptides.

### Brief Description of the Figures

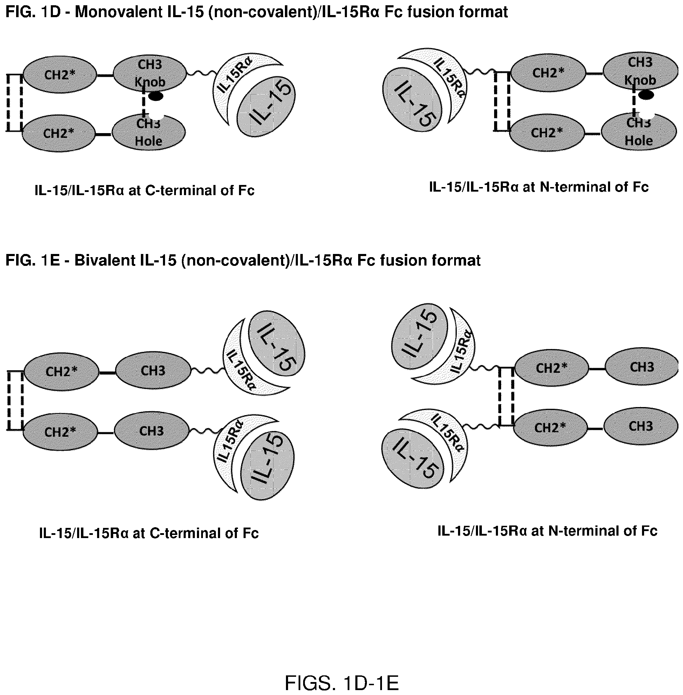

FIG. 1 depicts several formats for the IL-15/IL-15Rα-Fc fusion proteins of the present invention. (A) IL-15/IL-15Rα heterodimeric Fc fusion format. (B) Monovalent IL-15/IL-15Rα (non-covalent) Fc fusion format. (C) Bivalent IL-15/IL-15Rα (no-covalent) Fc fusion format; (D) Monovalent IL-15 (non-covalent)/ IL-15Rα Fc fusion protein format. (E) Bivalent IL-15 (non-covalent)/ IL-15Rα Fc fusion protein format. For each fusion protein format, the IL-15/IL-15Rα complex can be at either the C-terminus or N-terminus of the Fc domain; and IL-15Rα can be either IL-15RαSushi domain or IL-15RαECD.

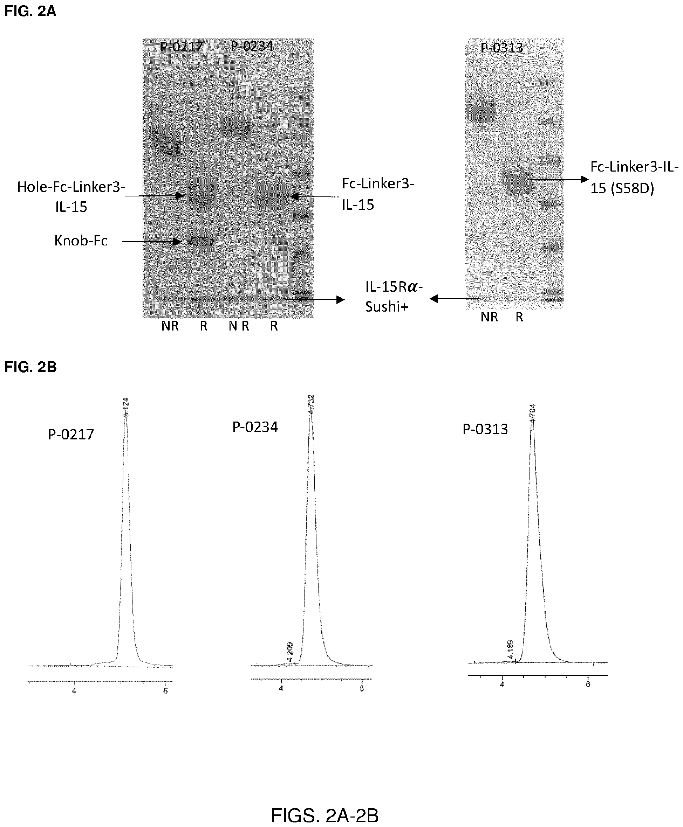

FIG. 2 depicts A) the purity and B) monomer percentage of illustrative IL-15/IL-15Rα (non-covalent)-Fc fusion proteins, P-0217, P-0234, and P-0313, as determined by SDS-PAGE and SEC-HPLC, respectively. All the three fusion proteins comprise IL-15/IL-15Rα complex at the C-terminus. P-0217 is a monovalent IL-15/IL-15Rα (non-covalent) Fc fusion, P-0234 is the dimeric counterpart of P-0217, and P-0313 shares the same fusion configuration as P-0234 but differs only with S58D substitution in the IL-15 domain.

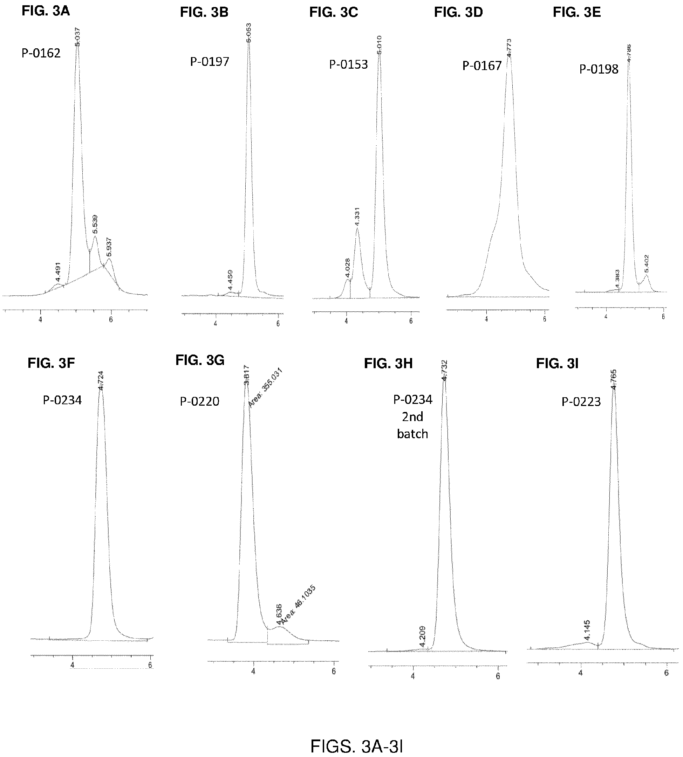

FIG. 3 depicts the SEC chromatograms of several IL-15/IL-15Rα-Fc fusion proteins of different configurations. These exemplary fusion proteins all comprise IL-15/IL-15Rα complex at the C-terminus unless otherwise stated. P-0162 is a monomeric IL-15 alone Fc fusion protein. P-0197 is a monovalent IL-15/IL-15Rα (non-covalent) Fc fusion with its schematic diagram depicted in FIG. 1B. P-0153 is a monomeric IL-15/IL-15Rα fusion with the heterodimeric Fc fusion format (FIG. 1A). P-0167 and P-0198 are the dimeric counterparts of P-0162 and P-0197, respectively. P-0234, and P-0220, and P-0223 are all bivalent IL-15/IL-15Rα (non-covalent) Fc fusion Proteins (FIG. 1C). P-0220 contains IL-15RαECD, P-0234 contains IL-15RαSushi+ domain; P-0223 differs from P-0234 with its IL-15/IL-15Rα complex attached to the N-terminus of Fc.

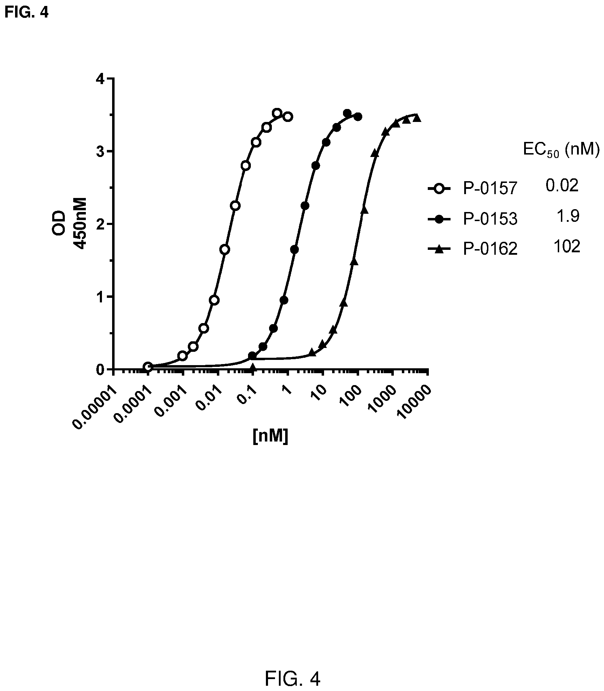

FIG. 4 depicts the effect of different IL-15/IL-15Rα Fc fusion formats on the binding activity to IL-15Rβ in ELISA assay. IL-15Rα is demonstrated to increase IL-15Rβ binding activity of IL-15 Fc fusion proteins. P-0157 is a N-terminal bivalent IL-15 (non-covalent)/IL-15RαSushi Fc fusion protein; P-0153 is a C-terminal IL-15/IL-15RαSushi heterodimeric Fc fusion protein; P-0162 is a C-terminal monovalent IL-15 Fc fusion protein without IL-15RαSushi complexed.

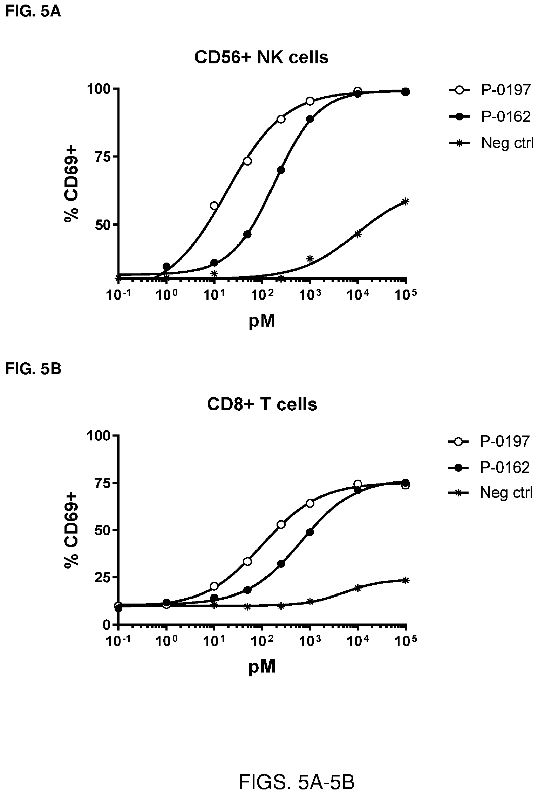

FIG. 5 depicts the effect of IL-15Rα on the biological activity of IL-15 Fc fusion proteins. IL-15Rα is demonstrated to enhance the biological activity of IL-15 Fc fusion proteins. Induction of CD69 positive NK (FIG. 5A) and CD8 T (FIG. 5B) cells was measured in an ex vivo human PBMC FACS based assay. P-0197 is a C-terminal monovalent IL-15/IL-15RαSushi (non-covalent) Fc fusion protein; P-0162 is a fusion protein with the same structure as P-0197 without IL-15RαSushi complexed.

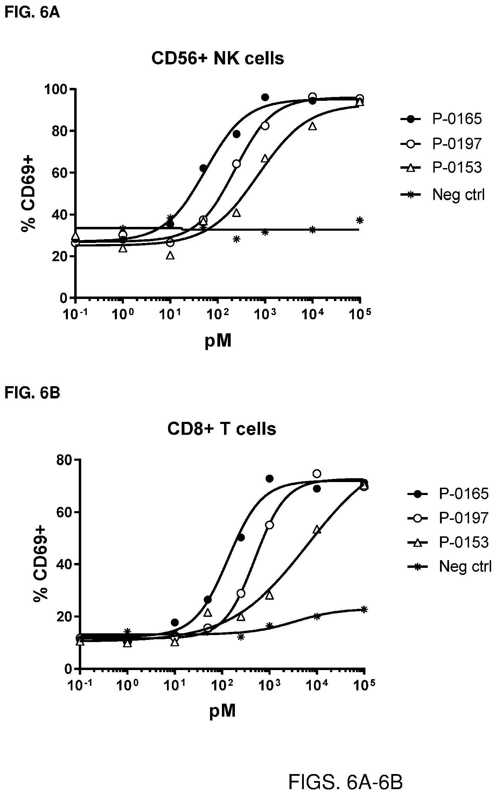

FIG. 6 depicts the effect of different configurations of IL-15/IL-15Rα complexation on the biological activity of IL-15 Fcfusion proteins. Induction of CD69 positive NK (FIG. 6A) and CD8+ T (FIG. 6B) cells was measured in an ex vivo human PBMC FACS based assay. P-0165 is a C-terminal monovalent IL-15 (non-covalent)/IL-15Rα Fc fusion protein; P-0197 is a C-terminal monovalent IL-15/IL-15Rα (non-covalent) Fc fusion protein; P-0153 is a C-terminal IL-15/1L-15Rα heterodimeric Fc fusion protein.

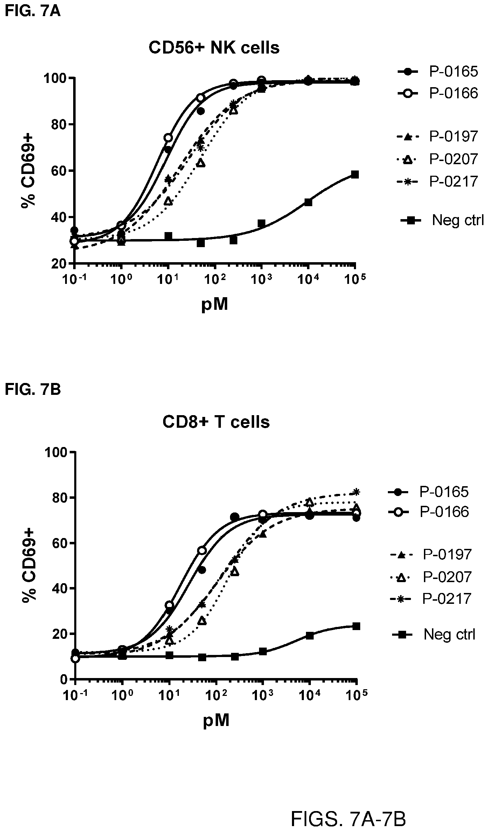

FIG. 7 depicts the effect of linkers on the biological activity of IL-15/IL-15Rα Fc fusion proteins at different formats. Induction of CD69 positive NK (FIG. 7A) and CD8 T (FIG. 7B) cells was measured in an ex vivo human PBMC FACS based assay. P-0165 & P-0166 are monovalent IL-15 (non-covalent)/IL-15Rα Fc fusions with a fifteen-amino acid rigid linker and ten-amino acid flexible linker, respectively. P-0197, P-0207, and P-0217 are monovalent IL-15/IL-15Rα (non-covalent) Fc fusion proteins with a 15 amino acid rigid, 10 amino acid flexible and 15 amino acid flexible linker, respectively.

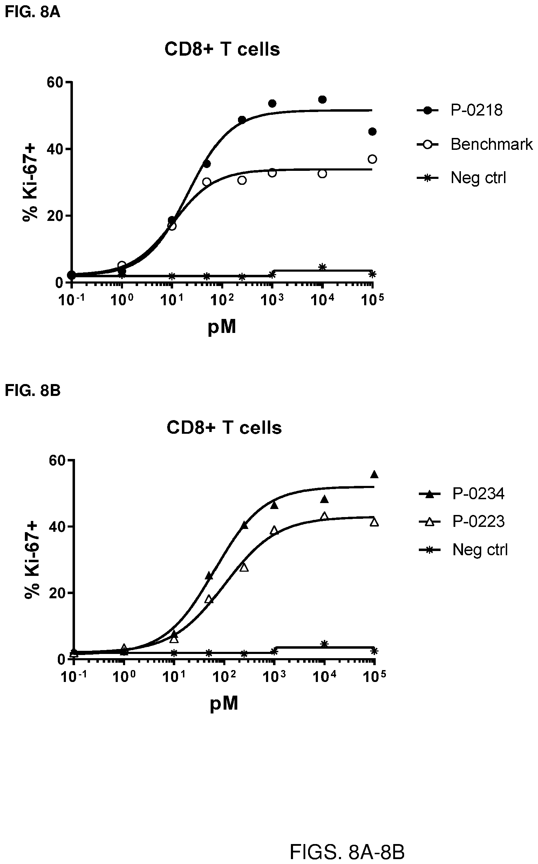

FIG. 8 depicts the effect of N- or C-terminal fusion on the activity of IL-15/IL-15Rα Fc fusion proteins. Percent Ki67 positive CD8 T cells was measured in an ex vivo human PBMC FACS based assay following the treatments. (A) P-0218 and the benchmark are the C-terminal and N-terminal bivalent IL-15 (non-covalent)/IL-15Rα Fc fusion proteins, respectively. (B) P-0234 and P-0223 are the C-terminal and N-terminal bivalent IL-15/IL-15Rα (non-covalent) Fc fusion proteins, respectively.

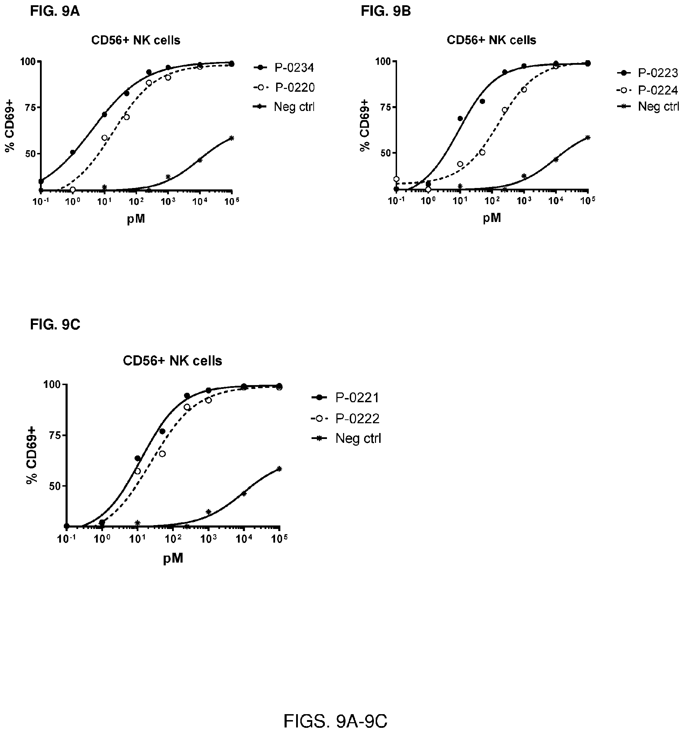

FIG. 9 depicts the effect of IL-15Rα full ECD or Sushi domain on the biological activity of IL-15/IL-15Rα Fc fusion proteins. Induction of CD69 positive NK cells was measured in an ex vivo human PBMC FACS based assay. (A) P-0234 and P-0220 are C-terminal bivalent IL-15/IL-15Rα (non-covalent) Fc fusion proteins with IL-15Rα sushi and full ECD, respectively. (B) P-0223 and P-0224 are N-terminal bivalent IL-15/IL-15Rα (non-covalent) Fcfusion proteins with IL-15Rα sushi and full ECD, respectively. (C) P-0221 and P-0222 are N-terminal monovalent IL-15/IL-15Rα (non-covalent) Fc fusion proteins with IL-15Rα sushi and full ECD, respectively.

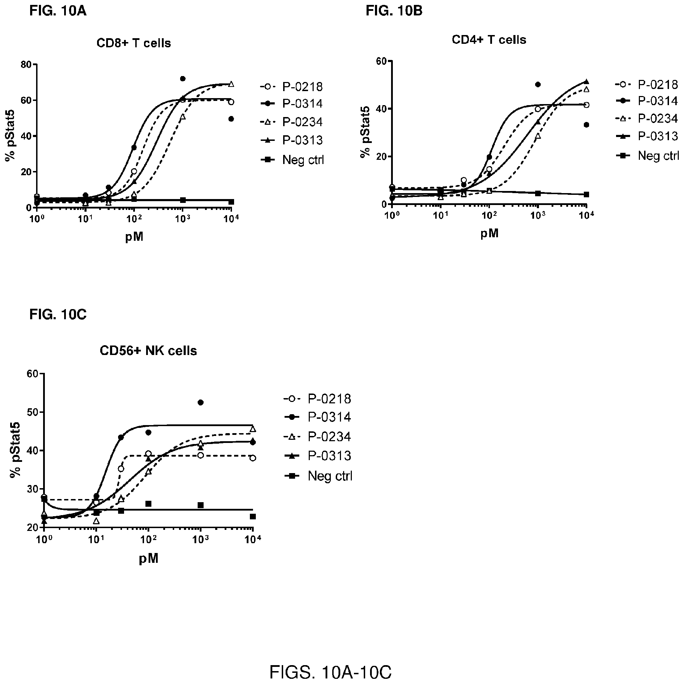

FIG. 10 depicts that the S58D substitution in the IL-15 polypeptide enhanced the ability of IL-15 fusion proteins to induce STAT5 phosphorylation on CD8+ T cells (A), CD4+ T cells (B), and NK cells (C). P-0218 and P-0314 are bivalent IL-15 (non-covalent)/IL-15Rα Fc fusion proteins, comprising IL-15 wild type and S58D variant, respectively. P-0234 and P-0313 are bivalent IL-15/IL-15Rα (non-covalent) Fc fusion proteins, comprising IL-15 wild type and S58D variant, respectively.

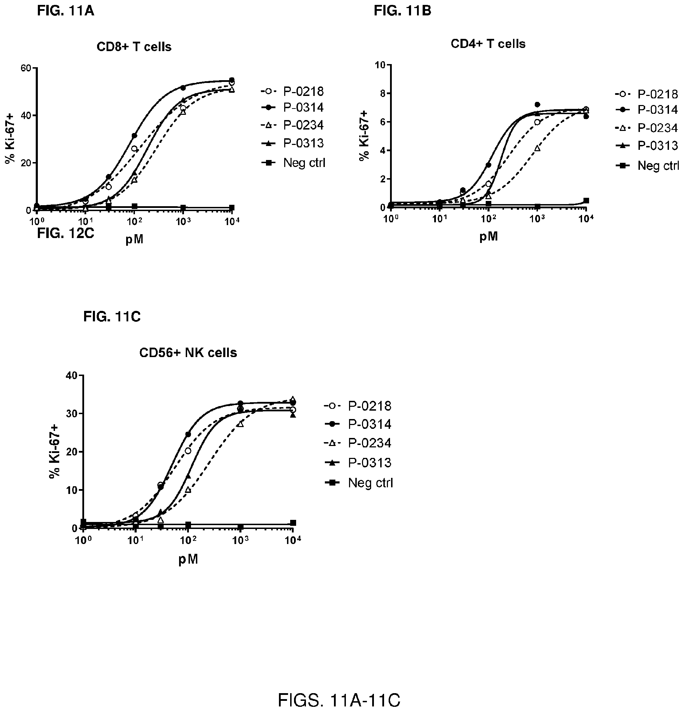

FIG. 11 depicts that IL-15 S58D variant-containing fusion proteins exhibited enhanced ability to induce Ki67 expression on CD8+ T cells (A), CD4+ T cells (B) and NK cells (C). P-0218 and P-0314 are bivalent IL-15 (non-covalent)/IL-15Rα Fc fusion proteins, comprising IL-15 wild type and S58D variant, respectively. P-0234 and P-0313 are bivalent IL-15/IL-15Rα (non-covalent) Fc fusion proteins, comprising IL-15 wild type and S58D variant, respectively.

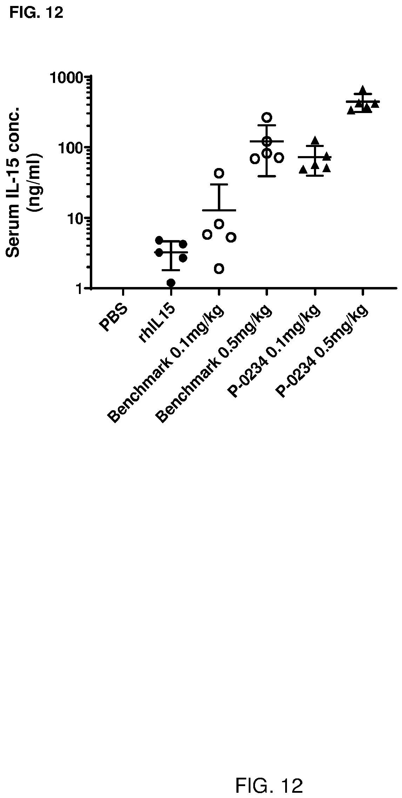

FIG. 12 depicts serum concentrations of IL-15 in mice treated with rhlL-15, the benchmark and P-0234 in a 4-day repeated dosing study. Female B Balb/C mice were intraperitoneally injected daily with vehicle, rhIL-15 (0.03 mg/kg), the benchmark (0.1 and 0.5mg/kg), or P-0234 (0.1 and 0.5 mg/kg). Terminal blood was collected one hour after the last injection on day 4 and serum IL-15 levels were measured using an ELISA assay.

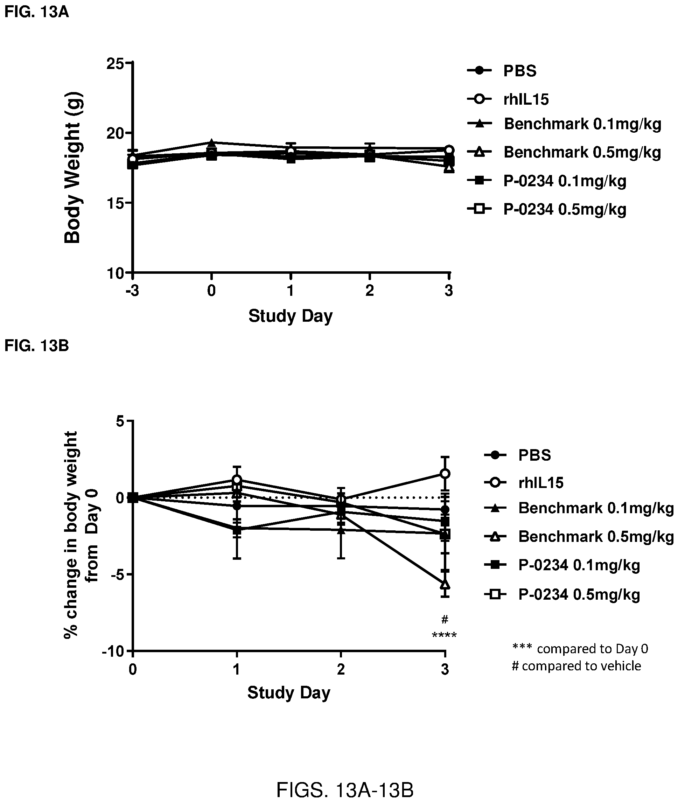

FIG. 13 depicts (A) body weight and (B) % change in body weight from Day 0 in Balb/C mice treated with rhlL-15, the benchmark and P-0234 during a 4-day repeated dosing study. Data are expressed as mean ± SEM. Statistical analysis was performed by one-way anova followed by Tukey's post hoc test. *** p<0.001 compared to Day 0; # p<0.05 compared to PBS group.

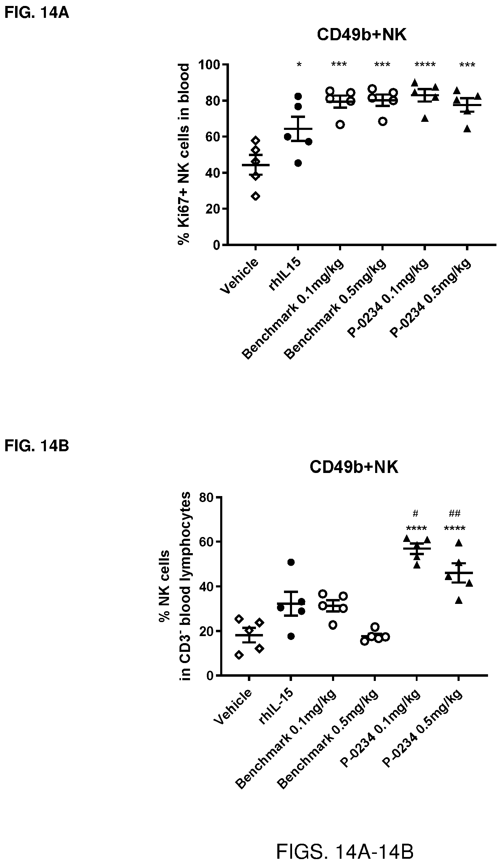

FIG. 14 depicts the effect of IL-15 compounds on the NK cell proliferation and expansion in the peripheral blood of Balb/C mice in a 4-day repeated dosing study. After 4 daily doses, blood was collected for Ki67 measurement and NK cell phenotyping by FACS. (A) Percentage of the proliferation marker Ki67 positive NK cells; (B) Percentage of NK cells in CD3 negative lymphocyte population. Data were expressed as mean ± SEM. Statistical analysis was performed by one-way anova followed by Tukey's post hoc test. **** p<0.0001 compared to vehicle group, # p<0.001 & ## p<0.01 compared to equivalent dose of the benchmark.

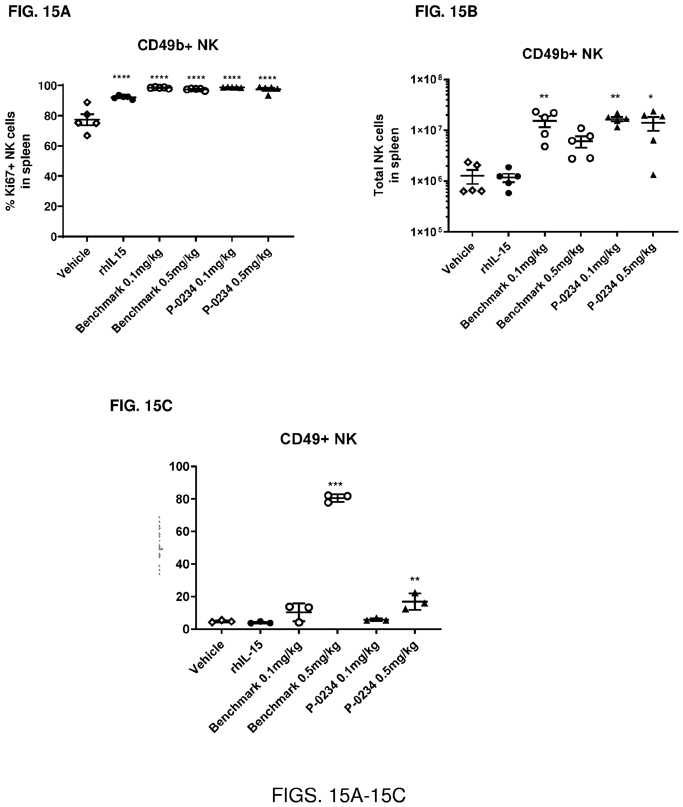

FIG. 15 depicts the effect of IL-15 compounds on the proliferation, expansion and activation of splenic NK cells of Balb/C mice in a 4-day repeated dosing study. (A) Percentage of the proliferation marker Ki67 positive splenic NK cells; (B) Total NK cells in the spleen; (C) Percentage of CD69 positive splenic NK cells. Data were expressed as mean ± SEM. Statistical analysis was performed by one-way anova followed by Tukey's post hoc test. **** p<0.0001, ** p<0.01, * p<0.05 compared to vehicle group.

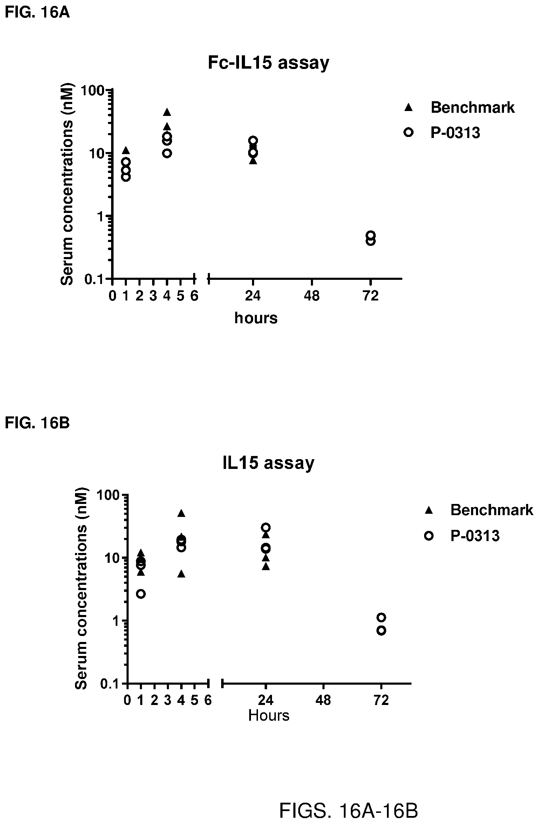

FIG. 16 depicts serum concentrations of P-0313 and the benchmark following a single intraperitoneal injection in Balb/C mice. Blood was collected from mice treated with 0.3 mg/kg P-0313 or the benchmark at -24 (pre-dose) and 1, 4, 24, 72, 144 and 192 hours after dose. (A) an in-house ELISA assay detecting human Fc-IL-15 complex; (B) A commercial ELISA assay detecting human IL-15.

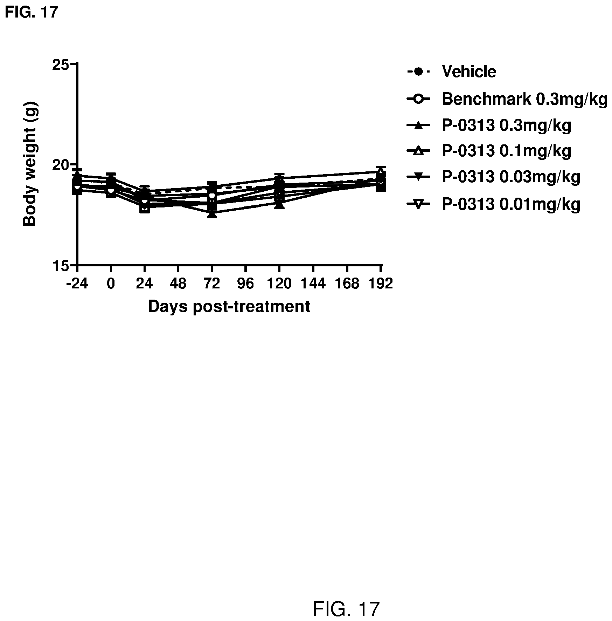

FIG. 17 depicts body weight in Balb/C mice following a single injection of P-0313 and the benchmark in a period of 8 days.

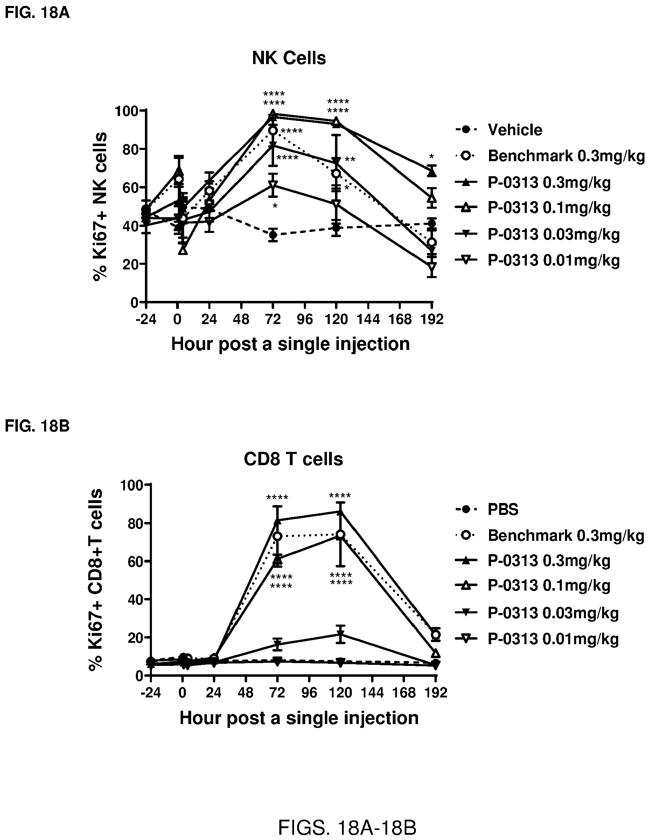

FIG. 18 depicts dose- and time-dependent effects of IL-15/IL-15Rα Fc fusion proteins on Ki67 expression on NK (A) and CD8+ T cells (B) following a single injection in Balb/C mice. Blood was collected at -24 (pre-dose), and 1, 4, 24, 72, 144 and 192 hours for lymphocyte phenotyping and Ki67 measurement by FACS analysis. Data are expressed as mean ± SEM. Statistical analysis was performed by two-way anova followed by Tukey's post hoc test. **** p<0.0001, ** p<0.01, * p<0.05 compared to PBS group at respective time point.

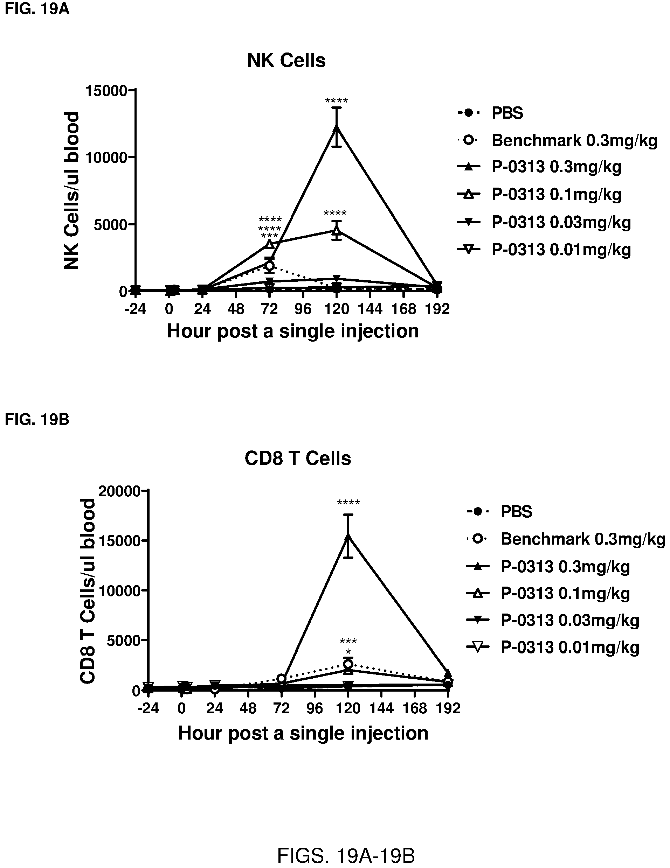

FIG. 19 depicts dose- and time-dependent effects of IL-15/IL-15Rα Fc fusion proteins on the expansion of NK (A) and CD8+ T cells (B) in peripheral blood following a single injection in Balb/C mice. Blood was collected at -24 (pre-dose), and 1, 4, 24, 72, 144 and 192 hours for lymphocyte phenotyping by FACS analysis. Data are expressed as mean ± SEM. Statistical analysis was performed by two-way anova followed by Tukey's post hoc test. **** p<0.0001, *** p<0.001, * p<0.05 compared to PBS group at respective time point.

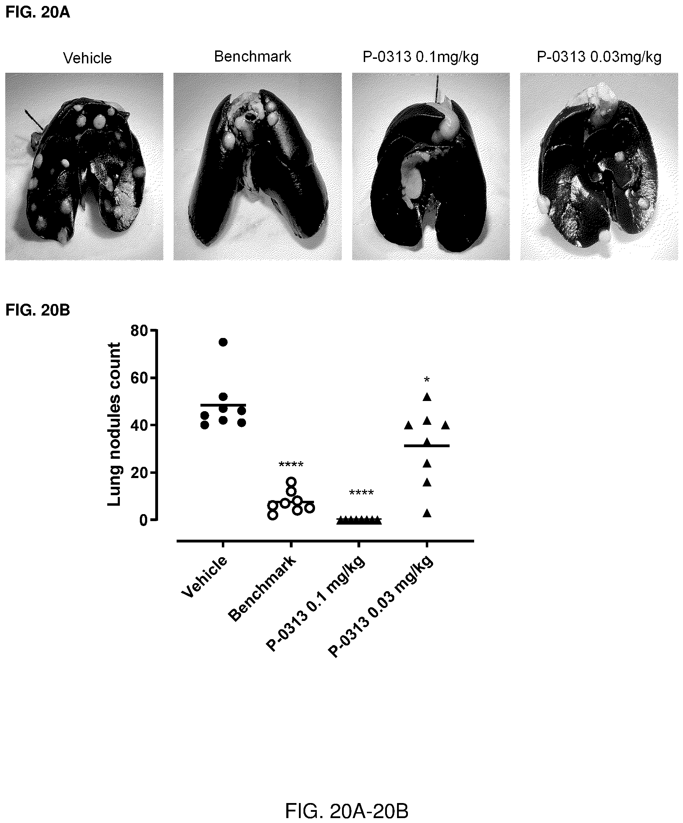

FIG. 20 depicts inhibition of lung metastasis by P-0313 and the benchmark in a mouse CT26 pulmonary metastasis model. Vehicle, the Benchmark (0.3mg/kg) or P-0313 (0.03 and 0.1 mg/kg) were given 3 × Q5D doses initiated one day after the injection of CT26 cells. Mice were sacrificed on day 16 for microscopic counting of the lung metastatic nodules. (A) Representative lung photographs obtained under light microscope illustrating metastatic nodules from each treatment group. (B) Lung nodule counts of all animals. Data are expressed as mean ± SEM. Statistical analysis was performed by one-way anova followed by Tukey's post hoc test. **** p<0.0001, * p<0.05 compared to PBS group.

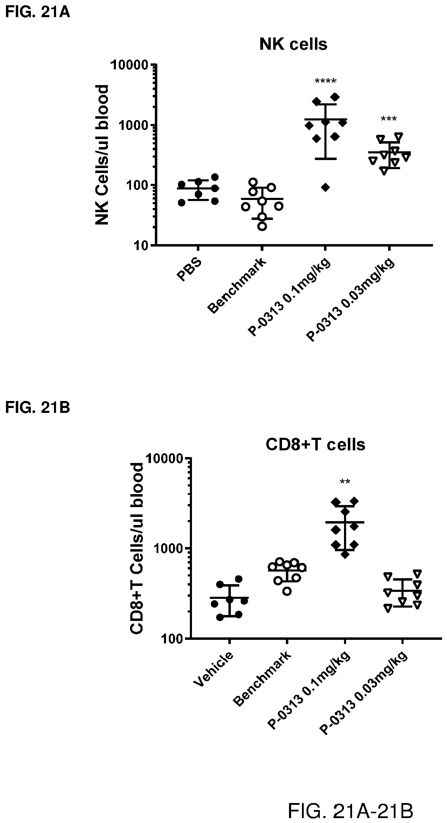

FIG. 21 depicts the immuno-pharmacodynamic profiling in CT26 pulmonary metastasis model following treatment with P-0313 or the benchmark. Increases in the number of circulating A) NK cells, and B) CD8+ T cells per µl whole blood in CT26 metastasis mice were determined by flow cytometry 3 days after three Q5D i.p. injections of P-0313, benchmark, or PBS. Data are expressed as mean ± SEM. Statistical analysis was performed by one-way anova followed by Tukey's post hoc test. **** p<0.0001, *** p<0.001, ** p<0.01 compared to PBS group.

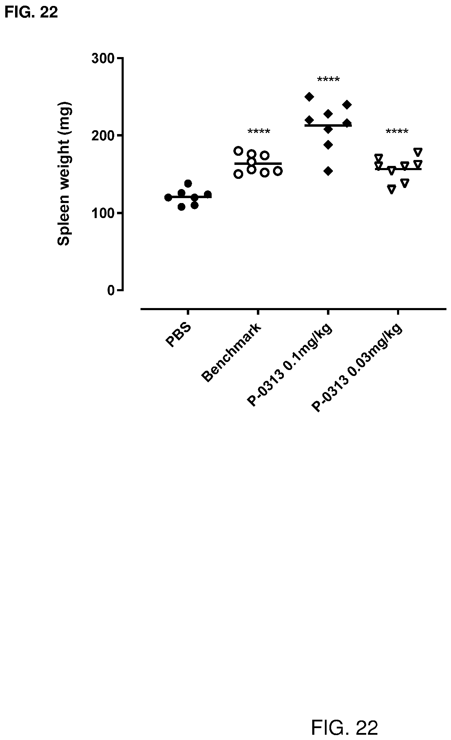

FIG. 22 depicts spleen weights in mice treated with P-0313 or the benchmark in CT26 pulmonary metastasis model. Spleen were collected 3 days after three Q5D i.p. injections of IL-15/IL-15Rα Fc fusion proteins. Data are expressed as mean ± SEM. Statistical analysis was performed by one-way anova followed by Tukey's post hoc test. **** p<0.0001 compared to PBS group.

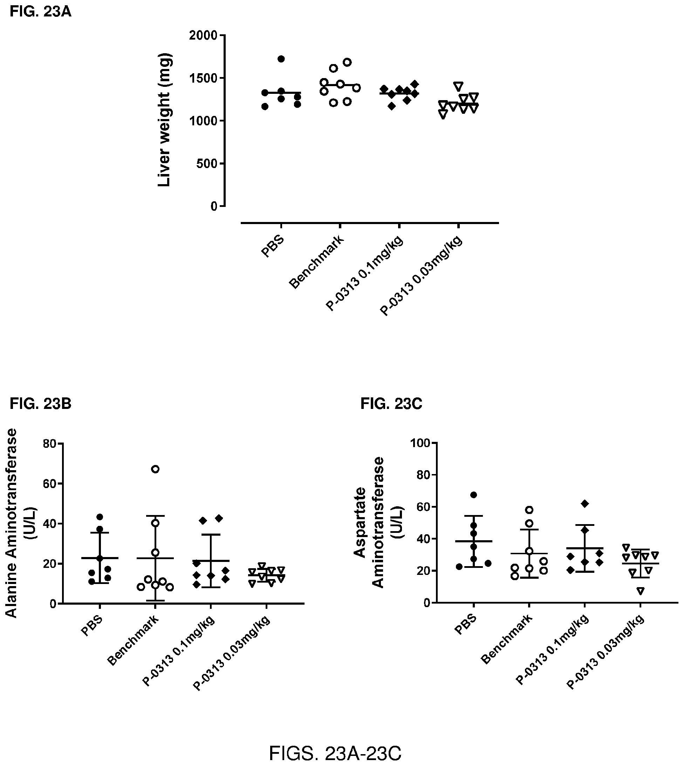

FIG. 23 depicts hepatotoxicity assessment in CT26 pulmonary metastasis mice treated with P-0313 or the benchmark. Liver were collected three days after three Q5D treatments. A) Liver weight; B) serum ALT level; and C) serum AST level. ALT and AST levels in serum were determined using commercial ELISA kit. Data are expressed as mean ± SEM.

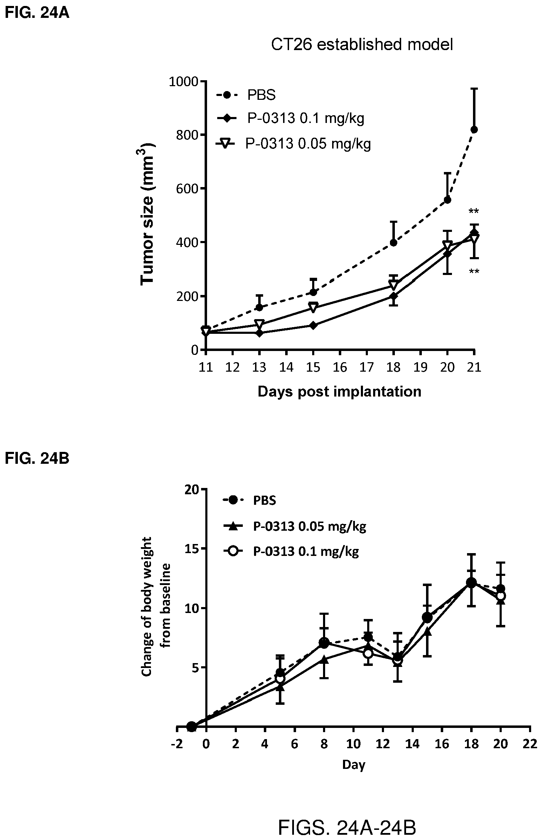

FIG. 24 depicts the antitumor efficacy of P-0313 in subcutaneously established CT26 murine colorectal tumor model. 1 × 10⁵ CT26 cells were subcutaneously injected on day 0. Vehicle (PBS) or P-0313 (0.1 or 0.05 mg/kg) were giving Q5D for two injections initiated when the average tumor volume was ~70 mm³ (day 11). (A) Growth curve of CT26 s. c. tumors. (B) Change of body weight from baseline. Data are expressed as mean ± SEM. Statistical analysis was performed by two-way anova followed by Tukey's post hoc test. ** p<0.0001 compared to PBS group.

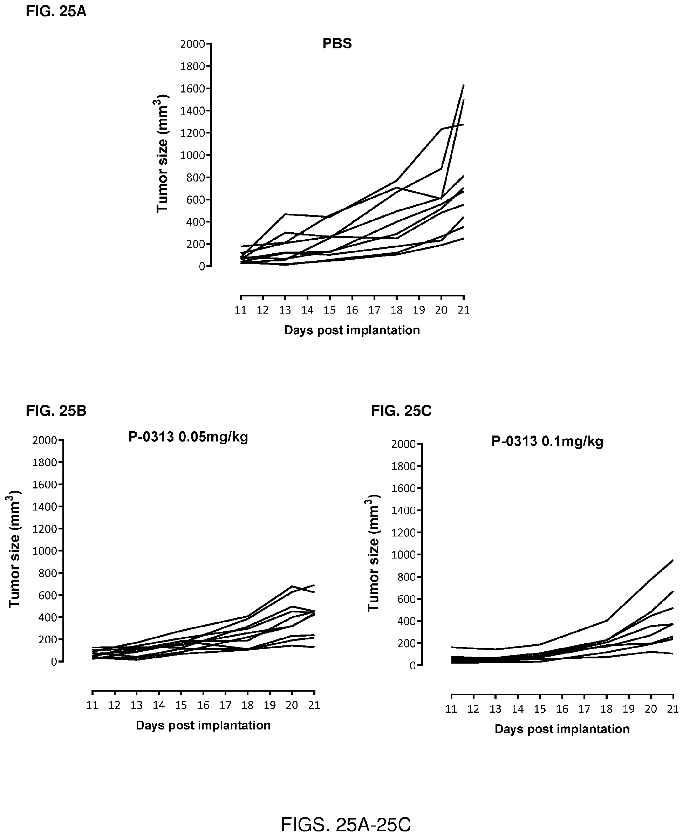

FIG. 25 depicts subcutaneous CT26 tumor growth curve in individual mouse receiving (A) vehicle PBS, (B) 0.05 mg/kg P-0313, or (C) 0.01 mg/kg P-0313. n = 10/group.

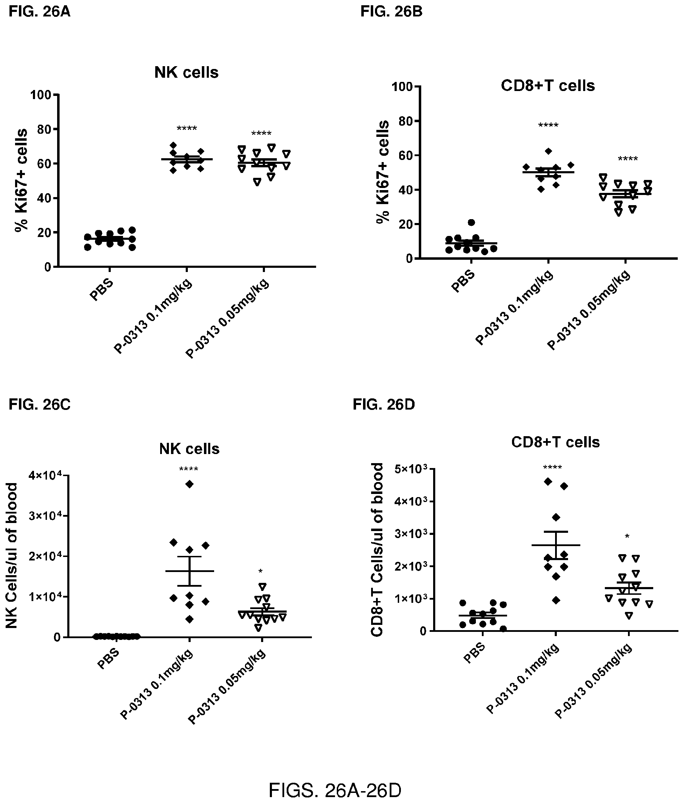

FIG. 26 depicts the NK and CD8 T cell proliferation and expansion in mice treated with P-0313 in CT26 murine colorectal carcinoma tumor model. Following two Q5D treatments initiated 11 days after tumor implantation, increases in Ki67 expression (A-B) and the number of circulating cells (per µl whole blood) (C-D) for NK cells and CD8+ T cells were determined on day 19 by flow cytometry. Data are expressed as mean ± SEM; Statistical analysis was performed by one-way anova followed by Tukey's post hoc test. **** p<0.0001, * P<0.05, compared to PBS group.

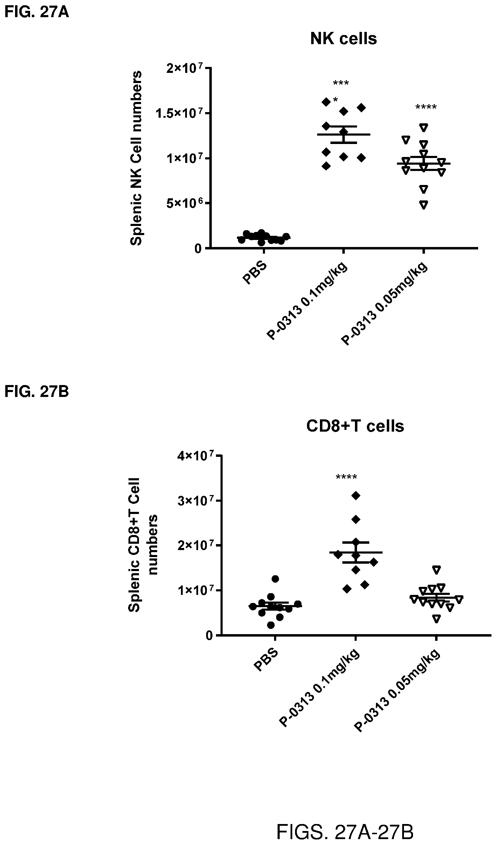

FIG. 27 depicts the immuno-phenotyping of splenic NK and CD8 T cells in the CT26 colorectal tumor-bearing mice treated with P-0313. Following two Q5D treatments initiated 11 days after tumor implantation, increases in the number of splenic NK cells (A) and CD8+ T cells (B) one day 21 were determined by flow cytometry. Data are expressed as mean ± SEM; Statistical analysis was performed by one-way anova followed by Tukey's post hoc test. **** p<0.0001 compared to PBS group.

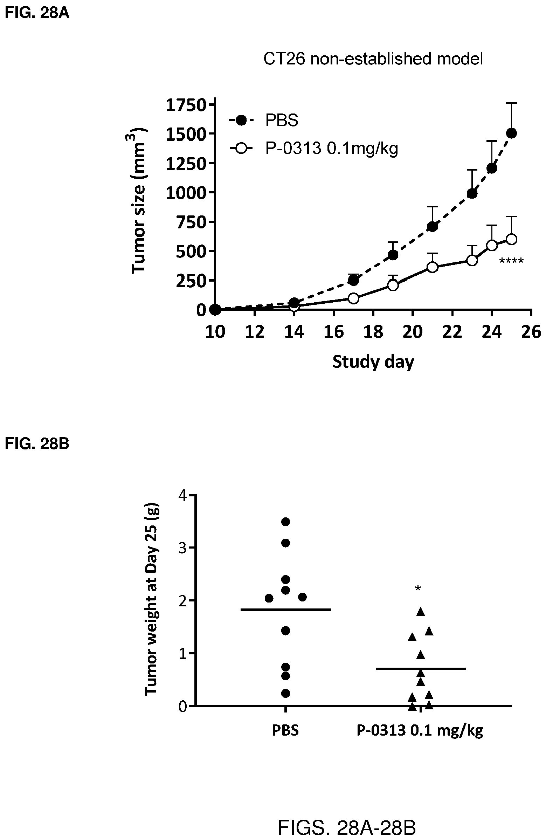

FIG. 28 depicts the antitumor efficacy of P-0313 in non-established CT26 colorectal tumor model. Three days after subcutaneous implantation of 1 × 10⁵ CT26 cells, mice were given vehicle (PBS) or P-0313 (0.1 mg/kg) five Q5D injections. (A) Growth curve of CT26 s. c. tumors after tumor cell implantation on day 0. (B) Tumor weight on Day 25. Data are expressed as mean ± SEM. Statistical analysis was performed by one-way anova followed by Tukey's post hoc test. *** p<0.001, * p<0.05, compared to PBS group

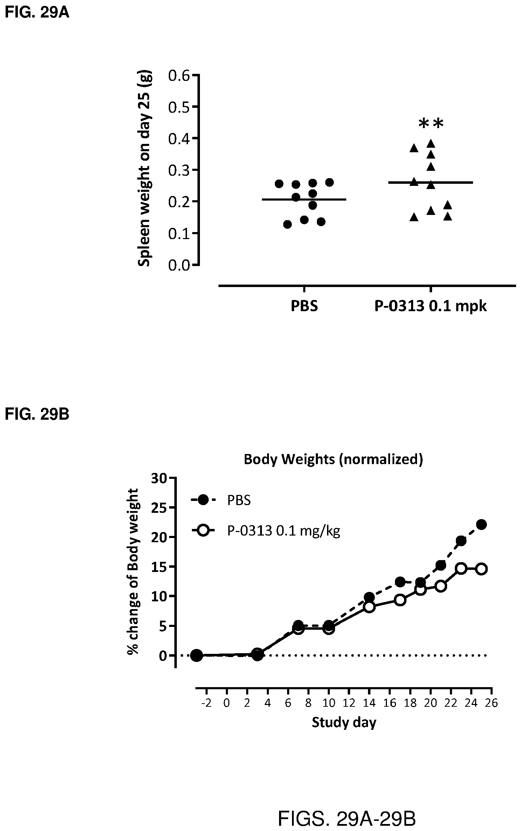

FIG. 29 depicts the spleen weights and the percent change of body weights in CT26 tumor-bearing mice treated with P-0313. Mice were given vehicle (PBS) or P-0313 (0.1 mg/kg) five Q5D injections three days after CT26 tumor cell implantation. (A) Spleen weighs on day 25. (B) Percent change of body weights over 25 days. Data are expressed as mean ± SEM. Statistical analysis was performed by one-way anova followed by Tukey's post hoc test. ** p<0.01, compared to PBS group.
FIG. 30 depicts exemplary IL-15/IL-15Rα (non-covalent) antibody fusion proteins with IL-15/IL-15Rα complex fused to the C-terminus of A) homodimeric heavy chains in bivalent format, B) heterodimeric heavy chain in monovalent format, and C) light chain.

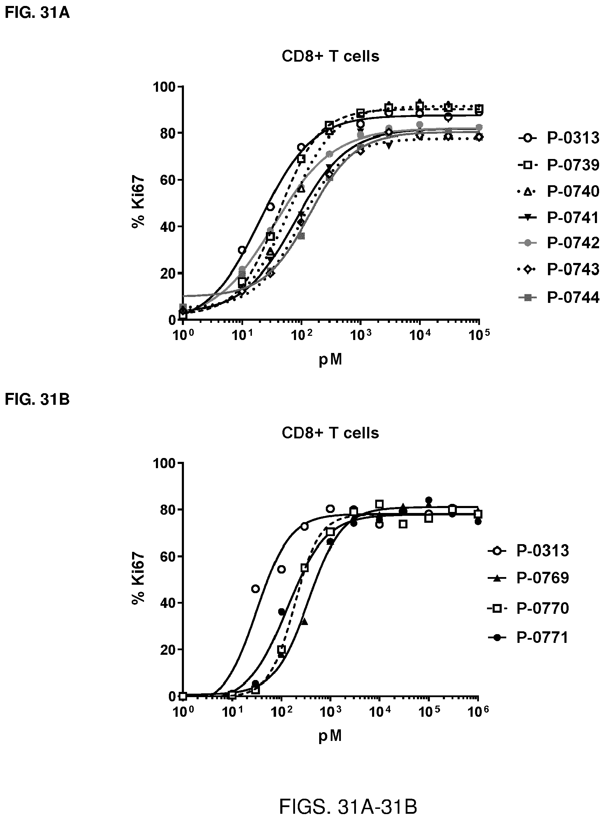

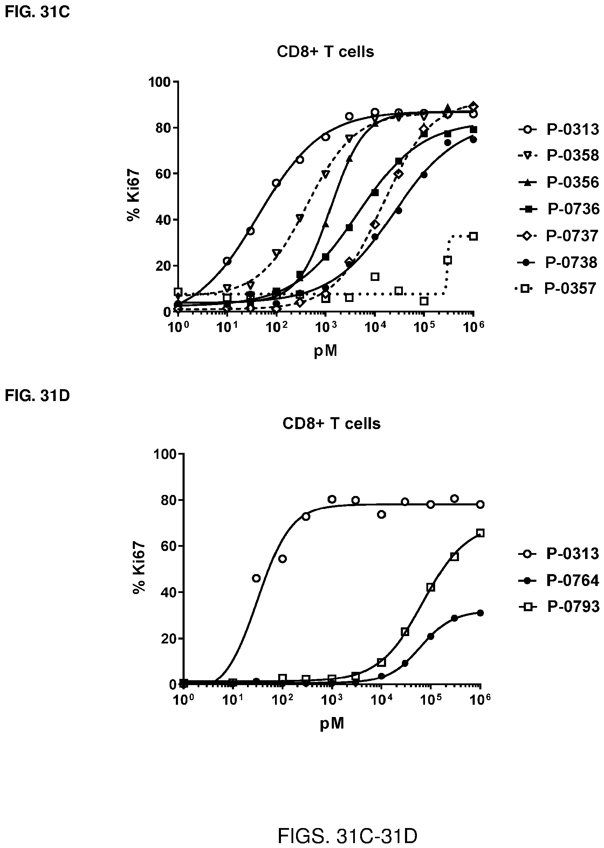

FIG. 31 depicts the impact of various IL-15 single amino acid substitutions at A) S58, B) V63, C) I68, or D) Q108 position on the cytokine's activity in inducing Ki67 expression on CD8+ T cells. All the exemplary IL-15 variant fusion proteins are of the bivalent IL-15/IL-15Rα (non-covalent) Fc fusion format with the exception of P-0764 and P-0793 (FIG. 31D), which are of monovalent IL-15/IL-15Rα (non-covalent) Fc fusion format. P-0313, a well-characterized bivalent IL-15 S58D/IL-15Rα (non-covalent) Fc fusion protein, was included as a control.

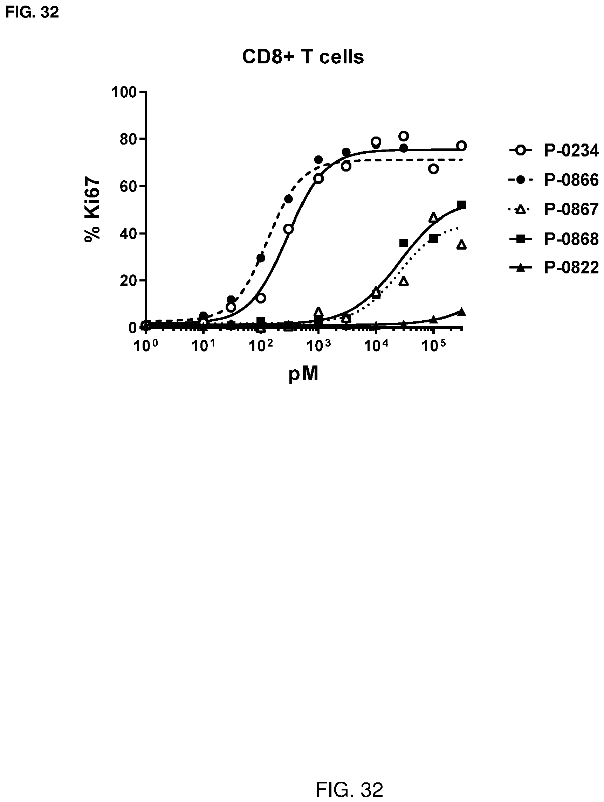

FIG. 32 depicts dose-dependent induction of Ki67 expression on CD8+ T cells following treatment with IL-15 variant fusion proteins in fresh human PBMC. P-0866, P-0867, and P-0868 are all of the bivalent IL-15/IL-15Rα (non-covalent) Fc fusion format and comprise 1, 2, and 3 amino acid deletion at the N-terminus of IL-15, respectively. P-0234 is of the same format comprising wild-type IL-15.

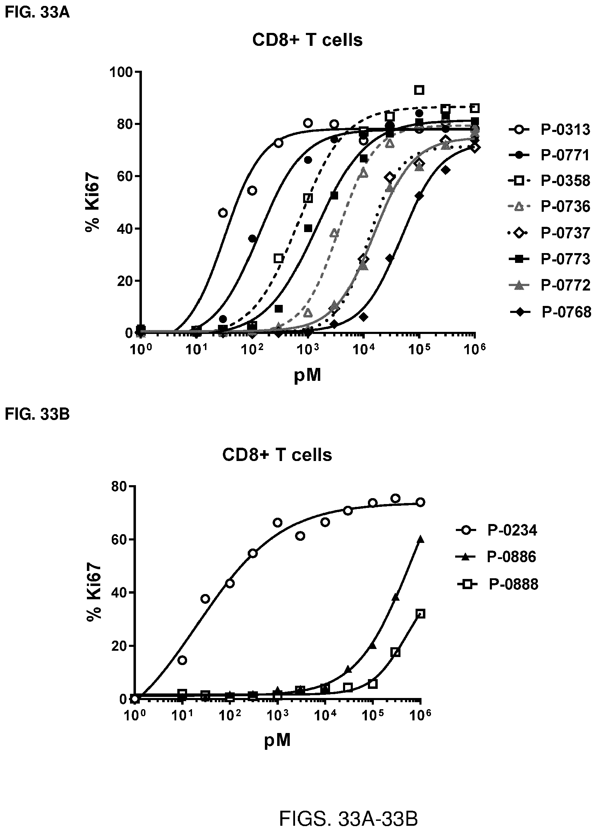

FIG. 33 depicts activity assessment of various IL-15 fusion proteins harboring combinational mutations by analyzing dose-dependent induction of Ki67 expression on CD8+ T cells in fresh human PBMC. A) P-0773, P-0772, and P-0768 comprises one additional V63A substitution to their respective single amino acid change-harboring equivalents, P-0358 (I68H), P-0736 (I68Q), and P-0737 (I68G). P-0771 contains a single V63A mutation. All the exemplary IL-15 variant fusion proteins are of the bivalent IL-15/IL-15Rα (non-covalent) Fc fusion format. B) P-0886 and P-0888 are bivalent IL-15/IL-15Rα (non-covalent) antibody fusion proteins. P-0886 comprises 2-amino acid deletions at the IL-15 N-terminus while P-0888 contains one additional V63A single amino acid substitution. P-0313 and P-0234 are highly potent bivalent IL-15/IL-15Rα (non-covalent) Fc fusion protein and were included as controls.

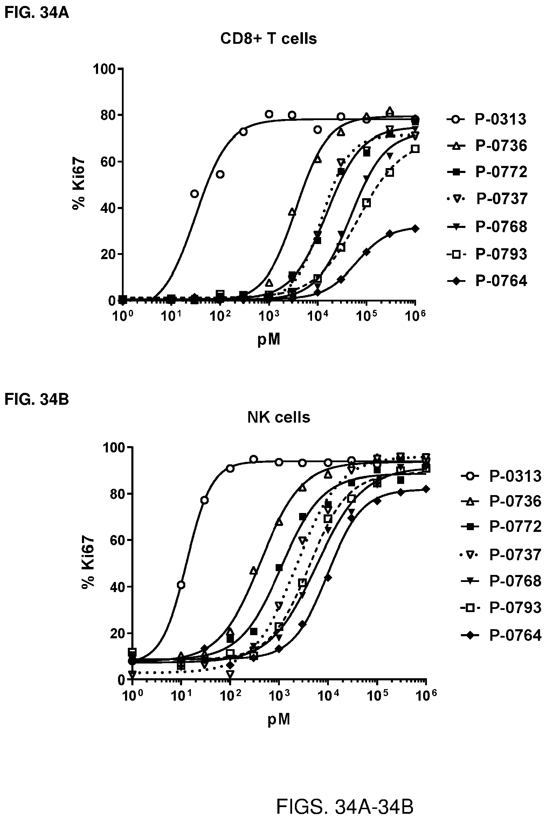

FIG. 34 depicts dose-dependent induction of Ki67 expression on A) CD8+ T cells and B) NK cells following treatment with various IL-15 variant fusion proteins in fresh human PBMC. Compared to the highly potent control, P-0313, all the test compounds demonstrated various levels of activity attenuation.

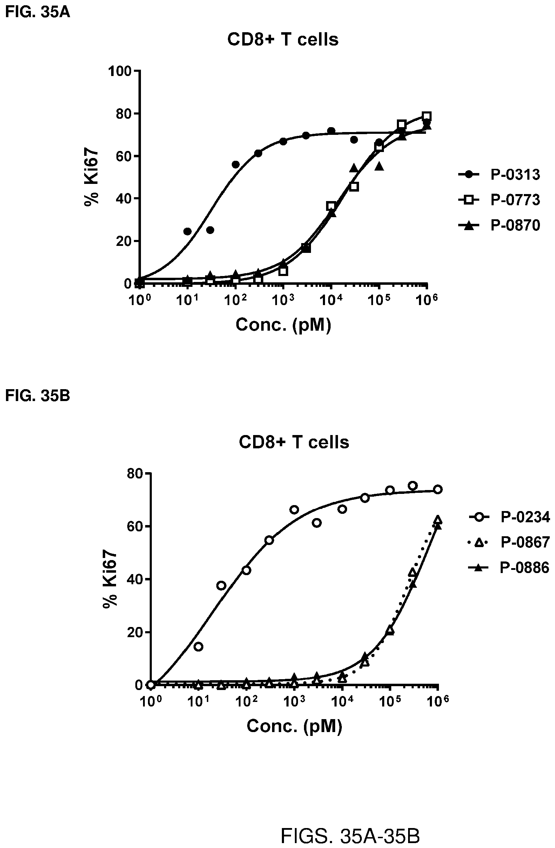

FIG. 35 depicts dose-dependent induction of Ki67 expression on CD8+ T cells following treatment with IL-15 variant fusion proteins in fresh human PBMC. A) P-773 is of the bivalent IL-15/IL-15Rα (non-covalent) Fc fusion format while P-0870 is P-0773's antibody fusion counterpart comprising the identical amino acid substitutions, V63A/I68H, to attenuate IL-15 potency. B) P-867 is of the bivalent IL-15/IL-15Rα (non-covalent) Fc fusion format comprising 2-amino acid deletion at the N-terminus of IL-15, while P-0886 is P-0867's antibody fusion counterpart.

### Mode(s) for Carrying out the Disclosure

The present disclosure provides novel and improved IL-15 fusion proteins for use in the treatment of cancer and other disorders. In various embodiments, the fusion proteins of the invention have two functional domains: an IL-15/IL-15RαSushi domain (also referred to herein as an "IL-15/IL-15RαSushi complex") and an Fc domain, each of which can take different forms, and configured such that the IL-15 is fused to the C-terminal or N-terminal of the Fc domain, and co-expressed and non-covalently complexed with IL-15Rα, IL-15RαSushi or IL-15RαECD or any functional fragment thereof (see FIG. 1). In various embodiments, the fusion proteins of the invention have two functional domains: an IL-15/IL-15RαSushi domain and an antibody or antibody fragment, each of which can take different forms, and configured such that the IL-15 is fused to the C-terminal or N-terminal of the antibody heavy chain or light chain, and co-expressed and non-covalently complexed with IL-15Rα, IL-15RαSushi or IL-15RαECD or any functional fragment thereof (exemplified in FIG. 30).

The present disclosure provides IL-15 variants with amino acid substitution, deletion, insertion and to functions as an IL-15 super-agonist, agonist, or antagonist for use in the treatment of cancer and other disorders.

The present inventors understood that in order to extend the circulating half-life of IL-15 or an IL-15 fusion protein, and/or to increase the biological activity of IL-15 or an IL-15 fusion protein, it is highly desirable to covalently link IL-15 to an human Fc or antibody/antibody fragment at either the N-terminus or C-terminus to enhance the presentation of IL-15 to its signaling receptors and to prevent the disassociation of IL-15 from the fusion protein. The present inventors further believed that it was highly desirable to create fusion protein complexes containing the IL-15Rα domain non-covalently bound to IL-15 to more naturally present IL-15 to IL-15 signaling receptors. Using the formats and strategies described herein, the present inventors demonstrate that increased protein expression, potentially reduced immunogenicity, and protection of IL-15 from degradation can be achieved. In various embodiments described herein, it is preferable to place the IL-15-IL-15Ra complex at the C-terminus to achieve enhanced biological activity, and developability.

The present disclosure thus provides IL-15 variants having one or more amino acid substitutions, deletions, or insertions and which function as an IL-15 antagonist with optimally attenuated potency so as to prevent pathway over-activation and reduce unwanted target sink to mitigate systematic toxicity and improve pharmacokinetics.

### Definitions

The terms "polypeptide", "peptide" and "protein" are used interchangeably herein to refer to a polymer of amino acid residues. In various embodiments, "peptides", "polypeptides", and "proteins" are chains of amino acids whose alpha carbons are linked through peptide bonds. The terminal amino acid at one end of the chain (amino terminal) therefore has a free amino group, while the terminal amino acid at the other end of the chain (carboxy terminal) has a free carboxyl group. As used herein, the term "amino terminus" (abbreviated N-terminus) refers to the free α-amino group on an amino acid at the amino terminal of a peptide or to the α-amino group (amino group when participating in a peptide bond) of an amino acid at any other location within the peptide. Similarly, the term "carboxy terminus" refers to the free carboxyl group on the carboxy terminus of a peptide or the carboxyl group of an amino acid at any other location within the peptide. Peptides also include essentially any polyamino acid including, but not limited to, peptide mimetics such as amino acids joined by an ether as opposed to an amide bond

Polypeptides of the disclosure include polypeptides that have been modified in any way and for any reason, for example, to: (1) reduce susceptibility to proteolysis, (2) reduce susceptibility to oxidation, (3) alter binding affinity for forming protein complexes, (4) alter binding affinities, and (5) confer or modify other physicochemical or functional properties. For example, single or multiple amino acid substitutions (e.g., conservative amino acid substitutions) may be made in the naturally occurring sequence (e.g., in the portion of the polypeptide outside the domain(s) forming intermolecular contacts). A "conservative amino acid substitution" refers to the substitution in a polypeptide of an amino acid with a functionally similar amino acid. The following six groups each contain amino acids that are conservative substitutions for one another:
1) Alanine (A), Serine (S), and Threonine (T)
2) Aspartic acid (D) and Glutamic acid (E)
3) Asparagine (N) and Glutamine (Q)
4) Arginine (R) and Lysine (K)
5) Isoleucine (I), Leucine (L), Methionine (M), and Valine (V)
6) Phenylalanine (F), Tyrosine (Y), and Tryptophan (W)

A "non-conservative amino acid substitution" refers to the substitution of a member of one of these classes for a member from another class. In making such changes, according to various embodiments, the hydropathic index of amino acids may be considered. Each amino acid has been assigned a hydropathic index on the basis of its hydrophobicity and charge characteristics. They are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is understood in the art (see, for example, Kyte et al., 1982, J. Mol. Biol. 157:105-131). It is known that certain amino acids may be substituted for other amino acids having a similar hydropathic index or score and still retain a similar biological activity. In making changes based upon the hydropathic index, in various embodiments, the substitution of amino acids whose hydropathic indices are within + 2 is included. In various embodiments, those that are within + 1 are included, and in various embodiments, those within + 0.5 are included.

It is also understood in the art that the substitution of like amino acids can be made effectively on the basis of hydrophilicity, particularly where the biologically functional protein or peptide thereby created is intended for use in immunological embodiments, as disclosed herein. In various embodiments, the greatest local average hydrophilicity of a protein, as governed by the hydrophilicity of its adjacent amino acids, correlates with its immunogenicity and antigenicity, i.e., with a biological property of the protein.

The following hydrophilicity values have been assigned to these amino acid residues: arginine (+3.0); lysine (+3.0); aspartate (+3.0.+-.1); glutamate (+3.0.+-.1); serine (+0.3); asparagine (+0.2); glutamine (+0.2); glycine (0); threonine (-0.4); proline (-0.5.+-.1); alanine (-0.5); histidine (-0.5); cysteine (-1.0); methionine (-1.3); valine (-1.5); leucine (-1.8); isoleucine (-1.8); tyrosine (-2.3); phenylalanine (-2.5) and tryptophan (-3.4). In making changes based upon similar hydrophilicity values, in various embodiments, the substitution of amino acids whose hydrophilicity values are within + 2 is included, in various embodiments, those that are within + 1 are included, and in various embodiments, those within + 0.5 are included.

Exemplary amino acid substitutions are set forth in Table 1.

**Table 1**

| | | |
|---|---|---|
| Original Residues | Exemplary Substitutions | Preferred Substitutions |
| Ala | Val, Leu, Ile | Val |
| Arg | Lys, Gln, Asn | Lys |
| Asn | Gln | |
| Asp | Glu | |
| Cys | Ser, Ala | Ser |
| Gln | Asn | Asn |
| Glu | Asp | Asp |
| Gly | Pro, Ala | Ala |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, 1,4 Diamino-butyric | Arg |
| | Acid, Gln, Asn | |
| Met | Leu, Phe, Ile | Leu |
| Phe | Leu, Val, Ile, Ala, Tyr | Leu |
| Pro | Ala | Gly |
| Ser | Thr, Ala, Cys | Thr |
| Thr | Ser | |
| Trp | Tyr, Phe | Tyr |
| Tyr | Trp, Phe, Thr, Ser | Phe |
| Val | Ile, Met, Leu, Phe, Ala, Norleucine | Leu |

A skilled artisan will be able to determine suitable variants of polypeptides as set forth herein using well-known techniques. In various embodiments, one skilled in the art may identify suitable areas of the molecule that may be changed without destroying activity by targeting regions not believed to be important for activity. In other embodiments, the skilled artisan can identify residues and portions of the molecules that are conserved among similar polypeptides. In further embodiments, even areas that may be important for biological activity or for structure may be subject to conservative amino acid substitutions without destroying the biological activity or without adversely affecting the polypeptide structure.

Additionally, one skilled in the art can review structure-function studies identifying residues in similar polypeptides that are important for activity or structure. In view of such a comparison, the skilled artisan can predict the importance of amino acid residues in a polypeptide that correspond to amino acid residues important for activity or structure in similar polypeptides. One skilled in the art may opt for chemically similar amino acid substitutions for such predicted important amino acid residues.

One skilled in the art can also analyze the three-dimensional structure and amino acid sequence in relation to that structure in similar polypeptides. In view of such information, one skilled in the art may predict the alignment of amino acid residues of a polypeptide with respect to its three-dimensional structure. In various embodiments, one skilled in the art may choose to not make radical changes to amino acid residues predicted to be on the surface of the polypeptide, since such residues may be involved in important interactions with other molecules. Moreover, one skilled in the art may generate test variants containing a single amino acid substitution at each desired amino acid residue. The variants can then be screened using activity assays known to those skilled in the art. Such variants could be used to gather information about suitable variants. For example, if one discovered that a change to a particular amino acid residue resulted in destroyed, undesirably reduced, or unsuitable activity, variants with such a change can be avoided. In other words, based on information gathered from such routine experiments, one skilled in the art can readily determine the amino acids where further substitutions should be avoided either alone or in combination with other mutations.

The term "polypeptide fragment" and "truncated polypeptide" as used herein refers to a polypeptide that has an amino-terminal and/or carboxy-terminal deletion as compared to a corresponding full-length protein. In certain embodiments, fragments can be, *e.g.,* at least 5, at least 10, at least 25, at least 50, at least 100, at least 150, at least 200, at least 250, at least 300, at least 350, at least 400, at least 450, at least 500, at least 600, at least 700, at least 800, at least 900 or at least 1000 amino acids in length. In certain embodiments, fragments can also be, e.g., at most 1000, at most 900, at most 800, at most 700, at most 600, at most 500, at most 450, at most 400, at most 350, at most 300, at most 250, at most 200, at most 150, at most 100, at most 50, at most 25, at most 10, or at most 5 amino acids in length. A fragment can further comprise, at either or both of its ends, one or more additional amino acids, for example, a sequence of amino acids from a different naturally-occurring protein (e.g., an Fc or leucine zipper domain) or an artificial amino acid sequence (e.g., an artificial linker sequence).

The terms "polypeptide variant", "hybrid polypeptide" and "polypeptide mutant" as used herein refers to a polypeptide that comprises an amino acid sequence wherein one or more amino acid residues are inserted into, deleted from and/or substituted into the amino acid sequence relative to another polypeptide sequence. In certain embodiments, the number of amino acid residues to be inserted, deleted, or substituted can be, e.g., at least 1, at least 2, at least 3, at least 4, at least 5, at least 10, at least 25, at least 50, at least 75, at least 100, at least 125, at least 150, at least 175, at least 200, at least 225, at least 250, at least 275, at least 300, at least 350, at least 400, at least 450 or at least 500 amino acids in length. Hybrids of the present disclosure include fusion proteins.

A "derivative" of a polypeptide is a polypeptide that has been chemically modified, e.g., conjugation to another chemical moiety such as, for example, polyethylene glycol, albumin (e.g., human serum albumin), phosphorylation, and glycosylation.

The term "% sequence identity" is used interchangeably herein with the term "% identity" and refers to the level of amino acid sequence identity between two or more peptide sequences or the level of nucleotide sequence identity between two or more nucleotide sequences, when aligned using a sequence alignment program. For example, as used herein, 80% identity means the same thing as 80% sequence identity determined by a defined algorithm and means that a given sequence is at least 80% identical to another length of another sequence. In certain embodiments, the % identity is selected from, e.g., at least 60%, at least 65%, at least 70%, at least 75%, at least 80%, at least 85%, at least 90%, at least 95%, or at least 99% or more sequence identity to a given sequence. In certain embodiments, the % identity is in the range of, e.g., about 60% to about 70%, about 70% to about 80%, about 80% to about 85%, about 85% to about 90%, about 90% to about 95%, or about 95% to about 99%.

The term "% sequence homology" is used interchangeably herein with the term "% homology" and refers to the level of amino acid sequence homology between two or more peptide sequences or the level of nucleotide sequence homology between two or more nucleotide sequences, when aligned using a sequence alignment program. For example, as used herein, 80% homology means the same thing as 80% sequence homology determined by a defined algorithm, and accordingly a homologue of a given sequence has greater than 80% sequence homology over a length of the given sequence. In certain embodiments, the % homology is selected from, e.g., at least 60%, at least 65%, at least 70%, at least 75%, at least 80%, at least 85%, at least 90%, at least 95%, or at least 99% or more sequence homology to a given sequence. In certain embodiments, the % homology is in the range of, e.g., about 60% to about 70%, about 70% to about 80%, about 80% to about 85%, about 85% to about 90%, about 90% to about 95%, or about 95% to about 99%.

Exemplary computer programs which can be used to determine identity between two sequences include, but are not limited to, the suite of BLAST programs, e.g., BLASTN, BLASTX, and TBLASTX, BLASTP and TBLASTN, publicly available on the Internet at the NCBI website. See also Altschul et al., J. Mol. Biol. 215:403-10, 1990 (with special reference to the published default setting, i.e., parameters w=4, t=17) and Altschul et al., Nucleic Acids Res., 25:3389-3402, 1997. Sequence searches are typically carried out using the BLASTP program when evaluating a given amino acid sequence relative to amino acid sequences in the GenBank Protein Sequences and other public databases. The BLASTX program is preferred for searching nucleic acid sequences that have been translated in all reading frames against amino acid sequences in the GenBank Protein Sequences and other public databases. Both BLASTP and BLASTX are run using default parameters of an open gap penalty of 11.0, and an extended gap penalty of 1.0, and utilize the BLOSUM-62 matrix. See Id.

In addition to calculating percent sequence identity, the BLAST algorithm also performs a statistical analysis of the similarity between two sequences (see, e.g., Karlin & Altschul, Proc. Nat'l. Acad. Sci. USA, 90:5873-5787, 1993). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is, e.g., less than about 0.1, less than about 0.01, or less than about 0.001.

The term "heterologous" as used herein refers to a composition or state that is not native or naturally found, for example, that may be achieved by replacing an existing natural composition or state with one that is derived from another source. Similarly, the expression of a protein in an organism other than the organism in which that protein is naturally expressed constitutes a heterologous expression system and a heterologous protein.

The term "antibody" as used herein refers to a protein comprising one or more polypeptides substantially or partially encoded by immunoglobulin genes or fragments of immunoglobulin genes and having specificity to a tumor antigen or specificity to a molecule overexpressed in a pathological state. The recognized immunoglobulin genes include the kappa, lambda, alpha, gamma, delta, epsilon and mu constant region genes, as well as subtypes of these genes and myriad of immunoglobulin variable region genes. Light chains (LC) are classified as either kappa or lambda. Heavy chains (HC) are classified as gamma, mu, alpha, delta, or epsilon, which in turn define the immunoglobulin classes, IgG, IgM, IgA, IgD and IgE, respectively. A typical immunoglobulin (e.g., antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition.

The term "Fc region" as used herein defines the C-terminal region of an immunoglobulin heavy chain, which may be generated by papain digestion of an intact antibody. The Fc region may be a native sequence Fc region or a variant Fc region. The Fc region of an immunoglobulin generally comprises two constant domains, a CH₂ domain and a CH₃ domain, and optionally comprises a CH₄ domain. The Fc portion of an antibody mediates several important effector functions e.g. cytokine induction, ADCC, phagocytosis, complement dependent cytotoxicity (CDC) and half-life/clearance rate of antibody and antigen-antibody complexes (e.g., the neonatal FcR (FcRn) binds to the Fc region of IgG at acidic pH in the endosome and protects IgG from degradation, thereby contributing to the long serum half-life of IgG). Replacements of amino acid residues in the Fc portion to alter antibody effector function are known in the art (see, e.g., Winter et al., U.S. Patent No. 5,648,260 and 5,624,821). Exemplary Fc amino acid substitutions to abolish binding to FcγRs and C1q, but retain FcRn binding including L234A/L235A/G237A mutations and L234A/L235A/P329G mutations.

"Polynucleotide" refers to a polymer composed of nucleotide units. Polynucleotides include naturally occurring nucleic acids, such as deoxyribonucleic acid ("DNA") and ribonucleic acid ("RNA") as well as nucleic acid analogs. Nucleic acid analogs include those which include non-naturally occurring bases, nucleotides that engage in linkages with other nucleotides other than the naturally occurring phosphodiester bond or which include bases attached through linkages other than phosphodiester bonds. Thus, nucleotide analogs include, for example and without limitation, phosphorothioates, phosphorodithioates, phosphorotriesters, phosphoramidates, boranophosphates, methylphosphonates, chiral-methyl phosphonates, 2-O-methyl ribonucleotides, peptide-nucleic acids (PNAs), and the like. Such polynucleotides can be synthesized, for example, using an automated DNA synthesizer. The term "nucleic acid" typically refers to large polynucleotides. The term "oligonucleotide" typically refers to short polynucleotides, generally no greater than about 50 nucleotides. It will be understood that when a nucleotide sequence is represented by a DNA sequence (i.e., A, T, G, C), this also includes an RNA sequence (i.e., A, U, G, C) in which "U" replaces "T."

Conventional notation is used herein to describe polynucleotide sequences: the left-hand end of a single-stranded polynucleotide sequence is the 5'-end; the left-hand direction of a double-stranded polynucleotide sequence is referred to as the 5'-direction. The direction of 5' to 3' addition of nucleotides to nascent RNA transcripts is referred to as the transcription direction. The DNA strand having the same sequence as an mRNA is referred to as the "coding strand"; sequences on the DNA strand having the same sequence as an mRNA transcribed from that DNA and which are located 5' to the 5'-end of the RNA transcript are referred to as "upstream sequences"; sequences on the DNA strand having the same sequence as the RNA and which are 3' to the 3' end of the coding RNA transcript are referred to as "downstream sequences."

"Complementary" refers to the topological compatibility or matching together of interacting surfaces of two polynucleotides. Thus, the two molecules can be described as complementary, and furthermore, the contact surface characteristics are complementary to each other. A first polynucleotide is complementary to a second polynucleotide if the nucleotide sequence of the first polynucleotide is substantially identical to the nucleotide sequence of the polynucleotide binding partner of the second polynucleotide, or if the first polynucleotide can hybridize to the second polynucleotide under stringent hybridization conditions.

A "vector" is a polynucleotide that can be used to introduce another nucleic acid linked to it into a cell. One type of vector is a "plasmid," which refers to a linear or circular double stranded DNA molecule into which additional nucleic acid segments can be ligated. Another type of vector is a viral vector (e.g., replication defective retroviruses, adenoviruses and adeno-associated viruses), wherein additional DNA segments can be introduced into the viral genome. Certain vectors are capable of autonomous replication in a host cell into which they are introduced (e.g., bacterial vectors comprising a bacterial origin of replication and episomal mammalian vectors). Other vectors (e.g., non-episomal mammalian vectors) are integrated into the genome of a host cell upon introduction into the host cell, and thereby are replicated along with the host genome. An "expression vector" is a type of vector that can direct the expression of a chosen polynucleotide.

A "regulatory sequence" is a nucleic acid that affects the expression (e.g., the level, timing, or location of expression) of a nucleic acid to which it is operably linked. The regulatory sequence can, for example, exert its effects directly on the regulated nucleic acid, or through the action of one or more other molecules (e.g., polypeptides that bind to the regulatory sequence and/or the nucleic acid). Examples of regulatory sequences include promoters, enhancers and other expression control elements (e.g., polyadenylation signals). Further examples of regulatory sequences are described in, for example, Goeddel, 1990, Gene Expression Technology: Methods in Enzymology 185, Academic Press, San Diego, Calif. and Baron et al., 1995, Nucleic Acids Res. 23:3605-06. A nucleotide sequence is "operably linked" to a regulatory sequence if the regulatory sequence affects the expression (e.g., the level, timing, or location of expression) of the nucleotide sequence.

A "host cell" is a cell that can be used to express a polynucleotide of the disclosure. A host cell can be a prokaryote, for example, *E. coli,* or it can be a eukaryote, for example, a single-celled eukaryote (e.g., a yeast or other fungus), a plant cell (e.g., a tobacco or tomato plant cell), an animal cell (e.g., a human cell, a monkey cell, a hamster cell, a rat cell, a mouse cell, or an insect cell) or a hybridoma. Typically, a host cell is a cultured cell that can be transformed or transfected with a polypeptide-encoding nucleic acid, which can then be expressed in the host cell. The phrase "recombinant host cell" can be used to denote a host cell that has been transformed or transfected with a nucleic acid to be expressed. A host cell also can be a cell that comprises the nucleic acid but does not express it at a desired level unless a regulatory sequence is introduced into the host cell such that it becomes operably linked with the nucleic acid. It is understood that the term host cell refers not only to the particular subject cell but also to the progeny or potential progeny of such a cell. Because certain modifications may occur in succeeding generations due to, e.g., mutation or environmental influence, such progeny may not, in fact, be identical to the parent cell, but are still included within the scope of the term as used herein.

The term "isolated molecule" (where the molecule is, for example, a polypeptide or a polynucleotide) is a molecule that by virtue of its origin or source of derivation (1) is not associated with naturally associated components that accompany it in its native state, (2) is substantially free of other molecules from the same species (3) is expressed by a cell from a different species, or (4) does not occur in nature. Thus, a molecule that is chemically synthesized, or expressed in a cellular system different from the cell from which it naturally originates, will be "isolated" from its naturally associated components. A molecule also may be rendered substantially free of naturally associated components by isolation, using purification techniques well known in the art. Molecule purity or homogeneity may be assayed by a number of means well known in the art. For example, the purity of a polypeptide sample may be assayed using polyacrylamide gel electrophoresis and staining of the gel to visualize the polypeptide using techniques well known in the art. For certain purposes, higher resolution may be provided by using HPLC or other means well known in the art for purification.

A protein or polypeptide is "substantially pure," "substantially homogeneous," or "substantially purified" when at least about 60% to 75% of a sample exhibits a single species of polypeptide. The polypeptide or protein may be monomeric or multimeric. A substantially pure polypeptide or protein will typically comprise about 50%, 60%, 70%, 80% or 90% W/W of a protein sample, more usually about 95%, and preferably will be over 99% pure. Protein purity or homogeneity may be indicated by a number of means well known in the art, such as polyacrylamide gel electrophoresis of a protein sample, followed by visualizing a single polypeptide band upon staining the gel with a stain well known in the art. For certain purposes, higher resolution may be provided by using HPLC or other means well known in the art for purification.

"Linker" refers to a molecule that joins two other molecules, either covalently, or through ionic, van der Waals or hydrogen bonds, e.g., a nucleic acid molecule that hybridizes to one complementary sequence at the 5' end and to another complementary sequence at the 3' end, thus joining two non-complementary sequences. A "cleavable linker" refers to a linker that can be degraded or otherwise severed to separate the two components connected by the cleavable linker. Cleavable linkers are generally cleaved by enzymes, typically peptidases, proteases, nucleases, lipases, and the like. Cleavable linkers may also be cleaved by environmental cues, such as, for example, changes in temperature, pH, salt concentration, etc.

The terms "label" or "labeled" as used herein refers to incorporation of another molecule in the antibody. In one embodiment, the label is a detectable marker, e.g., incorporation of a radiolabeled amino acid or attachment to a polypeptide of biotinyl moieties that can be detected by marked avidin (e.g., streptavidin containing a fluorescent marker or enzymatic activity that can be detected by optical or calorimetric methods). In another embodiment, the label or marker can be therapeutic, e.g., a drug conjugate or toxin. Various methods of labeling polypeptides and glycoproteins are known in the art and may be used. Examples of labels for polypeptides include, but are not limited to, the following: radioisotopes or radionuclides (e.g., ³H, ¹⁴C, ¹⁵N, ³⁵S, ⁹⁰Y, ⁹⁹Tc, ¹¹¹In, ¹²⁵I, ¹³¹I), fluorescent labels (e.g., FITC, rhodamine, lanthanide phosphors), enzymatic labels (e.g., horseradish peroxidase, β-galactosidase, luciferase, alkaline phosphatase), chemiluminescent markers, biotinyl groups, predetermined polypeptide epitopes recognized by a secondary reporter (e.g., leucine zipper pair sequences, binding sites for secondary antibodies, metal binding domains, epitope tags), magnetic agents, such as gadolinium chelates, toxins such as pertussis toxin, taxol, cytochalasin B, gramicidin D, ethidium bromide, emetine, mitomycin, etoposide, tenoposide, vincristine, vinblastine, colchicine, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. In some embodiments, labels are attached by spacer arms of various lengths to reduce potential steric hindrance.

The term "immunotherapy" refers to cancer treatments which include, but are not limited to, treatment using depleting antibodies to specific tumor antigens; treatment using antibody-drug conjugates; treatment using agonistic, antagonistic, or blocking antibodies to co-stimulatory or co-inhibitory molecules (immune checkpoints) such as CD276, CD272, CTLA-4, PD-1, PD-L1, CD40, SIRPα, CD47, OX-40, CD137, GITR, LAG3, ICOS, CD27, 4-1BB, TIM-3, B7-H4, Siglec-7, Siglec-8, Siglec-9, Siglec-15, and VISTA; treatment using bispecific T cell engaging antibodies (BiTE^{®}) such as blinatumomab: treatment involving administration of biological response modifiers such as IL-2, IL-12, IL-15, IL-21, GM-CSF, IFN-α, IFN-β and IFN-γ; treatment using therapeutic vaccines such as sipuleucel-T; treatment using dendritic cell vaccines, or tumor antigen peptide vaccines; treatment using chimeric antigen receptor (CAR)-T cells; treatment using CAR-NK cells; treatment using tumor infiltrating lymphocytes (TILs); treatment using adoptively transferred anti-tumor T cells (ex vivo expanded and/or TCR transgenic); treatment using TALL-104 cells; and treatment using immunostimulatory agents such as Toll-like receptor (TLR) agonists CpG and imiquimod, and treatment using vaccine such as BCG, whereas the combination therapy provides increased effector cell killing of tumor cells, i.e., a synergy exists between the IL-15 constructs and the immunotherapy when co-administered.

The term "effective amount" or "therapeutically effective amount" as used herein refers to an amount of a compound or composition sufficient to treat a specified disorder, condition or disease such as ameliorate, palliate, lessen, and/or delay one or more of its symptoms. In reference to NHL and other cancers or other unwanted cell proliferation, an effective amount comprises an amount sufficient to: (i) reduce the number of cancer cells; (ii) reduce tumor size; (iii) inhibit, retard, slow to some extent and preferably stop cancer cell infiltration into peripheral organs; (iv) inhibit (i.e., slow to some extent and preferably stop) tumor metastasis; (v) inhibit tumor growth; (vi) prevent or delay occurrence and/or recurrence of tumor; and/or (vii) relieve to some extent one or more of the symptoms associated with the cancer. An effective amount can be administered in one or more administrations.

The terms "patient," "individual," and "subject" may be used interchangeably and refer to a mammal, preferably a human or a non-human primate, but also domesticated mammals (e.g., canine or feline), laboratory mammals (e.g., mouse, rat, rabbit, hamster, guinea pig), and agricultural mammals (e.g., equine, bovine, porcine, ovine). In various embodiments, the patient can be a human (e.g., adult male, adult female, adolescent male, adolescent female, male child, female child) under the care of a physician or other health worker in a hospital, psychiatric care facility, as an outpatient, or other clinical context. In various embodiments, the patient may be an immunocompromised patient or a patient with a weakened immune system including, but not limited to patients having primary immune deficiency, AIDS; cancer and transplant patients who are taking certain immunosuppressive drugs; and those with inherited diseases that affect the immune system (e.g., congenital agammaglobulinemia, congenital IgA deficiency). In various embodiments, the patient has an immunogenic cancer, including, but not limited to bladder cancer, lung cancer, melanoma, and other cancers reported to have a high rate of mutations (Lawrence et al., Nature, 499(7457): 214-218, 2013).

"Pharmaceutical composition" refers to a composition suitable for pharmaceutical use in a mammal. A pharmaceutical composition comprises a pharmacologically effective amount of an active agent and a pharmaceutically acceptable carrier. "Pharmacologically effective amount" refers to that amount of an agent effective to produce the intended pharmacological result. "Pharmaceutically acceptable carrier" refers to any of the standard pharmaceutical carriers, vehicles, buffers, and excipients, such as a phosphate buffered saline solution, 5% aqueous solution of dextrose, and emulsions, such as an oil/water or water/oil emulsion, and various types of wetting agents and/or adjuvants. Suitable pharmaceutical carriers and formulations are described in Remington's Pharmaceutical Sciences, 21st Ed. 2005, Mack Publishing Co, Easton. A "pharmaceutically acceptable salt" is a salt that can be formulated into a compound for pharmaceutical use including, e.g., metal salts (sodium, potassium, magnesium, calcium, etc.) and salts of ammonia or organic amines.

The phrase "administering" or "cause to be administered" refers to the actions taken by a medical professional (e.g., a physician), or a person controlling medical care of a patient, that control and/or permit the administration of the agent(s)/compound(s) at issue to the patient. Causing to be administered can involve diagnosis and/or determination of an appropriate therapeutic regimen, and/or prescribing particular agent(s)/compounds for a patient. Such prescribing can include, for example, drafting a prescription form, annotating a medical record, and the like. Where administration is described herein, "causing to be administered" is also contemplated.

"Resistant or refractory cancer" refers to tumor cells or cancer that do not respond to previous anti-cancer therapy including, e.g., chemotherapy, surgery, radiation therapy, stem cell transplantation, and immunotherapy. Tumor cells can be resistant or refractory at the beginning of treatment, or they may become resistant or refractory during treatment. Refractory tumor cells include tumors that do not respond at the onset of treatment or respond initially for a short period but fail to respond to treatment. Refractory tumor cells also include tumors that respond to treatment with anticancer therapy but fail to respond to subsequent rounds of therapies. For purposes of this invention, refractory tumor cells also encompass tumors that appear to be inhibited by treatment with anticancer therapy but recur up to five years, sometimes up to ten years or longer after treatment is discontinued. The anticancer therapy can employ chemotherapeutic agents alone, radiation alone, targeted therapy alone, surgery alone, or combinations thereof. For ease of description and not limitation, it will be understood that the refractory tumor cells are interchangeable with resistant tumor.

The terms "treat", "treating" and "treatment" refer to a method of alleviating or abrogating a biological disorder and/or at least one of its attendant symptoms. As used herein, to "alleviate" a disease, disorder or condition means reducing the severity and/or occurrence frequency of the symptoms of the disease, disorder, or condition. Further, references herein to "treatment" include references to curative, palliative and prophylactic treatment.

It is understood that aspect and embodiments of the disclosure described herein include "consisting" and/or "consisting essentially of" aspects and embodiments.

Reference to "about" a value or parameter herein includes (and describes) variations that are directed to that value or parameter per se. For example, description referring to "about X" includes description of "X".

As used herein and in the appended claims, the singular forms "a," "or," and "the" include plural referents unless the context clearly dictates otherwise. It is understood that aspects and variations of the disclosure described herein include "consisting" and/or "consisting essentially of" aspects and variations.

### IL-15/IL-15RαSushi Complexes

Interleukin-15 (IL-15) is a cytokine identified by two independent groups based upon its ability to stimulate proliferation of the IL-2-dependent CTLL-2 T-cell line in the presence of neutralizing anti-IL-2 antibodies (Steel et al., Trends in Pharmacological Sciences, 33(1):35-41, 2012). IL-15 and Interleukin-2 (IL-2) have similar biologic properties in vitro, consistent with their shared receptor (R) signaling components (IL-2/15Rβγ_{c}). However, specificity for IL-15 versus IL-2 is provided by unique private α-chain receptors that complete the IL-15Rαβγ and IL-2Rαβγ heterotrimeric high-affinity receptor complexes and thereby allow differential responsiveness depending on the ligand and high-affinity receptor expressed. Intriguingly, both IL-15 and IL-15Rα transcripts have a much broader tissue distribution than IL-2/IL-2Rα. Further, multiple complex posttranscriptional regulatory mechanisms tightly control IL-15 expression. Thus, based upon complex regulation, as well as differential patterns of IL-15 and IL-15Rα expression, it is likely that the critical in vivo functions of this receptor/ligand pair differ from those of IL-2 and IL-2Rα. Studies to date examining the biology of IL-15 have identified several key nonredundant roles, such as IL-15's importance during natural killer (NK) cell, NK-T cell, and intestinal intraepithelial lymphocyte development and function. A role for IL-15 during autoimmune processes such as rheumatoid arthritis and malignancies such as adult T-cell leukemia suggest that dysregulation of IL-15 may result in deleterious effects for the host (Fehniger et al., Blood, 97:14-32, 2001).

As used herein, the terms "native IL-15" and "native interleukin-15" in the context of proteins or polypeptides refer to any naturally occurring mammalian interleukin-15 amino acid sequences, including immature or precursor and mature forms. Non-limiting examples of GenBank Accession Nos. for the amino acid sequence of various species of native mammalian interleukin-15 include NP_000576 (human, immature form), CAA62616 (human, immature form), NP_001009207 (Felis catus, immature form), AAB94536 (rattus, immature form), AAB41697 (rattus, immature form), NP_032383 (Mus musculus, immature form), AAR19080 (canine), AAB60398 (macaca mulatta, immature form), AAI00964 (human, immature form), AAH23698 (mus musculus, immature form), and AAH18149 (human). In various embodiments of the present invention, native IL-15 is the immature or precursor form of a naturally occurring mammalian IL-15. In other embodiments, native IL-15 is the mature form of a naturally occurring mammalian IL-15. In various embodiments, native IL-15 is the precursor form of naturally occurring human IL-15. In various embodiments, native IL-15 is the mature form of naturally occurring human IL-15. In various embodiments, the native IL-15 protein/polypeptide is isolated or purified. In various embodiments, the IL-15 domain is derived from the amino acid sequence of the human IL-15 precursor sequence set forth in SEQ ID NO: 1:

MRISKPHLRSISIQCYLCLLLNSHFLTEAGIHVFILGCFSAGLPKTEANWVNVISDLKKIED
LIQSMHIDATLYTESDVHPSCKVTAMKCFLLELQVISLESGDASIHDTVENLIILANNSLSS
NGNVTESGCKECEELEEKNIKEFLQSFVHIVQMFINTS (SEQ ID NO: 1)

IL-15 receptor is a type I cytokine receptor consisting of a beta (β) and gamma (γ) subunit that it shares with IL-2 receptor, and an alpha (α) subunit which binds IL-15 with a high affinity. The full-length human IL-15Rα is a type-1 transmembrane protein with multiple Nor O-linked glycosylation sites comprising a signal peptide of 32 AAs, an extracellular domain of 173 AAs, a transmembrane domain of 21 AAs, and a 37-AA cytoplasmic tail (Anderson et al., J. Biol Chem, 270:29862- 29869, 1995). It has been previously demonstrated that a natural soluble form of IL-15R alpha chain corresponding to the entire extracellular domain of IL-15R alpha behaves as a high affinity IL-15 antagonist. However, in sharp contrast with that finding, it was demonstrated that a recombinant, soluble sushi domain of IL-15R alpha, which bears most of the binding affinity for IL-15, behaves as a potent IL-15 agonist by enhancing its binding and biological effects (proliferation and protection from apoptosis) through the IL-15R beta/gamma heterodimer, whereas it does not affect IL-15 binding and function of the tripartite IL-15R alpha/beta/gamma membrane receptor. These results suggested that, if naturally produced, such soluble sushi domains might be involved in the IL-15 trans-presentation mechanism (Mortier et al., J. Biol Chem, 281(3):1612-1619, 2006).

As used herein, the terms "native IL-15Rα" and "native interleukin-15 receptor alpha" in the context of proteins or polypeptides refer to any naturally occurring mammalian interleukin-15 receptor alpha ("IL-15Rα") amino acid sequence, including immature or precursor and mature forms and naturally occurring isoforms. Non-limiting examples of GenBank Accession Nos. for the amino acid sequence of various native mammalian IL-15Rα include NP_002180 (human), ABK41438 (Macaca mulatta), NP_032384 (Mus musculus), Q60819 (Mus musculus), Q13261 (human). In various embodiments, native IL-15Rα is the immature form of a naturally occurring mammalian IL-15Rα polypeptide. In various embodiments, native IL-15Rα is the mature form of a naturally occurring mammalian IL-15Rα polypeptide. In various embodiments, native IL-15Rα is a form of a naturally occurring mammalian IL-15Rα polypeptide. In various embodiments, native IL-15Rα is the full-length form of a naturally occurring mammalian IL-15Rα polypeptide. In various embodiments, native IL-15Rα is the immature form of a naturally occurring human IL-15Rα polypeptide. In various embodiments, native IL-15Rα is the mature form of a naturally occurring human IL-15Rα polypeptide. In various embodiments, native IL-15Rα is the form of a naturally occurring human IL-15Rα polypeptide. In various embodiments, native IL-15Rα is the full-length form of a naturally occurring human IL-15Rα polypeptide. In various embodiments, a native IL-15Rα protein or polypeptide is isolated or purified. In various embodiments, the IL-15Rα domain is derived from the amino acid sequence of the human IL-15Rα sequence set forth in SEQ ID NO: 3:

MAPRRARGCRTLGLPALLLLLLLRPPATRGITCPPPMSVEHADIWVKSYSLYSRERYIC
NSGFKRKAGTSSLTECVLNKATNVAHWTTPSLKCIRDPALVHQRPAPPSTVTTAGVTP
QPESLSPSGKEPAASSPSSNNTAATTAAIVPGSQLMPSKSPSTGTTEISSHESSHGTPS
QTTAKNWELTASASHQPPGVYPQGHSDTTVAISTSTVLLCGLSAVSLLACYLKSRQTPP
LASVEMEAMEALPVTWGTSSRDEDLENCSHHL (SEQ ID NO: 3)

In various embodiments, native IL-15Rα is the full extracellular form of a naturally occurring human IL-15Rα polypeptide. In various embodiments, a native IL-15Rα protein or polypeptide is isolated or purified. In various embodiments, the IL-15Rα extracellular domain is derived from the amino acid sequence of the human IL-15Rα sequence set forth in SEQ ID NO: 4:

ITCPPPMSVEHADIWVKSYSLYSRERYICNSGFKRKAGTSSLTECVLNKATNVAHWTTP
SLKCIRDPALVHQRPAPPSTVTTAGVTPQPESLSPSGKEPAASSPSSNNTAATTAAIVP
GSQLMPSKSPSTGTTEISSHESSHGTPSQTTAKNWELTASASHQPPGVYPQGHSDTT
(SEQ ID NO: 4)

In various embodiments, the IL-15 fusion proteins of the present invention contain an IL-15/IL-15RαSushi complex wherein the IL-15 domain comprises the amino acid sequence of the mature human IL-15 polypeptide as set forth in SEQ ID NO: 2: 
NWVNVISDLKKIEDLIQSMHIDATLYTESDVHPSCKVTAMKCFLLELQVISLESGDASIHD
TVENLIILANNSLSSNGNVTESGCKECEELEEKNIKEFLQSFVHIVQMFINTS
 (SEQ ID NO: 2) and wherein the IL-15RαSushi domain comprises the amino acid sequence of the mature human IL-15Rα polypeptide as set forth in SEQ ID NO: 5:

ITCPPPMSVEHADIWVKSYSLYSRERYICNSGFKRKAGTSSLTECVLNKATNVAHWTTP
SLKCIRDPALVHQRPAPP (SEQ ID NO: 5)

In various embodiments, the IL-15 domain of the IL-15/IL-15RαSushi complex will be an IL-15 variant (or mutant) comprising a sequence derived from the sequence of the mature human IL-15 polypeptide as set forth in SEQ ID NO: 2. Variants (or mutants) of IL-15 are referred to herein using the native amino acid, its position in the mature sequence and the variant amino acid. For example, "hulL-15S58D" refers to human IL-15 comprising a substitution of S to D at position 58 of SEQ ID NO: 2. In various embodiments, the IL-15 variant binds the IL-15Rα polypeptide and functions as an IL-15 agonist or antagonist. In various embodiments, the IL-15 variants with agonist activity have super agonist activity. In various embodiments, the IL-15 variant can function as an IL-15 agonist or antagonist independent of its association with IL-15Rα. IL-15 agonists are exemplified by comparable or increased biological activity compared to wild type IL-15. IL-15 antagonists are exemplified by decreased biological activity compared to wild type IL-15 or by the ability to inhibit IL-15-mediated responses. In various embodiments, the IL-15 variant binds with increased or decreased activity to the IL-15Rβγ_{C} receptors. In various embodiments, the sequence of the IL-15 variant has at least one amino acid change, e.g. substitution or deletion, compared to the native IL-15 sequence; such changes resulting in IL-15 agonist or antagonist activity. In various embodiments, the amino acid substitutions/deletions are in the domains of IL-15 that interact with IL-15Rβ and/or γ_{C}. In various embodiments, the amino acid substitutions/deletions do not affect binding to the IL-15Rα polypeptide or the ability to produce the IL-15 variant. Suitable amino acid substitutions/deletions to generate IL-15 variants can be identified based on known IL-15 structures, comparisons of IL-15 with homologous molecules such as IL-2 with known structure, through rational or random mutagenesis and functional assays, as provided herein, or other empirical methods. Additionally, suitable amino acid substitutions can be conservative or non-conservative changes and insertions of additional amino acids. In various embodiments, the IL-15 variants of the invention contain one or more than one amino acid substitutions at position 30, 31, 32, 62, 63, 67, 68, or 108 of the mature human IL-15 sequence set forth in SEQ ID NO: 2. In various embodiments, the D30T, V31Y, H32E, S58R, S58Q, T62D, V63A, V63R, V63K, I68H, I68F, I68D, I68K, I68Q, I68G, Q108A, Q108S, Q108E, Q108K, or Q108M substitutions or substitution combinations result in IL-15 variants with antagonist activity and S58D substitutions result in IL-15 variants with agonist activity.

In various embodiments, the IL-15 domain of the IL-15/IL-15RαSushi complex will be a human IL-15 variant polypeptide with a deletion from position 111-114 (SEQ ID NO: 39). In various embodiments, the IL-15 domain of the IL-15/IL-15RαSushi complex will be a human IL-15 variant polypeptide with a deletion from position 109-114 (SEQ ID NO: 40). In various embodiments, the IL-15 domain of the IL-15/IL-15RαSushi complex will be a human IL-15 variant polypeptide with a deletion from position 108-114 (SEQ ID NO: 41). In various embodiments, the IL-15 domain of the IL-15/IL-15RαSushi complex will be a human IL-15 variant polypeptide with a deletion from position 105-114 (SEQ ID NO: 42). In various embodiments, the IL-15 domain of the IL-15/IL-15RαSushi complex will be a human IL-15 variant polypeptide with one, or two, or three, or four, or five, or six amino acid deletion at the N-terminus (SEQ ID NO: 56-61). In various embodiment, the IL-15 domain of the IL-15/IL-15RαSushi complex will be a human IL-15 variant polypeptide with combination of amino acid substitutions and deletions represented by SEQ ID NOs: 62 and 63. In various embodiments, the IL-15 domain of the IL-15/IL-15RαSushi complex will be a human IL-15 variant polypeptide with a `GS' (SEQ ID NO: 12) insertion after position N95 (SEQ ID NO: 43). In various embodiments, the IL-15 domain of the IL-15/IL-15RαSushi complex will be a human IL-15 variant polypeptide with a `GGSGG' (SEQ ID NO: 153) insertion after position N95 (SEQ ID NO: 44). In various embodiments, the IL-15 domain of the IL-15/IL-15RαSushi complex will be a human IL-15 variant polypeptide with a 'GSSGGSGGS' (SEQ ID NO: 154) insertion after position N95 (SEQ ID NO: 45).

### Fc Domains

Immunoglobulins of IgG class are among the most abundant proteins in human blood. Their circulation half-lives can reach as long as 21 days. Fusion proteins have been reported to combine the Fc regions of IgG with the domains of another protein, such as various cytokines and receptors (see, for example, Capon et al., Nature, 337:525-531, 1989; Chamow et al., Trends Biotechnol., 14:52-60, 1996); U.S. Pat. Nos. 5,116,964 and 5,541,087). The prototype fusion protein is a homodimeric protein linked through cysteine residues in the hinge region of IgG Fc, resulting in a molecule similar to an IgG molecule without the heavy chain variable and CH1 domains and light chains. The dimer nature of fusion proteins comprising the Fc domain may be advantageous in providing higher order interactions (i.e. bivalent or bispecific binding) with other molecules. Due to the structural homology, Fc fusion proteins exhibit *in vivo* pharmacokinetic profile comparable to that of human IgG with a similar isotype.

The term "Fc" refers to molecule or sequence comprising the sequence of a non-antigen-binding fragment of whole antibody, whether in monomeric or multimeric form. The original immunoglobulin source of the native Fc is preferably of human origin and may be any of the immunoglobulins. Native Fc's are made up of monomeric polypeptides that may be linked into dimeric or multimeric forms by covalent (i.e., disulfide bonds) and non-covalent association. The number of intermolecular disulfide bonds between monomeric subunits of native Fc molecules ranges from 1 to 4 depending on class (e.g., IgG, IgA, IgE) or subclass (e.g., IgG1, IgG2, IgG3, lgA1, IgGA2). One example of a native Fc is a disulfide-bonded dimer resulting from papain digestion of an IgG (see Ellison et al. (1982), Nucleic Acids Res. 10: 4071-9). The term "native Fc" as used herein is generic to the monomeric, dimeric, and multimeric forms. Fc domains containing binding sites for Protein A, Protein G, various Fc receptors and complement proteins.

In various embodiments, the term "Fc variant" refers to a molecule or sequence that is modified from a native Fc but still comprises a binding site for the salvage receptor, FcRn. International applications WO 97/34631 (published Sep. 25, 1997) and WO 96/32478 describe exemplary Fc variants, as well as interaction with the salvage receptor, and are hereby incorporated by reference. Furthermore, a native Fc comprises sites that may be removed because they provide structural features or biological activity that are not required for the fusion molecules of the present invention. Thus, in various embodiments, the term "Fc variant" comprises a molecule or sequence that lacks one or more native Fc sites or residues that affect or are involved in (1) disulfide bond formation, (2) incompatibility with a selected host cell (3) N-terminal or C-terminal heterogeneity upon expression in a selected host cell, (4) glycosylation, (5) interaction with complement, (6) binding to an Fc receptor other than a salvage receptor, or (7) antibody-dependent cellular cytotoxicity (ADCC).

The term "Fc domain" encompasses native Fc and Fc variant molecules and sequences as defined above. As with Fc variants and native Fc's, the term "Fc domain" includes molecules in monomeric or multimeric form, whether digested from whole antibody or produced by recombinant gene expression or by other means. In various embodiments, each of the Fc domain monomers in an Fc domain includes amino acid substitutions in the CH2 antibody constant domain to reduce the interaction or binding between the Fc domain and an Fcγ receptor. In various embodiments, each subunit of the Fc domain comprises three amino acid substitutions that reduce binding to an activating Fc receptor and/or effector function wherein said amino acid substitutions are L234A, L235A and G237A (SEQ ID NO: 6).

In various embodiments, each of the two Fc domain monomers in an Fc domain includes amino acid substitutions that promote the heterodimerization of the two monomers. In various other embodiments, heterodimerization of Fc domain monomers can be promoted by introducing different, but compatible, substitutions in the two Fc domain monomers, such as "knob-into-hole" residue pairs. The "knob-into-hole" technique is also disclosed in U.S. Pat. Publication No. 8,216,805. In yet another embodiment, one Fc domain monomer includes the knob mutation T366W and the other Fc domain monomer includes hole mutations T366S, L358A, and Y407V. In various embodiments, two Cys residues were introduced (S354C on the "knob" and Y349C on the "hole" side) that form a stabilizing disulfide bridge (SEQ ID NOS: 7 and 8).

In one aspect, the IL-15 fusion proteins of the present invention comprise an IL-15/IL-15RαSushi complex and at least one heterologous protein attached to the IL-15/IL-15RαSushi complex either directly or through a peptide linker sequence to form an IL-15 fusion protein. As used herein the term "fusion protein" refers to a protein having a heterologous polypeptide attached via recombinant DNA techniques. In various embodiments, the heterologous protein is an Fc domain (or functional fragment thereof) and the resultant fusion protein is an IL-15/IL-15RαSushi complex-Fc fusion protein. In various embodiments, IL-15/IL-15RαSushi complex are fused to at least one polypeptide that confers extended half-life on the fusion molecule. Such polypeptides include an IgG Fc or other polypeptides that bind to the neonatal Fcγ/receptor, human serum albumin, or polypeptides that bind to a protein having extended serum half-life, including IgGs, non-IgG immunoglobulin, proteins and non-protein agents, that have increased in vivo half-lives due to the presence of an IgG constant domain, or a portion thereof that binds the FcRn, having one or more amino acid modifications that increase the affinity of the constant domain or fragment for FcRn. Such proteins and molecules with increased half-lives have the advantage that smaller amounts and or less frequent dosing is required in the therapeutic, prophylactic or diagnostic use of such molecules (see, e.g., U.S. Patent No. 7,658,921). In various embodiments, the Fc domain is selected from the group consisting of human IgG1 Fc domain, human IgG2 Fc domain, human IgG3 Fc domain, human IgG4 Fc domain, IgA Fc domain, IgD Fc domain, IgE Fc domain, IgG Fc domain and IgM Fc domain; or any combination thereof. In various embodiments, the Fc domain includes an amino acid change that results in an Fc domain having altered complement or Fc receptor binding properties. Amino acid changes to produce an Fc domain with altered complement or Fc receptor binding properties are known in the art.

In various embodiments, an Fc domain may be mutated to further extend in vivo half-life. In various embodiments, each subunit of the Fc domain comprises three amino acid substitutions that enhance binding to human FcRn wherein said amino acid substitutions are M252Y, S254T, and T256E, disclosed in U.S. Pat. Publication No. 7,658,921 (SEQ ID NO: 105). In various embodiments, each subunit of the Fc domain comprises one amino acid substitution that enhanced binding to human FcRn wherein said amino acid substitution is N434A (SEQ ID NO: 106), disclosed in U.S. Pat. Publication No. 7,371,826. In various embodiments, each subunit of the Fc domain comprises one amino acid substitution that enhanced binding to human FcRn wherein said amino acid substitutions are M428L and N434S, disclosed in U.S. Pat. Publication No. 8,546,543. In various embodiments, half-life extension mutations can be combined with amino acid substitutions that reduce/abolish binding to an activating Fc receptor and thus diminish/abrogate effector function.

In various embodiments, the Fc domain sequence used to make dimeric IL-15/IL-15Rα complex-Fc fusion proteins is the IgG1-Fc domain with reduced/abolished effector function and having the amino acid sequence set forth in SEQ ID NO: 6.

In various embodiments, the Fc domain used to make dimeric IL-15/IL-15Rα complex-Fc fusion proteins is the IgG1-Fc domain with reduced/abolished effector function and extended half-life and having the amino acid sequence set forth in SEQ ID NO: 105.

In various embodiments, the Fc domain used to make dimeric IL-15/IL-15Rα complex-Fc fusion proteins is the IgG1-Fc domain with reduced/abolished effector function and extended half-life and having the amino acid sequence set forth in SEQ ID NO: 106.

In various embodiments, the heterodimeric Fc domain sequence used to make monovalent IL-15/IL-15Rα complex-Fc fusion proteins is the Knob-Fc domain with reduced/abolished effector function and having the sequence set forth in SEQ ID NO: 7.

In various embodiments, the heterodimeric Fc domain sequence used to make monovalent IL-15/IL-15Rα complex-Fc fusion proteins is the Hole-Fc domain with reduced/abolished effector function and having the sequence set forth in SEQ ID NO: 8.

In various embodiments, the heterodimeric Fc domain used to make monovalent IL-15/IL-15Rα complex-Fc fusion proteins is the Knob-Fc domain with reduced/abolished effector function and extended half-life and having the amino acid sequence set forth in SEQ ID NO: 107.

In various embodiments, the heterodimeric Fc domain used to make monovalent IL-15/IL-15Rα complex-Fc fusion proteins is the Hole-Fc domain with reduced/abolished effector function and extended half-life and having the amino acid sequence set forth in SEQ ID NO: 108.

### Tumor Associated Antigen Antibodies and Protein/Peptide Binders

In various embodiments, the heterologous protein may comprise an antibody, antibody fragment, or protein or peptide capable of binding to a tumor associated antigen (TAA) on a diseased cell or diseased tissue. The TAA can be any molecule, macromolecule, combination of molecules, etc. against which an immune response is desired. The TAA can be a protein that comprises more than one polypeptide subunit. For example, the protein can be a dimer, trimer, or higher order multimer. In various embodiments, two or more subunits of the protein can be connected with a covalent bond, such as, for example, a disulfide bond. In various embodiments, the subunits of the protein can be held together with non-covalent interactions. Thus, the TAA can be any peptide, polypeptide, protein, nucleic acid, lipid, carbohydrate, or small organic molecule, or any combination thereof, against which the skilled artisan wishes to induce an immune response. In various embodiments, the TAA is a peptide that comprises about 5, about 6, about 7, about 8, about 9, about 10, about 11, about 12, about 13, about 14, about 15, about 16, about 17, about 18, about 19, about 20, about 25, about 30, about 35, about 40, about 45, about 50, about 55, about 60, about 65, about 70, about 75, about 80, about 85, about 90, about 95, about 100, about 150, about 200, about 250, about 300, about 400, about 500, about 600, about 700, about 800, about 900 or about 1000 amino acids. In various embodiments, the peptide, polypeptide, or protein is a molecule that is commonly administered to subjects by injection. In various embodiments, after administration, the tumor-specific antibody or binding protein serves as a targeting moiety to guide the fusion molecule to the diseased site, such as a cancer site, where the active domain can interact with its cognate receptors on diseased cells or diseased tissue.

Any of the foregoing markers can be used as disease associated targets or TAA targets for the constructs of this invention. In various embodiments, the one or more disease associated targets or its variant, or TAA, TAA variant, or TAA mutant contemplated for use in the constructs and methods of the present disclosure is selected from, or derived from, the list provided in Table 2.

**Table 2**

| List of tumor associated antigens | |
|---|---|
| **Tumor Associated Antigen** | **RefSeq (protein)** |
| Her2/neu | NP_001005862 |
| Her3 | NP_001005915 |
| Her4 | NP_001036064 |
| EGF | NP_001171601 |
| EGFR | NP_005219 |
| CD2 | NP_001758 |
| CD3 | NM_000732 |
| CD5 | NP_055022 |
| CD7 | NP_006128 |
| CD13 | NP_001141 |
| CD19 | NP_001171569 |
| CD20 | NP_068769 |
| CD21 | NP_001006659 |
| CD22 | NP_001762 |
| CD23 | NP_001193948 |
| CD30 | NP_001234 |
| CD33 | NP_001234.3 |
| CD34 | NP_001020280 |
| CD38 | NP_001766 |
| CD40 | NP_001241 |
| CD46 | NP_002380 |
| CD55 | NP_000565 |
| CD59 | NP_000602 |
| CD69 | NP_001772 |
| CD70 | NM_001252 |
| CD71 | NP_001121620 |
| CD80 | NP_005182 |
| CD97 | NP_001020331 |
| CD117 | NP_000213 |
| CD127 | NP_002176 |
| CD134 | NP_003318 |
| CD137 | NP_001552 |
| CD138 | NP_001006947 |
| CD146 | NP_006491 |
| CD147 | NP_001719 |
| CD152 | NP_001032720 |
| CD154 | NP_000065 |
| CD195 | NP_000570 |
| CD200 | NP_001004196 |
| CD212 | NP_001276952 |
| CD223 | NP_002277 |
| CD253 | NP_001177871 |
| CD272 | NP_001078826 |
| CD274 (PD-L1) | NP_001254635 |
| CD276 | NP_001019907 |
| CD278 | NP_036224 |
| CD279 (PD-1) | NP_005009 |
| CD309 (VEGFR2) | NP_002244 |
| DR6 | NP_055267 |
| Kv1.3 | NP_002223 |
| 5E10 | NP_006279 |
| MUC1 | NP_001018016 |
| uPA | NP_002649 |
| SLAMF7 (CD319) | NP_001269517 |
| MAGE 3 | NP_005353 |
| MUC 16 (CA-125) | NP_078966 |
| KLK3 | NP_001025218 |
| K-ras | NP_004976 |
| Mesothelin | NP_001170826 |
| p53 | NP_000537 |
| Survivin | NP_001012270 |
| G250 (Renal Cell Carcinoma Antigen) | GenBank CAB82444.1 |
| PSMA | NP_001014986 |
| HLA-DR | NP_001020330 |
| 1D10 | NP_114143 |
| Collagen Type I | NP_000079 |
| Collagen Type II | NP_000080 |
| Fibronectin | XP_005246463 |
| Tenascin | NP_002151 |
| Matrix Metalloproteinase-2 (MMP-2) | NP_001121363 |
| Matrix Metalloproteinase-9 (MMP-9) | NP_004985 |
| Matrix Metalloproteinase-14 (MMP-14) | NP_004986 |
| Fibroblast Activation Protein (FAP) | NM_004460.3 |
| Siglec-8 | NP_055257 |
| Siglec-9 | NP_001185487 |
| Siglec-15 | NP_998767 |
| Legumain | NP_001008530 |
| Tyrosinase | NP_000363 |
| Melan-A (MART I) | NP_005502 |
| SSX-2 | NP_003138 |
| MAGE-1 | NP_004979 |
| NY-ESO-1 (CTAG1) | NP_001318 |
| PRAME | NP_006106 |
| PSA | NP_001639 |
| C35 | NP_115715 |
| SSX-4 | NP_783856 |
| gp100 (Pmel17) | NP_008859 |
| TTF1 | NP_003308 |
| mammaglobin | NP_002402 |
| Brst2 | NP_002643 |
| Mesothelin, isoform 1 | NP_005814 |
| Mesothelin, isoform 2 | NP_037536 |
| PSCA | NP_005663 |
| SYCP-1 | NP_003167 |
| PLK1 | NP_005321 |
| VEGF-A | NP_001020537.2 |
| Alpha fetoprotein (AFP) | NP_001125 |

Further examples of tumor-associated antigens include TRP-1, TRP-2, MAG-1, MAGE-3, BAGE, GAGE-1, GAGE-2, p15(58), CEA, RAGE, NY-BSO(LAGE), SCP-1, Hom/Mel-40, H-Ras, BCR-ABL, E2A-PRL, H4-RET, IGH-IGK, MYL-RAR, human papillomavirus (HPV) antigens E6 and E7, TSP-180, MAGE-4, MAGE-5, MAGE-6, p185erbB2, p180erbB-3, c-met nm-23H1, PSA, TAG-72-4, CA 19-9, CA 72-4, CAM 17.1, Numa, K-ras, β-Catenin, CDK4, Muni-1, p16, TAGE, PSCA, CT7, telomerase, 43-9F, 5T4, 791Tgp72, β-HCG, BCA225, BTAA, CA 15-3 (CA 27.29\BCAA), CA 195, CA 242, CA-50, CAM43, CD68\KF1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, and TPS.

### Immune Checkpoint Modulators

A number of immune-checkpoint protein antigens have been reported to be expressed on various immune cells, including, e.g., CD152 (expressed by activated CD8+ T cells, CD4+ T cells and regulatory T cells), CD279 (expressed on tumor infiltrating lymphocytes, expressed by activated T cells (both CD4 and CD8), regulatory T cells, activated B cells, activated NK cells, anergic T cells, monocytes, dendritic cells), CD274 (expressed on T cells, B cells, dendritic cells, macrophages, vascular endothelial cells, pancreatic islet cells), and CD223 (expressed by activated T cells, regulatory T cells, angergic T cells, NK cells, NKT cells, and plasmacytoid dendritic cells)(see, e.g., Pardoll, D., Nature Reviews Cancer, 12:252-264, 2012). Antibodies that bind to an antigen which is determined to be an immune-checkpoint protein are known to those skilled in the art. For example, various anti-CD276 antibodies have been described in the art (see, e.g., U.S. Pat. Public. No. 20120294796 (Johnson et al) and references cited therein); various anti-CD272 antibodies have been described in the art (see, e.g., U.S. Pat. Public. No. 20140017255 (Mataraza et al) and references cited therein); various anti-CD152/CTLA-4 antibodies have been described in the art (see, e.g., U.S. Pat. Public. No. 20130136749 (Korman et al) and references cited therein); various anti-LAG-3/CD223 antibodies have been described in the art (see, e.g., U.S. Pat. Public. No. 20110150892 (Thudium et al) and references cited therein); various anti-CD279/PD-1 antibodies have been described in the art (see, e.g., U.S. Patent No. 7,488,802 (Collins et al) and references cited therein); various anti-CD274/PD-L1 antibodies have been described in the art (see, e.g., U.S. Pat. Public. No. 20130122014 (Korman et al) and references cited therein); various anti-TIM-3 antibodies have been described in the art (see, e.g., U.S. Pat. Public. No. 20140044728 (Takayanagi et al) and references cited therein); and various anti-B7-H4 antibodies have been described in the art (see, e.g., U.S. Pat. Public. No. 20110085970 (Terrett et al) and references cited therein). Each of these references is hereby incorporated by reference in its entirety for the specific antibodies and sequences taught therein.

In various embodiments, the heterologous protein may comprise an antibody, antibody fragment, or protein or peptide that exhibit binding to an immune-checkpoint protein antigen that is present on the surface of an immune cell. In various embodiments, the immune-checkpoint protein antigen is selected from the group consisting of, but not limited to, CD276, CD272, CD152, CD223, CD279 (PD-1), CD274 (PD-L1), CD40, SIRPα, CD47, OX-40, GITR, ICOS, CD27, 4-1BB, TIM-3, B7-H4, Siglec-7, Siglec-8, Siglec-9, Siglec-15, and VISTA.

In various embodiments, the antibody is a humanized anti-FAP antibody comprising the heavy chain and light chain sequence set forth in SEQ ID NOS: 109-110. In various embodiments, the antibody is an antagonistic Programmed Death-1 (PD-1) antibody or antibody fragment. In various embodiments, the antibody is an antagonistic humanized PD-1 antibody comprising the heavy chain and light chain amino acid sequences set forth in SEQ ID NO: 111-112. In various embodiments, the antibody is an antagonistic human PD-1 antibody comprising the heavy chain and light chain amino acid sequences set forth in SEQ ID NO: 113-114. In various embodiments, the antibody is an antagonistic Programmed Death Ligand-1 (PD-L1) antibody or antibody fragment.

### Linkers

In various embodiments, the heterologous protein (e.g., Fc domain or antibody/antibody fragment) is covalently linked to the IL-15 polypeptide of the IL-15/IL-15RαSushi (or functional fragment thereof) complex by polypeptide linker sequence. In various embodiments, the linker may be an artificial sequence of between 5, 10, 15, 20, 30, 40 or more amino acids that are relatively free of secondary structure. In various embodiments, the linker is rich in G/S content (e.g., at least about 60%, 70%, 80%, 90%, or more of the amino acids in the linker are G or S). In various embodiments, the linker is selected from the group of sequences set forth in SEQ ID NOs: 9-12, SEQ ID NO: 47, and SEQ ID NOs: 153-154. Each peptide linker sequence can be selected independently.

### Examples of novel IL-15/IL-15RαSushi complex fusion proteins

In various embodiments, exemplary IL-15/IL-15RαSushi heterodimeric Fc fusion proteins of the present invention are listed in Table 3A with fusion protein ID numbers and fusion protein Chain 1 and Chain 2 SEQ ID NOs.

**Table 3A**

| Exemplary IL-15/IL-15RαSushi heterodimeric Fc fusion protein | | |
|---|---|---|
| Fusion protein ID | Fusion protein SEQ ID NOs: | |
| | Chain 1 | Chain 2 |
| P-0153 | 13 | 14 |
| P-0156 | 15 | 16 |
| P-0155 | 17 | 18 |

In various embodiments, exemplary monovalent IL-15/IL-15RαSushi (non-covalent) Fc fusion proteins of the present invention are listed in Table 3B with fusion protein ID numbers and fusion protein Chain 1, Chain 2 and Chain 3 SEQ ID NOs.

**Table 3B**

| Exemplary monovalent IL-15/IL-15RαSushi (non-covalent) Fc fusion protein | | | |
|---|---|---|---|
| Fusion protein ID | Fusion protein SEQ ID NOs: | | |
| | Chain 1 | Chain 2 | Chain 3 |
| P-0197 | 13 | 5 | 7 |
| P-0201 | 15 | 5 | 7 |
| P-0207 | 18 | 5 | 7 |
| P-0217 | 54 | 5 | 7 |
| P-0219 | 54 | 4 | 7 |
| P-0221 | 19 | 5 | 7 |
| P-0222 | 19 | 4 | 7 |

In various embodiments, exemplary bivalent IL-15/IL-15RαSushi (non-covalent) Fc fusion proteins of the present invention are listed in Table 3C with fusion protein ID numbers and fusion protein Chain 1, Chain 2 and Chain 3 SEQ ID NOs.

**Table 3C**

| Exemplary bivalent IL-15/IL-15RαSushi (non-covalent) Fc fusion protein | | | |
|---|---|---|---|
| Fusion protein ID | Fusion protein SEQ ID NOs: | | |
| | Chain 1 | Chain 2 | Chain 3 |
| P-0198 | 13 | 5 | 55 |
| P-0234 | 20 | 5 | × |
| P-0223 | 21 | 5 | × |

In various embodiments, exemplary bivalent IL-15/IL-15RαECD (non-covalent) Fc fusion proteins of the present invention are listed in Table 3D with fusion protein ID numbers and fusion protein Chain 1 and Chain 2 SEQ ID NOs.

**Table 3D**

| Exemplary bivalent IL-15/IL-15RαECD (non-covalent) Fc fusion proteins | | |
|---|---|---|
| Fusion protein ID | Fusion protein SEQ ID NOs: | |
| | Chain 1 | Chain 2 |
| P-0220 | 20 | 4 |
| P-0224 | 21 | 4 |

In various embodiments, exemplary monovalent IL-15 (non-covalent)/IL-15RαSushi Fc fusion proteins of the present invention are listed in Table 3E with fusion protein ID numbers and fusion protein Chain 1, Chain 2 and Chain 3 SEQ ID NOs.

**Table 3E**

| Exemplary monovalent IL-15 (non-covalent)/IL-15RαSushi Fc fusion proteins | | | |
|---|---|---|---|
| Fusion protein ID | Fusion protein SEQ ID NOs: | | |
| | Chain 1 | Chain 2 | Chain 3 |
| P-0165 | 2 | 14 | 8 |
| P-0166 | 2 | 22 | 8 |

In various embodiments, the bivalent IL-15 (non-covalent)/IL-15RαSushi Fc fusion protein of the present invention (also referred to hereinafter as "P-0218") comprises a chain 1 (IL-15) having the amino acid sequences set forth in SEQ ID NO: 2, a chain 2 (Fc-linker-IL-15R*α*-Sushi+) having the amino acid sequence set forth in SEQ ID NO: 23.

In various embodiments, the IL-15/IL-15RαSushi complex will comprise an IL-15 variant having the amino acid sequence selected from the group consisting of the sequences set forth in SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, and SEQ ID NO: 81.

In various embodiments, exemplary IL-15/IL-15RαSushi Fc fusion proteins comprising S58D mutations in IL-15 and of various formats illustrated in FIG. 1 of the present invention are listed in Table 3F with fusion protein ID numbers and fusion protein Chain 1, Chain 2 and Chain 3 SEQ ID NOs.

**Table 3F**

| Exemplary IL-15/IL-15Rα Fc fusion proteins comprising S58D mutations in IL-15 | | | |
|---|---|---|---|
| Fusion protein ID | Fusion protein SEQ ID NOs: | | |
| | Chain 1 | Chain 2 | Chain 3 |
| P-0313 | 46 | 5 | × |
| P-0314 | 23 | 24 | × |
| P-0666 | 48 | 5 | 7 |
| P-0667 | 49 | 5 | 7 |
| P-0668 | 50 | 5 | × |
| P-0669 | 24 | 22 | 8 |
| P-0670 | 24 | 51 | 8 |
| P-0671 | 24 | 52 | × |

In various embodiments, additional IL-15 variant/IL-15Rα complex-Fc fusion proteins comprising two or more heterodimeric chains are set forth in Table 3G:

**Table 3G**

| Additional IL-15 variant /IL-15Rα complex-Fc fusion proteins | | | |
|---|---|---|---|
| Fusion protein ID | Fusion protein SEQ ID NOs: | | |
| | Chain 1 | Chain 2 | Chain 3 |
| P-0739 | 82 | 5 | × |
| P-0740 | 83 | 5 | × |
| P-0741 | 84 | 5 | × |
| P-0742 | 85 | 5 | × |
| P-0743 | 86 | 5 | × |
| P-0744 | 87 | 5 | × |
| P-0771 | 88 | 5 | × |
| P-0184 | 26 | 14 | 8 |
| P-0769 | 89 | 5 | × |
| P-0770 | 90 | 5 | × |
| P-0356 | 91 | 5 | × |
| P-0357 | 92 | 5 | × |
| P-0358 | 93 | 5 | × |
| P-0738 | 94 | 5 | × |
| P-0736 | 95 | 5 | × |
| P-0737 | 96 | 5 | × |
| P-0764 | 97 | 7 | 5 |
| P-0793 | 98 | 7 | 5 |
| P-0805 | 99 | 8 | 5 |
| P-0797 | 100 | 8 | 5 |
| P-0796 | 101 | 8 | 5 |
| P-0773 | 102 | 5 | × |
| P-0772 | 103 | 5 | × |
| P-0768 | 104 | 5 | × |
| P-0866 | 149 | 5 | × |
| P-0867 | 150 | 5 | × |
| P-0868 | 151 | 5 | × |
| P-0822 | 152 | 5 | x |

In various embodiments, the use of IL-15 variants with attenuated potency in IL-15/IL15Rα- antibody fusion protein can facilitate the establishment of stoichiometric balance between the IL-15 and the targeting antibody to achieve optimal dosing at which the antibody can achieve sufficient target occupancy while the IL-15 moiety does not cause over activation of the pathway, so as to minimize peripheral activation, mitigate antigen-sink, and promote tumor targeting via the antibody arm.

In various embodiments, exemplary IL-15 variant/IL-15Rα complex-PD-1 antagonist antibody fusion proteins comprising two or more heterodimeric chains are set forth in Table 3H:

**Table 3H**

| Exemplary IL-15 variant/IL-15Rα complex- PD-1 antagonist antibody fusion proteins | | | | |
|---|---|---|---|---|
| Fusion protein ID | Fusion protein SEQ ID NOs: | | | |
| | Chain 1 | Chain 2 | Chain 3 | Chain 4 |
| P-0820 | 138 | 114 | 5 | x |
| P-0801 | 139 | 144 | 114 | 5 |
| P-0821 | 140 | 144 | 114 | 5 |
| P-0870 | 141 | 114 | 5 | x |
| P-0856 | 142 | 144 | 114 | 5 |
| P-0857 | 143 | 144 | 114 | 5 |
| P-0858 | 145 | 114 | 5 | x |
| P-0886 | 146 | 114 | 5 | x |
| P-0887 | 147 | 114 | 5 | x |
| P-0888 | 148 | 114 | 5 | x |

### Polynucleotides

In another aspect, the present disclosure provides isolated nucleic acid molecules comprising a polynucleotide encoding IL-15, an IL-15 variant, IL-15Rα, an IL-15Rα variant, an Fc, an Fc variant, an IL-15-Fc fusion protein, an IL-15RαSushi-Fc fusion protein, or an IL-15/IL-15RαSushi-Fc fusion protein, an antibody targeting a TAA or immune checkpoint modulator, an antibody fragment, an IL-15-antibody fusion protein, an IL-15/IL-15RαSushi-antibody fusion protein of the present disclosure. The subject nucleic acids may be single-stranded or double stranded. Such nucleic acids may be DNA or RNA molecules. DNA includes, for example, cDNA, genomic DNA, synthetic DNA, DNA amplified by PCR, and combinations thereof. Genomic DNA encoding IL-15/IL-15RαSushi complexes is obtained from genomic libraries which are available for a number of species. Synthetic DNA is available from chemical synthesis of overlapping oligonucleotide fragments followed by assembly of the fragments to reconstitute part or all of the coding regions and flanking sequences. RNA may be obtained from prokaryotic expression vectors which direct high-level synthesis of mRNA, such as vectors using T7 promoters and RNA polymerase. cDNA is obtained from libraries prepared from mRNA isolated from various tissues that express IL-15. The DNA molecules of the disclosure include full-length genes as well as polynucleotides and fragments thereof. The full-length gene may also include sequences encoding the N-terminal signal sequence. Such nucleic acids may be used, for example, in methods for making the novel IL-15/IL-15RαSushi fusion proteins.

In various embodiments, the isolated nucleic acid molecules comprise the polynucleotides described herein, and further comprise a polynucleotide encoding at least one heterologous protein described herein. In various embodiments, the nucleic acid molecules further comprise polynucleotides encoding the linkers or hinge linkers described herein.

In various embodiments, the recombinant nucleic acids of the present disclosure may be operably linked to one or more regulatory nucleotide sequences in an expression construct. Regulatory sequences are art-recognized and are selected to direct expression of the IL-15/IL-15RαSushi fusion protein. Accordingly, the term regulatory sequence includes promoters, enhancers, and other expression control elements. Exemplary regulatory sequences are described in Goeddel; Gene Expression Technology: Methods in Enzymology, Academic Press, San Diego, Calif. (1990). Typically, said one or more regulatory nucleotide sequences may include, but are not limited to, promoter sequences, leader or signal sequences, ribosomal binding sites, transcriptional start and termination sequences, translational start and termination sequences, and enhancer or activator sequences. Constitutive or inducible promoters as known in the art are contemplated by the present disclosure. The promoters may be either naturally occurring promoters, or hybrid promoters that combine elements of more than one promoter. An expression construct may be present in a cell on an episome, such as a plasmid, or the expression construct may be inserted in a chromosome. In various embodiments, the expression vector contains a selectable marker gene to allow the selection of transformed host cells. Selectable marker genes are well known in the art and will vary with the host cell used.

In another aspect of the present disclosure, the subject nucleic acid is provided in an expression vector comprising a nucleotide sequence encoding an IL-15/IL-15RαSushi complex and operably linked to at least one regulatory sequence. The term "expression vector" refers to a plasmid, phage, virus or vector for expressing a polypeptide from a polynucleotide sequence. Vectors suitable for expression in host cells are readily available and the nucleic acid molecules are inserted into the vectors using standard recombinant DNA techniques. Such vectors can include a wide variety of expression control sequences that control the expression of a DNA sequence when operatively linked to it may be used in these vectors to express DNA sequences encoding an IL-15/IL-15RαSushi-Fc or IL-15/IL-15RαSushi-antibody fusion protein. Such useful expression control sequences, include, for example, the early and late promoters of SV40, tet promoter, adenovirus or cytomegalovirus immediate early promoter, RSV promoters, the lac system, the trp system, the TAC or TRC system, T7 promoter whose expression is directed by T7 RNA polymerase, the major operator and promoter regions of phage lambda , the control regions for fd coat protein, the promoter for 3-phosphoglycerate kinase or other glycolytic enzymes, the promoters of acid phosphatase, e.g., PhoS, the promoters of the yeast a-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed. Moreover, the vector's copy number, the ability to control that copy number and the expression of any other protein encoded by the vector, such as antibiotic markers, should also be considered.

A recombinant nucleic acid of the present disclosure can be produced by ligating the cloned gene, or a portion thereof, into a vector suitable for expression in either prokaryotic cells, eukaryotic cells (yeast, avian, insect or mammalian), or both. Expression vehicles for production of a recombinant IL-15/IL-15RαSushi complex include plasmids and other vectors. For instance, suitable vectors include plasmids of the types: pBR322-derived plasmids, pEMBL-derived plasmids, pEX-derived plasmids, pBTac-derived plasmids and pUC-derived plasmids for expression in prokaryotic cells, such as *E*. *coli.*

Some mammalian expression vectors contain both prokaryotic sequences to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papilloma virus (BPV-1), or Epstein-Barr virus (pHEBo, pREP-derived and p205) can be used for transient expression of proteins in eukaryotic cells. Examples of other viral (including retroviral) expression systems can be found below in the description of gene therapy delivery systems. The various methods employed in the preparation of the plasmids and in transformation of host organisms are well known in the art. For other suitable expression systems for both prokaryotic and eukaryotic cells, as well as general recombinant procedures, see Molecular Cloning A Laboratory Manual, 2nd Ed., ed. by Sambrook, Fritsch and Maniatis (Cold Spring Harbor Laboratory Press, 1989) Chapters 16 and 17. In some instances, it may be desirable to express the recombinant polypeptides by the use of a baculovirus expression system. Examples of such baculovirus expression systems include pVL-derived vectors (such as pVL1392, pVL1393 and pVL941), pAcUW-derived vectors (such as pAcUW1), and pBlueBac-derived vectors (such as the B-gal containing pBlueBac III).

In various embodiments, a vector will be designed for production of the subject IL-15/IL-15RαSushi-Fc or IL-15/IL-15RαSushi-antibody fusion proteins in CHO cells, such as a Pcmv-Script vector (Stratagene, La Jolla, Calif.), pcDNA4 vectors (Invitrogen, Carlsbad, Calif.) and pCI-neo vectors (Promega, Madison, Wis.). As will be apparent, the subject gene constructs can be used to cause expression of the subject IL-15/IL-15RαSushi-Fc or IL-15/IL-15RαSushi-antibody fusion proteins in cells propagated in culture, e.g., to produce proteins, including fusion proteins or variant proteins, for purification.

This present disclosure also pertains to a host cell transfected with a recombinant gene including a nucleotide sequence coding an amino acid sequence for one or more of the subject IL-15/IL-15RαSushi-Fc or IL-15/IL-15RαSushi-antibody fusion protein. The host cell may be any prokaryotic or eukaryotic cell. For example, an IL-15/IL-15RαSushi complex of the present disclosure may be expressed in bacterial cells such as *E*. *coli,* insect cells (e.g., using a baculovirus expression system), yeast, or mammalian cells. Other suitable host cells are known to those skilled in the art.

Accordingly, the present disclosure further pertains to methods of producing the subject IL-15/IL-15RαSushi-Fc or IL-15/IL-15RαSushi-antibody fusion proteins. For example, a host cell transfected with an expression vector encoding an IL-15/IL-15RαSushi complex can be cultured under appropriate conditions to allow expression of the IL-15/IL-15RαSushi complex to occur. The IL-15/IL-15RαSushi complex may be secreted and isolated from a mixture of cells and medium containing the IL-15/IL-15RαSushi-Fc or IL-15/IL-15RαSushi-antibody fusion protein. Alternatively, the IL-15/IL-15RαSushi complex may be retained cytoplasmically or in a membrane fraction and the cells harvested, lysed and the protein isolated. A cell culture includes host cells, media and other byproducts. Suitable media for cell culture are well known in the art.

The polypeptides and proteins of the present disclosure can be purified according to protein purification techniques are well known to those of skill in the art. These techniques involve, at one level, the crude fractionation of the proteinaceous and non-proteinaceous fractions. Having separated the peptide polypeptides from other proteins, the peptide or polypeptide of interest can be further purified using chromatographic and electrophoretic techniques to achieve partial or complete purification (or purification to homogeneity). The term "isolated polypeptide" or "purified polypeptide" as used herein, is intended to refer to a composition, isolatable from other components, wherein the polypeptide is purified to any degree relative to its naturally-obtainable state. A purified polypeptide therefore also refers to a polypeptide that is free from the environment in which it may naturally occur. Generally, "purified" will refer to a polypeptide composition that has been subjected to fractionation to remove various other components, and which composition substantially retains its expressed biological activity. Where the term "substantially purified" is used, this designation will refer to a peptide or polypeptide composition in which the polypeptide or peptide forms the major component of the composition, such as constituting about 50%, about 60%, about 70%, about 80%, about 85%, or about 90% or more of the proteins in the composition.

Various techniques suitable for use in purification will be well known to those of skill in the art. These include, for example, precipitation with ammonium sulphate, PEG, antibodies (immunoprecipitation) and the like or by heat denaturation, followed by centrifugation; chromatography such as affinity chromatography (Protein-A columns), ion exchange, gel filtration, reverse phase, hydroxyapatite, hydrophobic interaction chromatography; isoelectric focusing; gel electrophoresis; and combinations of these techniques. As is generally known in the art, it is believed that the order of conducting the various purification steps may be changed, or that certain steps may be omitted, and still result in a suitable method for the preparation of a substantially purified polypeptide.

### Pharmaceutical Compositions

In another aspect, the present disclosure provides a pharmaceutical composition comprising the IL-15/IL-15RαSushi-Fc or IL-15/IL-15RαSushi-antibody fusion proteins in admixture with a pharmaceutically acceptable carrier. Such pharmaceutically acceptable carriers are well known and understood by those of ordinary skill and have been extensively described (see, e.g., Remington's Pharmaceutical Sciences, 18th Edition, A. R. Gennaro, ed., Mack Publishing Company, 1990). The pharmaceutically acceptable carriers may be included for purposes of modifying, maintaining or preserving, for example, the pH, osmolarity, viscosity, clarity, color, isotonicity, odor, sterility, stability, rate of dissolution or release, adsorption or penetration of the composition. Such pharmaceutical compositions may influence the physical state, stability, rate of *in vivo* release, and rate of *in vivo* clearance of the polypeptide. Suitable pharmaceutically acceptable carriers include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCl, citrates, phosphates, other organic acids); bulking agents (such as mannitol or glycine), chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides and other carbohydrates (such as glucose, mannose, or dextrins); proteins (such as serum albumin, gelatin or immunoglobulins); coloring; flavoring and diluting agents; emulsifying agents; hydrophilic polymers (such as polyvinylpyrrolidone); low molecular weight polypeptides; salt-forming counter ions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate 80, triton, tromethamine, lecithin, cholesterol, tyloxapal); stability enhancing agents (sucrose or sorbitol); tonicity enhancing agents (such as alkali metal halides (preferably sodium or potassium chloride, mannitol sorbitol); delivery vehicles; diluents; excipients and/or pharmaceutical adjuvants.

The primary vehicle or carrier in a pharmaceutical composition may be either aqueous or non-aqueous in nature. For example, a suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles. Other exemplary pharmaceutical compositions comprise Tris buffer of about pH 7.0-8.5, or acetate buffer of about pH 4.0-5.5, which may further include sorbitol or a suitable substitute thereof. In one embodiment of the present disclosure, compositions may be prepared for storage by mixing the selected composition having the desired degree of purity with optional formulation agents (Remington's Pharmaceutical Sciences, supra) in the form of a lyophilized cake or an aqueous solution. Further, the therapeutic composition may be formulated as a lyophilizate using appropriate excipients such as sucrose. The optimal pharmaceutical composition will be determined by one of ordinary skill in the art depending upon, for example, the intended route of administration, delivery format, and desired dosage.

When parenteral administration is contemplated, the therapeutic pharmaceutical compositions may be in the form of a pyrogen-free, parenterally acceptable aqueous solution comprising the desired IL-15/IL-15RαSushi complex in a pharmaceutically acceptable vehicle. A particularly suitable vehicle for parenteral injection is sterile distilled water in which a polypeptide is formulated as a sterile, isotonic solution, properly preserved. In various embodiments, pharmaceutical formulations suitable for injectable administration may be formulated in aqueous solutions, preferably in physiologically compatible buffers such as Hanks' solution, Ringer's solution, or physiologically buffered saline. Aqueous injection suspensions may contain substances that increase the viscosity of the suspension, such as sodium carboxymethyl cellulose, sorbitol, or dextran. Additionally, suspensions of the active compounds may be prepared as appropriate oily injection suspensions. Optionally, the suspension may also contain suitable stabilizers or agents to increase the solubility of the compounds and allow for the preparation of highly concentrated solutions.

In various embodiments, the therapeutic pharmaceutical compositions may be formulated for targeted delivery using a colloidal dispersion system. Colloidal dispersion systems include macromolecule complexes, nanocapsules, microspheres, beads, and lipid-based systems including oil-in-water emulsions, micelles, mixed micelles, and liposomes. Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Illustrative phospholipids include egg phosphatidylcholine, dipalmitoylphosphatidylcholine, and distearoylphosphatidylcholine. The targeting of liposomes is also possible based on, for example, organ-specificity, cell-specificity, and organelle-specificity and is known in the art.

In various embodiments, oral administration of the pharmaceutical compositions is contemplated. Pharmaceutical compositions that are administered in this fashion can be formulated with or without those carriers customarily used in the compounding of solid dosage forms such as tablets and capsules. In solid dosage forms for oral administration (capsules, tablets, pills, dragees, powders, granules, and the like), one or more therapeutic compounds of the present disclosure may be mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose, and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like. Liquid dosage forms for oral administration include pharmaceutically acceptable emulsions, microemulsions, solutions, suspensions, syrups, and elixirs. In addition to the active ingredient, the liquid dosage forms may contain inert diluents commonly used in the art, such as water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor, and sesame oils), glycerol, tetrahydrofuryl alcohol, polyethylene glycols and fatty acid esters of sorbitan, and mixtures thereof Besides inert diluents, the oral compositions can also include adjuvants such as wetting agents, emulsifying and suspending agents, sweetening, flavoring, coloring, perfuming, and preservative agents.

In various embodiments, topical administration of the pharmaceutical compositions either to skin or to mucosal membranes, is contemplated. The topical formulations may further include one or more of the wide variety of agents known to be effective as skin or stratum corneum penetration enhancers. Examples of these are 2-pyrrolidone, N-methyl-2-pyrrolidone, dimethylacetamide, dimethylformamide, propylene glycol, methyl or isopropyl alcohol, dimethyl sulfoxide, and azone. Additional agents may further be included to make the formulation cosmetically acceptable. Examples of these are fats, waxes, oils, dyes, fragrances, preservatives, stabilizers, and surface-active agents. Keratolytic agents such as those known in the art may also be included. Examples are salicylic acid and sulfur. Dosage forms for the topical or transdermal administration include powders, sprays, ointments, pastes, creams, lotions, gels, solutions, patches, and inhalants. The active compound may be mixed under sterile conditions with a pharmaceutically acceptable carrier, and with any preservatives, buffers, or propellants which may be required. The ointments, pastes, creams and gels may contain, in addition to a subject compound of the disclosure (e.g., an IL-15/IL-15RαSushi-Fc fusion protein), excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Additional pharmaceutical compositions contemplated for use herein include formulations involving polypeptides in sustained- or controlled-delivery formulations. In various embodiments, pharmaceutical compositions may be formulated in nanoparticles, as slow release hydrogel, or incorporated into oncolytic viruses. Such nanoparticles methods include, e.g., encapsulation in nanoparticles composed of polymers with a hydrophobic backbone and hydrophilic branches as drug carriers, encapsulation in microparticles, insertion into liposomes in emulsions, and conjugation to other molecules. Examples of nanoparticles include mucoadhesive nanoparticles coated with chitosan and Carbopol (Takeuchi et al., Adv. Drug Deliv. Rev. 47(1):39-54, 2001) and nanoparticles containing charged combination polyesters, poly (2-sulfobutyl-vinyl alcohol) and poly (D,L-lactic-co-glycolic acid) (Jung et al., Eur. J. Pharm. Biopharm. 50(1):147-160, 2000). Albumin-based nanoparticle compositions have been developed as a drug delivery system for delivering hydrophobic drugs such as a taxane. See, for example, U.S. Pat. Nos. 5,916,596; 6,506,405; 6,749,868; 6,537,579; 7,820,788; and 7,923,536. Abraxane^{®}, an albumin stabilized nanoparticle formulation of paclitaxel, was approved in the United States in 2005 and subsequently in various other countries for treating metastatic breast cancer.

Techniques for formulating a variety of other sustained- or controlled-delivery means, such as liposome carriers, bio-erodible microparticles or porous beads and depot injections, are also known to those skilled in the art.

An effective amount of a pharmaceutical composition to be employed therapeutically will depend, for example, upon the therapeutic context and objectives. One skilled in the art will appreciate that the appropriate dosage levels for treatment will thus vary depending, in part, upon the molecule delivered, the indication for which the polypeptide is being used, the route of administration, and the size (body weight, body surface or organ size) and condition (the age and general health) of the patient. Accordingly, the clinician may titer the dosage and modify the route of administration to obtain the optimal therapeutic effect. A typical dosage may range from about 0.0001 mg/kg to up to about 100 mg/kg or more, depending on the factors mentioned above. Polypeptide compositions may be preferably injected or administered subcutaneously or intravenously. Long-acting pharmaceutical compositions may be administered every three to four days, every week, biweekly, or monthly depending on the half-life and clearance rate of the particular formulation. The frequency of dosing will depend upon the pharmacokinetic parameters of the polypeptide in the formulation used. Typically, a composition is administered until a dosage is reached that achieves the desired effect. The composition may therefore be administered as a single dose, or as multiple doses (at the same or different concentrations/dosages) over time, or as a continuous infusion. Further refinement of the appropriate dosage is routinely made. Appropriate dosages may be ascertained through use of appropriate dose-response data.

The route of administration of the pharmaceutical composition is in accord with known methods, e.g. orally, through injection by intravenous, intraperitoneal, intratumoral, intracerebral (intra-parenchymal), intracerebroventricular, intramuscular, intra-ocular, intraarterial, intraportal, intralesional routes, intramedullary, intrathecal, intravesicular, intraventricular, transdermal, subcutaneous, or intraperitoneal; as well as intranasal, enteral, topical, sublingual, urethral, vaginal, or rectal means, by sustained release systems or by implantation devices. Where desired, the compositions may be administered by bolus injection or continuously by infusion, or by implantation device. Alternatively, or additionally, the composition may be administered locally via implantation of a membrane, sponge, or another appropriate material on to which the desired molecule has been absorbed or encapsulated. Where an implantation device is used, the device may be implanted into any suitable tissue or organ, and delivery of the desired molecule may be via diffusion, timed-release bolus, or continuous administration.

### Therapeutic Uses

The present disclosure provides for a method of treating cancer cells in a subject, comprising administering to said subject a therapeutically effective amount (either as monotherapy or in a combination therapy regimen) of an IL-15/IL-15RαSushi-Fc or IL-15/IL-15RaSushi-antibody fusion protein of the present disclosure in pharmaceutically acceptable carrier, wherein such administration inhibits the growth and/or proliferation of a cancer cell. Specifically, an IL-15/IL-15RαSushi-Fc fusion protein of the present disclosure is useful in treating disorders characterized as cancer. Such disorders include, but are not limited to solid tumors, such as cancers of the breast, respiratory tract, brain, reproductive organs, digestive tract, urinary tract, eye, liver, skin, head and neck, thyroid, parathyroid and their distant metastases, lymphomas, sarcomas, multiple myeloma and leukemia. Examples of breast cancer include, but are not limited to invasive ductal carcinoma, invasive lobular carcinoma, ductal carcinoma in situ, and lobular carcinoma in situ. Examples of cancers of the respiratory tract include but are not limited to small-cell and non-small-cell lung carcinoma, as well as bronchial adenoma and pleuropulmonary blastoma. Examples of brain cancers include but are not limited to brain stem and hypothalamic glioma, cerebellar and cerebral astrocytoma, neuroblastoma, medulloblastoma, ependymoma, as well as neuroectodermal and pineal tumor. Tumors of the male reproductive organs include but are not limited to prostate and testicular cancer. Tumors of the female reproductive organs include, but are not limited to endometrial, cervical, ovarian, vaginal, and vulvar cancer, as well as sarcoma of the uterus. Tumors of the digestive tract include, but are not limited to anal, colon, colorectal, esophageal, gallbladder, gastric, liver, breast, pancreatic, rectal, small-intestine, and salivary gland cancers. Tumors of the urinary tract include, but are not limited to bladder, penile, kidney, renal pelvis, ureter, and urethral cancers. Eye cancers include but are not limited to intraocular melanoma and retinoblastoma. Examples of liver cancers include but are not limited to hepatocellular carcinoma (liver cell carcinomas with or without fibrolamellar variant), cholangiocarcinoma (intrahepatic bile duct carcinoma), and mixed hepatocellular cholangiocarcinoma. Skin cancers include, but are not limited to squamous cell carcinoma, Kaposi's sarcoma, malignant melanoma, Merkel cell skin cancer, and non-melanoma skin cancer. Head-and-neck cancers include, but are not limited to nasopharyngeal cancer, and lip and oral cavity cancer. Lymphomas include, but are not limited to AIDS-related lymphoma, non-Hodgkin's lymphoma, cutaneous T-cell lymphoma, Hodgkin's disease, and lymphoma of the central nervous system. Sarcomas include but are not limited to sarcoma of the soft tissue, osteosarcoma, malignant fibrous histiocytoma, lymphosarcoma, and rhabdomyosarcoma. Leukemias include, but are not limited to acute myeloid leukemia, acute lymphoblastic leukemia, chronic lymphocytic leukemia, chronic myelogenous leukemia, and hairy cell leukemia. In certain embodiments, the cancer will be a cancer with high expression of TGF-β family member, such as activin A, myostatin, TGF-β and GDF15, e.g., pancreatic cancer, gastric cancer, ovarian cancer, colorectal cancer, melanoma leukemia, lung cancer, prostate cancer, brain cancer, bladder cancer, and head-neck cancer.

The present disclosure provides for a method of treating refractory or resistance liquid or solid tumors through enhancing the therapeutic effect of existing cancer therapeutics as an adjuvant.

The present disclosure also provides for a method of treating viral infection including hepatitis A, hepatitis B, hepatitis C, AIDS in HIV infection, human papillomavirus (HPV) infection, genital warts, etc. in a subject, comprising administering to human patient in need of an IL-15/IL-15RαSushi-Fc or IL-15/IL-15RαSushi-antibody fusion protein of the present disclosure in pharmaceutically acceptable carrier, wherein such administration inhibits the growth and/or replication of virus.

Therapeutically effective amount" or "therapeutically effective dose" refers to that amount of the therapeutic agent being administered which will relieve to some extent one or more of the symptoms of the disorder being treated.

A therapeutically effective dose can be estimated initially from cell culture assays by determining an IC₅₀. A dose can then be formulated in animal models to achieve a circulating plasma concentration range that includes the IC₅₀ as determined in cell culture. Such information can be used to more accurately determine useful doses in humans. Levels in plasma may be measured, for example, by HPLC. The exact composition, route of administration and dosage can be chosen by the individual physician in view of the subject's condition.

Dosage regimens can be adjusted to provide the optimum desired response (e.g., a therapeutic or prophylactic response). For example, a single bolus can be administered, several divided doses (multiple or repeat or maintenance) can be administered over time and the dose can be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the present disclosure will be dictated primarily by the unique characteristics of the antibody and the particular therapeutic or prophylactic effect to be achieved.

Thus, the skilled artisan would appreciate, based upon the disclosure provided herein, that the dose and dosing regimen is adjusted in accordance with methods well-known in the therapeutic arts. That is, the maximum tolerable dose can be readily established, and the effective amount providing a detectable therapeutic benefit to a subject may also be determined, as can the temporal requirements for administering each agent to provide a detectable therapeutic benefit to the subject. Accordingly, while certain dose and administration regimens are exemplified herein, these examples in no way limit the dose and administration regimen that may be provided to a subject in practicing the present disclosure.

It is to be noted that dosage values may vary with the type and severity of the condition to be alleviated and may include single or multiple doses. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition. Further, the dosage regimen with the compositions of this disclosure may be based on a variety of factors, including the type of disease, the age, weight, sex, medical condition of the subject, the severity of the condition, the route of administration, and the particular antibody employed. Thus, the dosage regimen can vary widely, but can be determined routinely using standard methods. For example, doses may be adjusted based on pharmacokinetic or pharmacodynamic parameters, which may include clinical effects such as toxic effects and/or laboratory values. Thus, the present disclosure encompasses intra-subject dose-escalation as determined by the skilled artisan. Determining appropriate dosages and regimens are well-known in the relevant art and would be understood to be encompassed by the skilled artisan once provided the teachings disclosed herein.

An exemplary, non-limiting daily dosing range for a therapeutically or prophylactically effective amount of an IL-15/IL-15RαSushi fusion protein of the disclosure can be 0.0001 to 100 mg/kg, 0.0001 to 90 mg/kg, 0.0001 to 80 mg/kg, 0.0001 to 70 mg/kg, 0.0001 to 60 mg/kg, 0.0001 to 50 mg/kg, 0.0001 to 40 mg/kg, 0.0001 to 30 mg/kg, 0.0001 to 20 mg/kg, 0.0001 to 10 mg/kg, 0.0001 to 5 mg/kg, 0.0001 to 4 mg/kg, 0.0001 to 3 mg/kg, 0.0001 to 2 mg/kg, 0.0001 to 1 mg/kg, 0.0010 to 50 mg/kg, 0.0010 to 40 mg/kg, 0.0010 to 30 mg/kg, 0.0010 to 20 mg/kg, 0.0010 to 10 mg/kg, 0.0010 to 5 mg/kg, 0.0010 to 4 mg/kg, 0.0010 to 3 mg/kg, 0.0010 to 2 mg/kg, 0.0010 to 1 mg/kg, 0.01 to 50 mg/kg, 0.01 to 40 mg/kg, 0.01 to 30 mg/kg, 0.01 to 20 mg/kg, 0.01 to 10 mg/kg, 0.01 to 5 mg/kg, 0.01 to 4 mg/kg, 0.01 to 3 mg/kg, 0.01 to 2 mg/kg, 0.01 to 1 mg/kg, 0.1 to 50 mg/kg, 0.1 to 40 mg/kg, 0.1 to 30 mg/kg, 0.1 to 20 mg/kg, 0.1 to 10 mg/kg, 0.1 to 5 mg/kg, 0.1 to 4 mg/kg, 0.1 to 3 mg/kg, 0.1 to 2 mg/kg, or 0.1 mg/kg, 1 to 50 mg/kg, 1 to 40 mg/kg, 1 to 30 mg/kg, 1 to 20 mg/kg, 1 to 10 mg/kg, 1 to 5 mg/kg, 1 to 4 mg/kg, 1 to 3 mg/kg, 1 to 2 mg/kg, or 1 to 1 mg/kg body weight. It is to be noted that dosage values may vary with the type and severity of the conditions to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

Toxicity and therapeutic index of the pharmaceutical compositions of the disclosure can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effective dose is the therapeutic index and it can be expressed as the ratio LD₅₀/ED₅₀. Compositions that exhibit large therapeutic indices are generally preferred.

The dosing frequency of the administration of the IL-15/IL-15RaSushi fusion protein pharmaceutical composition depends on the nature of the therapy and the particular disease being treated. The subject can be treated at regular intervals, such as weekly or monthly, until a desired therapeutic result is achieved. Exemplary dosing frequencies include, but are not limited to: once or twice a week without break; once or twice a week, every other week; once every 2 weeks; once every 3 weeks; weakly without break for 2 weeks, then monthly; weakly without break for 3 weeks, then monthly; once every other month; once every three months; once every four months; once every five months; or once every six months, or yearly.

### Combination Therapy

As used herein, the terms "co-administration", "co-administered" and "in combination with", referring to an IL-15/IL-15RαSushi fusion protein of the disclosure and one or more other therapeutic agents, is intended to mean, and does refer to and include the following: simultaneous administration of such combination of an IL-15/IL-15RaSushi fusion protein of the disclosure and therapeutic agent(s) to a subject in need of treatment, when such components are formulated together into a single dosage form which releases said components at substantially the same time to said subject; substantially simultaneous administration of such combination of an IL-15/IL-15RαSushi fusion protein of the disclosure and therapeutic agent(s) to a subject in need of treatment, when such components are formulated apart from each other into separate dosage forms which are taken at substantially the same time by said subject, whereupon said components are released at substantially the same time to said subject; sequential administration of such combination of an IL-15/IL-15RaSushi fusion protein of the disclosure and therapeutic agent(s) to a subject in need of treatment, when such components are formulated apart from each other into separate dosage forms which are taken at consecutive times by said subject with a significant time interval between each administration, whereupon said components are released at substantially different times to said subject; and sequential administration of such combination of an IL-15/IL-15RaSushi fusion protein of the disclosure and therapeutic agent(s) to a subject in need of treatment, when such components are formulated together into a single dosage form which releases said components in a controlled manner whereupon they are concurrently, consecutively, and/or overlappingly released at the same and/or different times to said subject, where each part may be administered by either the same or a different route.

In another aspect, the present disclosure provides a method for treating cancer or cancer metastasis in a subject, comprising administering a therapeutically effective amount of the pharmaceutical compositions of the invention in combination with a second therapy, including, but not limited to immunotherapy, cytotoxic chemotherapy, small molecule kinase inhibitor targeted therapy, surgery, radiation therapy, and stem cell transplantation. For example, such methods can be used in prophylactic cancer prevention, prevention of cancer recurrence and metastases after surgery, and as an adjuvant of other conventional cancer therapy. The present disclosure recognizes that the effectiveness of conventional cancer therapies (e.g., chemotherapy, radiation therapy, phototherapy, immunotherapy, and surgery) can be enhanced through the use of the combination methods described herein.

A wide array of conventional compounds has been shown to have anti-neoplastic activities. These compounds have been used as pharmaceutical agents in chemotherapy to shrink solid tumors, prevent metastases and further growth, or decrease the number of malignant T-cells in leukemic or bone marrow malignancies. Although chemotherapy has been effective in treating various types of malignancies, many anti-neoplastic compounds induce undesirable side effects. It has been shown that when two or more different treatments are combined, the treatments may work synergistically and allow reduction of dosage of each of the treatments, thereby reducing the detrimental side effects exerted by each compound at higher dosages. In other instances, malignancies that are refractory to a treatment may respond to a combination therapy of two or more different treatments.

In various embodiments, a second anti-cancer agent, such as a chemotherapeutic agent, will be administered to the patient. The list of exemplary chemotherapeutic agent includes, but is not limited to, daunorubicin, dactinomycin, doxorubicin, bleomycin, mitomycin, nitrogen mustard, chlorambucil, melphalan, cyclophosphamide, 6-mercaptopurine, 6-thioguanine, bendamustine, cytarabine (CA), 5-fluorouracil (5-FU), floxuridine (5-FUdR), methotrexate (MTX), colchicine, vincristine, vinblastine, etoposide, teniposide, cisplatin, carboplatin, oxaliplatin, pentostatin, cladribine, cytarabine, gemcitabine, pralatrexate, mitoxantrone, diethylstilbestrol (DES), fluradabine, ifosfamide, hydroxyureataxanes (such as paclitaxel and doxetaxel) and/or anthracycline antibiotics, as well as combinations of agents such as, but not limited to, DA-EPOCH, CHOP, CVP or FOLFOX. In various embodiments, the dosages of such chemotherapeutic agents include, but is not limited to, about any of 10 mg/m², 20 mg/m², 30 mg/m², 40 mg/m², 50 mg/m², 60 mg/m², 75 mg/m², 80 mg/m², 90 mg/m², 100 mg/m², 120 mg/m², 150 mg/m², 175 mg/m², 200 mg/m², 210 mg/m², 220 mg/m², 230 mg/m², 240 mg/m², 250 mg/m², 260 mg/m², and 300 mg/m².

In various embodiments, the combination therapy methods of the present disclosure may further comprise administering to the subject a therapeutically effective amount of immunotherapy, including, but are not limited to, treatment using depleting antibodies to specific tumor antigens; treatment using antibody-drug conjugates; treatment using agonistic, antagonistic, or blocking antibodies to co-stimulatory or co-inhibitory molecules (immune checkpoints) such as CD276, CD272, CTLA-4, PD-1, PD-L1, CD40, SIRPα, CD47, OX-40, CD137, GITR, LAG3, ICOS, CD27, 4-1BB, TIM-3, B7-H4, Siglec 7, Siglec 8, Siglec 9, Siglec 15 and VISTA; treatment using bispecific T cell engaging antibodies (BiTE^{®}) such as blinatumomab: treatment involving administration of biological response modifiers such as IL-2, IL-7, IL-12, IL-21, GM-CSF, IFN-α, IFN-β and IFN-y; treatment using therapeutic vaccines such as sipuleucel-T; treatment using dendritic cell vaccines, or tumor antigen peptide vaccines; treatment using NK cells; treatment using TCR-T cells; treatment using chimeric antigen receptor (CAR)-T cells; treatment using CAR-NK cells; treatment using iPS induced-NK cells, iPS induced TCR-T cells, iPS induced CAR-T cells or iPS induced CAR-NK cells; treatment using dendric cells; treatment using tumor infiltrating lymphocytes (TILs); treatment using adoptively transferred anti-tumor T cells (ex vivo expanded and/or TCR transgenic); treatment using vaccine such as Bacille Calmette-Guerine (BCG); treatment using TALL-104 cells; and treatment using immunostimulatory agents such as Toll-like receptor (TLR) agonists CpG, and imiquimod; wherein the combination therapy provides increased effector cell killing of tumor cells, i.e., a synergy exists between the IL-15/IL-15RαSushi-Fc fusion proteins and the immunotherapy when co-administered.

In various embodiments, the combination therapy comprises administering an IL-15/IL-15RαSushi-Fc or IL-15/IL-15RaSushi-antibody fusion protein and the second agent composition simultaneously, either in the same pharmaceutical composition or in separate pharmaceutical composition. In various embodiments, an IL-15/IL-15RaSushi fusion protein composition and the second agent composition are administered sequentially, i.e., an IL-15/IL-15RaSushi fusion protein composition is administered either prior to or after the administration of the second agent composition. In various embodiments, the administrations of an IL-15/IL-15RaSushi fusion protein composition and the second agent composition are concurrent, i.e., the administration period of an IL-15/IL-15RaSushi fusion protein composition and the second agent composition overlap with each other. In various embodiments, the administrations of an IL-15/IL-15RaSushi fusion protein composition and the second agent composition are non-concurrent. For example, in various embodiments, the administration of an IL-15/IL-15RaSushi fusion protein composition is terminated before the second agent composition is administered. In various embodiments, the administration second agent composition is terminated before an IL-15/IL-15RaSushi fusion protein composition is administered.

The following examples are offered to more fully illustrate the disclosure but are not construed as limiting the scope thereof.

### Example 1

### Construction, expression, and purification of IL-15/IL-15RαSushi-Fc fusion proteins

All genes were codon optimized for expression in mammalian cells, which were synthesized and subcloned into the recipient mammalian expression vector (GenScript). Protein expression is driven by an CMV promoter and a synthetic SV40 polyA signal sequence is present at the 3' end of the CDS. A leader sequence has been engineered at the N-terminus of the constructs to ensure appropriate signaling and processing for secretion.

The fusion proteins were produced by co-transfecting HEK293-F cells growing in suspension with the mammalian expression vectors using polyethylenimine (PEI, 25,000 MW linear, Polysciences). If there were two or more expression vectors, the vectors will be transfected with a 1: 1 ratio. For transfection, HEK293 cells were cultivated in serum-free Freestyle^{™} 293 Expression Medium (ThermoFisher). For production in 1000 ml shaking flasks (maximum working volume 330 mL), HEK293 cells at density of 0.8 × 10⁶ cells/ml were seeded 24 hours before transfection. Expression vectors to a total amount of 330 µg DNA were mixed with 16.7 ml Opti-mem Medium (ThermoFisher). After addition of 0.33 mg PE! diluted in 16.7 ml Opti-mem Medium, the mixture was vortexed for 15 seconds and subsequently incubated for 10 min at room temperature. The DNA/PEI solution was then added to the cells and incubated at 37°C in an incubator with 8% CO₂ atmosphere. Sodium butyrate (Millipore Sigma) at the final concentration of 2 mg/L was added to the cell culture on day 4 to help sustain protein expression. After 6 days cultivation, supernatant was collected for purification by centrifugation for 20 min at 2200 rpm. The solution was sterile filtered (0.22 µm filter, Corning).

Alternatively, the constructs were produced in ExpiCHO cells (ThermoFisher) following manufacturer's instructions.

The secreted protein was purified from cell culture supernatants using Protein A affinity chromatography. Cell culture supernatant was loaded onto a HiTrap MabSelect SuRe 5 ml column (GE Healthcare) equilibrated with 5 column volumes (CV) of phosphate buffered saline, pH 7.2 (ThermoFisher). Unbound protein was removed by washing with 5 CVs of PBS pH 7.2, and target protein was eluted with 25 mM sodium citrate, 25 mM sodium chloride, pH 3.2. Protein solution was neutralized by adding 3% of 1 M Tris pH 10.2. Target protein was concentrated and buffer exchanged to PBS, pH 7.2 using Amicon^{®} Ultra-15 Ultracel 10K (Merck Millipore)

Purity and molecular weight of the purified molecules were analyzed by SDS-PAGE in the presence and absence of a reducing agent and staining with Coomassie (Imperial^{™} protein stain, ThermoFisher). The NuPAGE^{®} Pre-Cast gel system (4-12% Bis-Tris, ThermoFisher) was used according to the manufacturer's instruction. The aggregate content of the molecules was analyzed on an Agilent 1200 high-performance liquid chromatography (HPLC) system. Samples were injected onto an AdvanceBio size-exclusion column (300Å, 4.6 × 150 mm, 2.7 µm, LC column, Agilent) using 150 mM sodium phosphate, pH 7.0 as the mobile phase at 25 °C.

The protein concentration of purified protein samples was determined by measuring the absorbance at 280 nm using a Nanodrop spectrophotometer (ThermoFisher) divided by the molar extinction coefficient calculated based on the amino acid sequence. Endotoxin level of purified protein samples were measured using Endosafe nexgen-PTS (Charles River) according to the manufacturer's instruction.

As an example to demonstrate the protein profile of isolated IL-15/IL-15Rα Fc fusion constructs, SDS-PAGE analyses of P-0217, P-0234, and P-0313 are shown in FIG. 2A. P-0217, P-0234, and P-0313 are all IL-15/IL-15Rα (non-covalent)-Fc fusion proteins comprising IL-15/IL-15Rα complex at the C-terminus. P-0217 is a monovalent IL-15/IL-15Rα (non-covalent) Fc fusion, P-0234 is the dimeric counterpart of P-0217, and P-0313 shares the same fusion configuration as P-0234 but differs only with S58D substitution in the IL-15 domain.

The IL-15Rα sushi+ domain with a calculated molecular weight of 8.6 kDa was non-covalently associated with IL-15 or IL-15 variant fused to Fc domain for both monovalent and bivalent Fc fusions, and it was dissociated under denaturing conditions and migrated to the expected position as a sharp band (FIG. 2A). The presence of IL-15Rα-sushi+ band on the gel confirmed the non-covalent association between IL-15 and IL-15Rα during cell culture growth; such association was maintained during Protein A purification under low-pH elution conditions.

Size exclusion chromatogram in FIG. 2B indicated low aggregation propensity for the two fusion formats as only 1-2% aggregation was present for all three fusion proteins after the initial protein A capture step without further polishing. The sharp main peak further suggests the tight albeit non-covalent association of IL-15 and I L-15Ra under native buffer conditions.

Further, the expression level of the fusion proteins was comparable (within 2-fold difference) to that of Fc-only protein under the same vector and culturing conditions. The high yield and low aggregation propensity demonstrated favorable developability profile of both monovalent and bivalent of IL-15/IL-15Rα (non-covalent) Fc fusion proteins. Additionally, amino acid substitution in IL-15, exemplified by P-0313, did not impact expression profile of the fusion protein; P-0313 exhibited almost identical purity and aggregation propensity as its wild type equivalent P-0234 (FIG. 2).

### Example 2

### Purity-focused developability assessment of different fusion protein formats underscored the role of properly complexed IL-15Rα domain in enhancing fusion protein developability profile

SEC analysis of the protein A purified samples was used to assess the impact of different fusion formats on protein aggregation propensity and purity. It was the observation of the inventors that while protein expression level may vary between different batches due to cell growth variations, protein aggregation propensity and purity seemed to be an intrinsic property associated with a particular protein with little variance from lot to lot, as exemplified by FIG. 3F and 3H for P-0234.

First, the impact of complexation of IL-15Rα on protein purity of IL-15-Fc fusion proteins were evaluated based on 5 molecules. P-0162 is a C-terminal monomeric IL-15 alone Fc fusion protein containing a Hole-Fc-Linker1-IL-15 chain (SEQ ID NO: 13) and an empty Knob-Fc chain (SEQ ID NO: 7). P-0197 is a C-terminal monovalent IL-15/IL-15Rα (non-covalent) Fc fusion with its schematic diagram depicted in FIG. 1B. P-0197 differs from P-0162 by the presence of a free IL-15RαSushi+ domain that is non-covalently complexed with IL-15. P-0153 is a N-terminal monomeric IL-15/IL-15Rα fusion with the heterodimeric Fc fusion format with its cartoon diagram shown in FIG. 1A. P-0167 (SEQ ID NOs: 13 and 55) and P-0198 are the dimer counterparts of P-0162 and P-0197, respectively. The size exclusion diagrams of the 5 molecules are illustrated in FIG. 3A-3E.

As seen in FIG. 3A, IL-15-Fc monomeric fusion without IL-15Rα has a monomer content of 84.5% and the majority of impurities are of lower molecular weights. In contrast, P-0197 contains 98.6% monomer content (FIG. 3B), and such significant improvement in purity was apparently contributed from the free I L-15RαSushi+ domain. The effect of free IL-15RαSushi+ domain on the protein quality of IL-15-Fc fusion proteins were further highlighted in the dimeric formats, which is depicted with P-0167 and P-0198 SEC chromatograms in FIGS 3D and 3E, respectively. P-0167 contains a broad and irregular peak with shoulders on both sides of the main peak. More notably, P-0167 exhibited significantly compromised ex vivo activity in activating NK and T cells of fresh human PBMC, which was likely due to the incorrect folding of the protein. However, P-0198, which contains a non-covalently bound IL-15RαSushi+ domain in dimeric form, demonstrated a sharp main peak with 90.5 % monomer content (FIG. 3E). Somewhat intriguing, when the IL-15RαSushi+ domain was not free, but covalently fused to a matching heterodimeric Fc as in P-0153, its complexation with IL-15-Fc did not yield any improvement in protein purity; rather, the protein sample contains >25% dimer and higher molecular weight soluble aggregates (FIG. 3C). Fusion of both IL-15 and IL-15Rα to Fc domains likely created spatial constrains that prevented them from interacting in the physiological way. In summary, IL-15RαSushi+ domain can significantly improve IL-15-Fc fusion protein purity and biophysical property, but only when IL-15Rα domain can associate with IL-15 in a favorable and unconstrained manner.

Second, the effect of IL-15RαECD (SEQ ID NO: 4) vs. IL-15RαSushi+ (SEQ ID NO: 5) on the developability of IL-15/IL-15Rα-Fc fusion proteins were evaluated by comparing P-0234 and P-0220. Both constructs are C-terminal Fc fusion sharing the same configuration as depicted in Figure 1C with IL-15RαSushi+ domain in P-0234, and IL-15RαECD in P-0220. Their SEC chromatograms are shown in FIGS. 3F and 3G, respectively. P-0220, the protein comprising IL-15RαECD domain, not only has a lower purity than its IL-15RαSushi+-containing counterpart P-0234 (88.5% vs. 100%), but also expressed at a 2.5-fold lower level in the same batch of cells. In short, it is evident that IL-15RαSushi+ domain is a preferred partner over IL-15RαECD in constructing more developable IL-15/IL-15Rα fusion proteins.

Further, the impact of fusion terminus on the developability of IL-15/IL-15Rα-Fc fusion proteins was evaluated. P-0234 and P-0223 are dimeric IL-15/IL-15Rα (non-covalent) Fc fusion proteins with the IL-15/IL-15RαSushi complex attached to the C-terminus and N-terminus of Fc, respectively. Their SEC chromatograms (FIG. 3H and 3I) show rather subtle difference in purity (98.5% vs. 93.9%). However purified P-0223 consistently contains a broad peak of higher molecular weight species and a small but appreciable peak containing lower molecular impurities, which were virtually absent in P-0234. Despite the small differences, P-0234 with C-terminal fusion has unarguably better SEC purity profile and is the preferred format from developability point of view considering that both molecules expressed at a comparable level.

In conclusion, complexing IL-15Rα subunit with IL-15-Fc fusion can significantly improve expression, purity, and reduce aggregation; and such improvement requires proper IL-15Rα association with IL-15 with minimal spatial constrains. And IL-15RαSushi+, the truncated and functional version of IL-15Rα ECD, appeared to be preferred over the full length ECD based on both productivity and purity assessment. Further, placing IL-15/IL-15Rα complex to the C-terminus of Fc is advantageous over N-terminus fusion to achieve high purity. Consequently, the dimeric IL-15/IL-15Rα (non-covalent) C-terminal Fc fusion format, exemplified by P-0234, integrates all the preferable components and represents the superior format.

### Example 3

### Non-covalent association of IL-15Rα enhances receptor binding and biological activities of IL-15/IL-15Rα Fc fusion proteins

IL-15 binds to its specific receptor IL-15Rα with high affinity and both are expressed on antigen presenting cells. The association of IL-15Rα with IL-15 trans-presents IL-15 to a heterodimeric receptor complex composed of IL-15Rβ and γ_{c} on the responding lymphocytes, including NK, T and B cells. The formation of the ligand-trimeric receptor complex (IL-15-IL-15Rαβγ) initiates intracellular signaling cascades leading to downstream biological effects. The complexity of the IL-15 receptor biology eludes challenges to engineer an optimal IL-15 fusion protein that enables a conformationally effective ligand-trimeric receptor signaling complex.

We hypothesized that the covalent linkage of IL-15 to the Fc portion of the human IgG either at the N-terminus or C-terminus would be more advantageous for *in vivo* half-life extension than IL-15 in non-covalent association with an IL-15Rα Fc fusion protein. We further postulated that IL-15Rα domain is required for IL-15 Fc fusion protein to enhance its interaction with the intermediate affinity IL-2Rβγ receptor and facilitate the formation of high affinity ligand-trimeric receptor signaling complex. Furthermore, we believe that the non-covalent association of IL-15Rα domain with the IL-15 Fc fusion protein retains a natural IL-15 and IL-15Ra association and is an optimal conformation for IL-15 trans-presentation. Moreover, we hypothesized it is feasible to produce such a fusion protein complex due to the extremely high binding affinity between IL-15 and IL-15Rα domain.

Different formats of IL-15 Fc fusion proteins containing IL-15/IL-15Rα domain complex in various configurations were constructed. The binding activity of the fusion proteins to IL-15Rβ subunit was determined and their biological activity in stimulating lymphocyte activation was analyzed by measuring CD69 expression on human CD8 and NK cells. The exemplary structural diagrams of the fusion proteins are shown in FIG 1.

The binding activity was tested in ELISA assay. Briefly, Nunc Maxisorp (ThermoFisher) plates were coated overnight at 4°C with huIL-15R*β*-6His at 1 *µ*g/well (100 *µ*l/well) in bicarbonate buffer pH 9.4 (ThermoFisher). After washing 3 times with PBS/0.05% Tween20, plates were incubated with SuperBlock (300 *µ*l/well) at room temperature for 2 hrs. to block nonspecific binding. After washing, IL-15 compounds each at 3-fold serial dilutions with blocking buffer were added to the plates (100 *µ*l/well) and incubated at room temperature for 1 hr. After washing, the Fc fusions were detected by 1 hr. incubation at room temperature with a goat anti-human IgG Fc secondary antibody conjugated with horseradish peroxidase (HRP) at 1:5000 dilution in blocking buffer (ThermoFisher) (100 *µ*l/well). After washing, TMB substrate (ThermoFisher) was added (100ul/well). Plates were sealed and incubated 5-20min at room temperature in dark. Reaction was stopped by adding 2N sulfuric acid (Ricca Chemical) (50uL/well) and absorbance was read at 450-590 nm.

The biological activity was determined by measuring the induction of CD69 expression on human NK and CD8 T cells. CD69 is a cell surface glycoprotein that is induced early during lymphocyte activation. An ex vivo human peripheral blood mononuclear cell (PBMC) assay was established to analyze the number/percent of NK or CD8 T cells expressing CD69 following IL-15 treatment. Specifically, human PBMCs were isolated by Ficoll-Hypaque centrifugation from the buffy coat purchased from Blood Oklahoma Institute. Purified human PBMCs were treated with serial dilutions of each IL-15 test compound and incubated at 37 °C for 48 hours. Cells were collected by 300 xg centrifugation and resuspended in FACS buffer. After blocking Fc receptor by adding human TruStain FcX (1:50 dilution), cells were stained with anti-human CD56-FITC, anti-human CD69-PE and anti-human CD8-APC antibodies (1:50 dilution). After 30-minutes incubation with the antibodies at room temperature, cells were collected and washed, resuspended in FACS buffer and ready for the flow cytometric analysis. CD69 expression was determined on CD56+ NK and CD8+ T cells and data are expressed as % of CD69 positive cells in gated population.

P-0157 is an N-terminal bivalent IL-15 (non-covalent)/IL-15RaSushi Fc fusion protein; P-0153 is a C-terminal IL-15/IL-15RaSushi heterodimeric Fc fusion protein; P-0162 is a C-terminal monovalent Fc-IL-15 fusion protein without IL-15RaSushi complexed. An ELISA binding assay indicated that the incorporation of IL-15RaSushi domain significantly increased the binding strength of the IL-15 Fcfusion proteins (P-0157 & P0153) to the IL-15Rβ as compared to the fusion protein without the IL-15Rα complexed (P-0162) (FIG. 4), suggesting the essential role of IL-15Rα in facilitating the fusion proteins' interaction with the receptor. Furthermore, the receptor binding activity was reduced when both IL-15 and IL-15Rα were covalently conjugated to the Fc in a heterodimeric form (P-0153) as opposed to IL-15 bounded non-covalently to IL-15Rα Fc fusion protein (P-0157) (FIG. 4), suggesting a conformationally constrained fusion format would negatively affect the receptor binding activity.

Consistent with the binding potency, the incorporation of IL-15Rα also increased the biological activity of the IL-15 fusion protein as compared to the fusion protein without IL-15Rα. P-0197 is a C-terminal monovalent IL-15/IL-15Rα (non-covalent) Fc fusion protein and P-0162 share the same structure as P-0197 without IL-15Rα sushi included. P-0197 demonstrated 10-fold and 6-fold increased potency in induction of CD69 positive NK cells (FIG. 5A) and CD8 T cells (FIG. 5B) compared to P-0162, respectively, attributed to the inclusion of IL-15Rα.

Lastly, the biological activity of three C-terminal monovalent IL-15/IL-15Rα Fc fusion proteins was compared. P-0165 is an Fc fusion protein with IL-15Rα fused to the Fc domain and IL-15 non-covalently bound; P-0197 is configured reversely with IL-15 fused to the Fc domain and IL-15Rα non-covalently bound; P-0153 has a heterodimeric structure with both IL-15 and IL-15Rα fused to the Fc domain. As shown in FIG. 6, the fusion proteins with non-covalent complexation with either IL-15 or IL-15Rα demonstrated better potency in induction of CD69 positive NK (FIG. 6A) and CD8 T cells (FIG. 6B) than the fusion protein with both IL-15/IL-15Rα covalently and heterodimerically fused to Fc. Data suggest a conformationally optimal association between IL-15 and IL-15Rα is critical for IL-15 fusion protein to bind its receptor and exert its biological activity. Increased conformational constrains, e.g. the heterodimeric Fc fusion format, negatively affect the biological activity.

### Example 4

### Effect of linker on the activity of IL-15/IL-15Rα Fc fusion proteins

Selection of a suitable linker to join the protein domains is critical in fusion protein engineering. The peptide linker not only provides a spatial distance between the fusion protein domains and allows their independent folding, but also directly affects the structural stability and functional property of the fusion proteins. Here we examine the effect of flexibility and length of a linker on the biological activity of IL-15/IL-15Rα Fc fusion proteins.

The biological activity was determined by measuring the induction of CD69 expression on human NK and CD8 T cells in an ex vivo human PBMC FACS-based assays as described previously. P-0165 and P-0166 are monomeric IL-15 (non-covalent)/IL-15Rα Fc fusions with a 10 amino acid rigid and flexible linker, respectively. P-0197, P-0207 and P-0217 are all monovalent IL-15/IL-15Rα (non-covalent) Fc fusion proteins. P-0197 comprises a rigid linker, P-0207 contains a 10-amino acid GS rich flexible linker, and P-0217 has a 15-amino acid flexible linker. Results (FIG. 7A & 7B) indicated that the rigidity or length of the peptide linker joining the Fc and IL-15 (P-0165 & P-0166) or Fc and IL-15Ra (P-0197, P-0207 and P-0217) didn't affect the biological activity of the fusion proteins tested.

### Example 5

### Effect of valency on the activity of IL-15/IL-15Rα fusion proteins

Fc fusion proteins for therapeutic use are mostly homodimeric because the IgG1 Fc naturally homodimerizes due to the disulfide bonds formed in the hinge region. The dimeric protein has advantages in avidity, stability, quantity, size and function. However, Fc engineering could generate monomeric Fc fusion proteins. The alteration could affect the biological activity, pharmacokinetics, side effects, or reduce the size of dimeric protein for tissue penetration. To evaluate the effect of valency on the biological activity of IL-15/IL-15Rα Fc fusion proteins, both monomeric and homodimeric forms with different IL-15/IL-15Rα Fc fusion configurations were constructed and biological activity tested.

The biological activity was determined by measuring the induction of CD69 expression on human NK and CD8 T cells in an ex vivo human PBMC FACS-based assays as described previously. Results indicated that the homodimeric form of IL-15/IL-15Ra Fc fusion proteins showed approximately 2-fold enhancement in the biological activity compared to the respective monomeric counterpart across all the fusion formats tested (Table 4), suggesting the dimeric valency may offer advantages in functionality.

**Table 4**

| Effect of the IL-15/IL-15RαSushi complex valency on the biological activity of IL-15/IL-15Rα fusion proteins | | | |
|---|---|---|---|
| Protein ID | Description | activation EC₅₀ (pM) | |
| | | CD8⁺ T cell | CD56⁺ NK |
| P-0166 | IL-15 (non-covalent)/IL-15Ra Fc monomer, C-terminal | 17.4 | 5.9 |
| P-0218 | Dimeric counterpart of P-0166 | 10 | 3.1 |
| P-0217 | IL-15/IL-15Ra (non-covalent) Fc monomer, C-terminal | 139 | 28.3 |
| P-0234 | Dimeric counterpart of P-0217 | 45.8 | 4.1 |
| P-0197 | IL-15/IL-15Ra (non-covalent) Fc monomer, C-terminal, rigid linker | 498 | X |
| P-0198 | Dimeric counterpart of P-0197 | 293 | X |
| P-0221 | IL-15/IL-15Ra (non-covalent) Fc monomer, N-terminal | 50.3 | 12.5 |
| P-0223 | Dimeric counterpart of P-0221 | 47.2 | 9.1 |

### Example 6

### Effect of N- or C-terminal Fc fusion on the biological activity of IL-15

Fc fusion protein can be constructed by placing IL-15/IL-15Rα complex to either the N- or C-terminus of Fc connected by a spacing linker. The optimal scaffold is determined by whether the fusion protein is folded and expressed correctly, and whether the biological activity is retained. The IL-15/IL-15Rα Fc fusion proteins were generated by attaching the IL-15/IL-15Rα complex to either the N- or C-terminus of Fc spaced by a linker. The IL-15/IL-15Rα complex was also configured differently in the context of the C- and N-terminal fusions. P-0218 is the C-terminal bivalent IL-15 (non-covalent)/IL-15Rα Fc fusion protein, and the benchmark is the N-terminal counterpart of P-0218 additionally harboring N72D substitution in IL-15. P-0234 is the C-terminal bivalent IL-15/IL-15Rα (non-covalent) Fc fusion protein, and P-0223 is the N-terminal counterpart of P-0234.

The biological activity of the fusion proteins was determined by measuring Ki67 expression in the nucleus of NK and CD8 T cells following IL-15 compound treatment. IL-15 is a potent lymphocyte growth factor that stimulates NK, T and B cell proliferation and differentiation. Ki67 is a nuclear protein induced in all active phases of cell cycle (G1, S, G2 and M), but not in quiescent phase (G0) and therefore is a marker for cell proliferation.

An ex vivo human PBMC assay was established. Briefly, purified human PBMCs were treated with serial dilutions of IL-15 test compounds and incubated at 37 °C for 5 days. On Day 5, cells were washed once with FACS buffer (1% FBS/PBS) and first stained with Fc-blocker and surface marker antibodies, including anti-human CD56-FITC, anti-human CD8-APC and anti-human CD4-Percp-cy5.5 (1:50 dilution). After 30-minutes incubation and wash, cell pellets were fully resuspended by 200µl/well of 1x Foxp3 fixation & permeabilization working solution and incubated for 30-minutes at room temperature in dark. After centrifugation, 200 µl of 1x permeabilization buffer were added to each well for another wash. Cell pellets were resuspended in permeabilization buffer with anti-human Ki67-PE (1:10 dilution). After 30-minutes incubation at room temperature, cells were collected and washed, resuspended in FACS buffer and ready for the flow cytometric analysis. Data are expressed as % of Ki67 positive cells in gated population.

As shown in FIG. 8, the C-terminal Fc fusions (P-0218 and P-0234) consistently demonstrated stronger induction of Ki67 positive CD8 T cells or CD8 T cell proliferation than the N-terminal Fc fusion counterparts (the benchmark and P-0223) (FIG. 8A and 8B). Data suggest that the C-terminus of Fc is a preferred site for linkage of IL-15/IL-15Rα complex and allows preservation of the biological activity.

### Example 7

### Effect of receptor-alpha domain selection on the activity of IL-15/IL-15Rα Fc fusion proteins

IL-15 binds to the extracellular domain (ECD) of IL-15Rα and the binding is mainly contributed to a conserved protein binding motif called sushi-domain. For construction of a fusion protein, the short and truncated version of IL-15Rα may be desirable to reduce the size and structural complexity. To ensure the binding specificity and affinity, the full or part (the Sushi domain with additional 12 AA) of the ECD domain conferring the binding of IL-15 was constructed into the fusion proteins and the functional activity was determined.

P-0234 and P-0220 are the C-terminal dimeric IL-15/IL-15Rα (non-covalent) fusion proteins, where IL-15Rα is sushi and full ECD, respectively. P-0223 and P-0224 are N-terminal dimeric IL-15/IL-15Rα (non-covalent) Fc fusion proteins, where IL-15Rα is sushi and full ECD, respectively. P-0221 and P-0222 are N-terminal monovalent IL-15/IL-15Rα (non-covalent) Fc fusion proteins, where IL-15Rα is sushi and full ECD, respectively. Results indicated that the fusion proteins non-covalently complexed with the IL-15Rα sushi domain were more potent than those complexed with IL-15Rα full ECD in inducing CD69 positive NK cells, irrespective of the fusion format as N-terminal or C-terminal, dimeric or monomeric (FIG. 9). Data suggest the IL-15Rα sushi domain is more desirable than the full ECD to construct IL-15/IL-15Ra Fc fusion proteins and to confer an optimal conformation for IL-15 to interact with the signaling receptors.

### Example 8

### IL-15 mutants and the binding activity of the variant fusion proteins to IL-15Rβ

In searching for IL-15 agonist, super-agonist or antagonist, deletion, insertion or point mutations were introduced into the human IL-15 peptide sequence at the contact interface between IL-15 and receptor beta or gamma. The variants were introduced into different formats of the IL-15/IL-15Rα Fc fusion proteins and the binding activity to IL-15Rβ was quantified by Enzyme-linked Immunosorbent Assays (ELISAs) as described previously.

Table 5 shows the IL-15Rβ binding activity of the C-terminal IL-15 variant/IL-15Rα heterodimeric Fc fusion proteins. The IL-15 variants have amino acid deletion, insertion or point mutations introduced in the human IL-15 peptide. The truncation of 3 amino acids from the C-terminus of IL-15 retained the binding activity of the fusion protein to IL-15Rβ as compared to the full-length wide type IL-15 fusion protein, while further truncation of 6 or 9 amino acids from the C-terminus of IL-15 led to gradual reductions in the binding activity of the fusion proteins. GS insertion with various lengths after N95 all resulted in reductions of the IL-15Rβ binding activity of the fusion proteins.

Table 6 shows the IL-15Rβ binding activity of the monomeric IL-15 variant (non-covalent) /IL-15Ra Fc fusion proteins, where the human IL-15 domain contains single amino acid substitution at positions 58, 62, 63, 67 or 68. P-0185, the Fc fusion protein containing IL-15 (I67V) variant, demonstrated a similar binding activity to IL-15Rβ as the wild type fusion protein. P-0182, a fusion protein containing an IL-15 variant having an amino acid substitution at position 58 from serine to aspartic acid (S58D), demonstrated 4-fold enhancement in the binding potency to IL-15Rβ compared to wild type fusion protein. The substitutions at positions 62, 63 and 68 of the IL-15 resulted in different degrees of reductions in IL-15Rβ binding activity of the fusion proteins.

Table 7 shows the IL-15Rβ binding activity of the dimeric IL-15 variant /IL-15Ra (non-covalent) Fc fusion proteins, where the human IL-15 contains single amino acid substitution at positions 58 or 68 or amino acid insertion after N95. Similarly, as seen in P-0182 (Table 4), P-0313, which contains the same IL-15 variant with S58D, demonstrated 2-fold enhancement in the binding potency to IL-15Rβ as compared to its respective wild type fusion protein P-0234. The data strengthened the notion that the S58D substitution in the IL-15 peptide may tune IL-15 into a super-agonist attributed to the enhanced receptor binding activity.

In summary, IL-15 variant Fc fusion proteins were created and identified with differential IL-15Rβ binding activities. Some of the IL-15 variants exhibited reduced potency to bind to IL-15Rβ as compared to their wildtype counterparts (Tables 5-7). Some variants, such as P-0173 andP-0185, retained the binding activity to IL-15Rβ similarly as the wild-type (Table 5). A Single point mutation with the substitution of serine at position 58 to aspartic acid in the human IL-15 domain offered enhanced binding activity of the fusion proteins (P-0182 & P-0313) to IL-15Rβ (Tables 6 & 7).

**Table 5**

| IL-15Rβ binding activity of the IL-15 variant /IL-15Rα heterodimeric Fc proteins | | | |
|---|---|---|---|
| Protein ID | IL-15/IL-15Rα heterodimeric | ELISA binding | |
| | Fc fusion format | EC₅₀ (nM) | Fold change |
| P-0153 | IL-15 wild type | 0.65 | 1 |
| P-0173 | IL-15d1 (111-114 deletion) | 0.66 | 1 |
| P-0174 | IL-15d2 (108-114 deletion) | 4.54 | 7 |
| P-0175 | IL-15d3 (105-114 deletion) | 23.1 | 36 |
| P-0176 | IL-15i1 (GS insertion after N95) | 10.2 | 16 |
| P-0177 | IL-15i2 (GGSGG insertion after N95) | 38.0 | 59 |
| P-0178 | IL-15i3 (GSSGGSGGS insertion after N95) | 23.1 | 36 |

**Table 6**

| IL-15Rβ binding activity of the monomeric IL-15 variant (non-covalent) ZIL-15Ra Fc fusion proteins | | | |
|---|---|---|---|
| Protein ID | Monovalent IL-15 (non-covalent)/ IL-15Rα Fc fusion format | ELISA binding EC₅₀ (nM) | Fold change |
| P-0165 | IL-15 wild type | 1.24 | 1 |
| P-0182 | IL-15S58D | 0.31 | 0.25 |
| P-0183 | IL-15 T62D | 6.29 | 5 |
| P-0184 | IL-15 V63F | 15.6 | 13 |
| P-0185 | IL-15 I67V | 1.96 | 1.6 |
| P-0186 | IL-15 I68F | 106 | 86 |

**Table 7**

| IL-15Rβ binding activity of the dimeric IL-15 variant /IL-15Rα (non-covalent) Fc fusion proteins | | | |
|---|---|---|---|
| Protein ID | Bivalent IL-15 /IL-15Rα (non-covalent) Fc fusion format | ELISA binding EC₅₀ (nM) | Fold change |
| P-0234 | IL-15 wild type | 0.83 | 1 |
| P-0313 | IL-15-(S58D) | 0.36 | 0.43 |
| P-0361 | IL-15i1 ('GS' insertion after N95) | 7.66 | 9 |

### Example 9

### T cell activation activity of IL-15 S58D variant /IL-15Rα Fc fusion proteins

The Fc fusion proteins of IL-15 variant /IL-15Rα complex were evaluated for their functional activity in stimulating lymphocyte activation. An ex vivo human PBMC assay was established to analyze the number/percent of CD8 T cells expressing CD69, a lymphocyte activation marker, as previously described.

P-0234 is a C-terminal dimeric IL-15/IL-15Rα sushi (non-covalent) Fc fusion protein optimized by a combination of preferred configurations, including covalent IL-15 linkage to Fc, C-terminal fusion, dimeric valency and non-covalent IL-15Rα sushi complexation. P-0313 is the S58D counterpart of P-0234. It shares the same fusion configuration as P-0234 but differs only with S58D substitution in the IL-15 polypeptide. P-0313 demonstrated increased IL-15Rβ binding activity compared to the wild type counterpart P-0234 previously (Table 7). Consistent with the enhanced binding activity to IL-15Rβ, the variant P-0313 harboring the S58D mutation also demonstrated an increased potency in inducing CD69 positive T cells (7 pM vs 12 pM for P-0313 and P-0234, respectively), confirming P-0313 exhibits super-agonist activity.

### Example 10

### The signaling activity of IL-15 S58D/IL-15Ra Fc fusion proteins

The IL-15 S58D variant demonstrated increased binding activity to IL-15Rβ and enhanced potency in stimulating CD69 positive lymphocytes. The current study examined the signaling activity of IL-15 S58D/IL-15Ra fusion proteins in stimulating intracellular phosphorylation of the signal transducer and activator of transcription 5 (pSTAT5) in NK and T cells.

STAT5 phosphorylation was determined by intracellular FACS analysis in an ex vivo human PBMC assay following IL-15 compound treatment. Briefly, purified human PBMCs were treated with serial dilutions of IL-15 test compounds and incubated at 37°C for 15 minutes. At the end of the treatment, cells were washed once with FACS buffer (1% FBS/PBS) and incubated in 150 µl/well of pre-warmed Cytofix fixation buffer at 37°C for 15 minutes. Fixed cells were washed again and resuspended in 150 µl/well pre-cooled Perm buffer II at 4°C for 30 minutes. After blocking Fc receptor by adding human TruStain FcX (1:50 dilution), cells were stained with anti-human CD56-FITC, anti-human pSTAT5-PE, anti-human CD8-APC and anti-human CD4-Percp-cy5.5 (1:50 dilution). After 45-minutes incubation with the antibodies at room temperature, cells were collected and washed, resuspended in FACS buffer and ready for the flow cytometric analysis. Data are expressed as % of pSTAT5 positive cells in gated population.

The S58D mutation was introduced into two formats of the Fc fusion proteins: the bivalent IL-15 (non-covalent)/IL-15Ra Fc fusion and the bivalent IL-15/IL-15Ra (non-covalent) Fc fusion proteins. P-0218 and P-0314 are the C-terminal dimeric IL-15 (non-covalent)/IL-15Ra Fc fusion proteins containing wild type and S58D variant IL-15, respectively. P-0234 and P-0313 are C-terminal dimeric IL-15/IL-15Rα (non-covalent) Fc fusion proteins containing wild type and S58D variant IL-15, respectively. Regardless of the fusion configurations, the IL-15(S58D) variant fusion proteins (P-0314 and P-0313) demonstrated about 2-fold increase in potency to stimulate STAT5 phosphorylation as compared to their respective wide-type fusion proteins (P-0218 and P-0234) in CD8 (FIG. 10A), CD4 (FIG. 10B) T cells, as well as NK cells (FIG. 10C). Data confirmed the S58D substitution in the IL-15 peptide leads to a super-agonist activity of various IL-15/IL-15Rα fusion proteins.

### Example 11

### The cell proliferation activity of IL-15S58D /IL-15-Ra Fc fusion proteins

Following observed increases in the ability to bind to IL-15Rβ, stimulate STAT5 phosphorylation and induce CD69 expression, the IL-15S58D variant Fc fusion proteins were tested for their ability to stimulate cell proliferation in comparison with the wild type fusion proteins by measuring Ki67 expression in NK and CD8 T cells. Human PBMC were treated with increasing doses of IL-15 fusion molecules and Ki67 expression was determined by intracellular FACS analysis gated on CD56+ NK and CD8+ T cell populations as previously described.

Similar to what were observed for STAT5 phosphorylation as shown in (FIG.11), the fusion proteins of the IL-15 S58D variant also demonstrated 2-fold increase in potency to stimulate Ki67 expression as compared to the wild types in CD8+ (FIG. 11A) and CD4+ (FIG. 11B) T cells, as well as CD56+ NK cells (FIG. 11C). These data strengthened that the introduction of S58D mutation in the IL-15 domain provides enhancement in a range of biological activities, including receptor binding, intracellular signaling, activation of cell surface marker and cell proliferation.

### Example 12

### A four-day repeated dosing study with P-0234 in comparison with rhlL-15 and benchmark in mice

The IL-15/IL-15Rα Fc fusion proteins demonstrated strong abilities to bind to IL-15Rβ, induce intracellular signaling cascade and stimulate proliferation of NK and CD8 T lymphocytes in vitro and ex vivo. Here we examined the serum exposure and the effect of various IL-15 compounds on NK cell proliferation and expansion in mice. The tested proteins include recombinant human native IL-15 (rhIL-15), P-0234 (a C-terminal bivalent IL-15/IL-15Rα (non-covalent) Fc fusion protein), and the benchmark compound (a N-terminal bivalent IL-15(non-covalent)/IL-15Rα Fc fusion protein comprising N72D mutation in IL-15).

Seven-week old female Balb/c mice were received from Charles River Laboratory and acclimated in house for at least 7 days before the study. Mice were given daily i.p. injections with equivalent molar doses of IL-15 compounds for 4 days. The treatments include vehicle, 0.03 mg/kg rhIL-15 (40 pmol/kg), 0.1 and 0.5mg/kg benchmark (40 and 200 pmol/kg), and 0.1 and 0.5 mg/kg P-0234 (40 and 200 pmol/kg). Each group had 5 mice. Body weight was recorded daily prior to and during the treatment. Mice were sacrificed one hour after the last injection and terminal blood was collected via cardiac puncture.

Heparinized whole blood and spleen were collected for NK cell phenotyping and Ki67 intracellular staining. After lysing red blood cells and blocking Fc-receptors with purified anti-mouse CD16/CD32 (1:50 dilution), mononuclear blood and splenic cells in a single-cell suspension were stained with NK cell surface markers, including anti-mouse CD3-FITC and anti-mouse CD49b-APC (1:50 dilution), for 30 minutes at room temperature in dark. For intracellular Ki67 staining, cell pellets were fully resuspended by 200 ul/well of 1x Foxp3 fixation/permeabilization working solution and incubated for 30-minutes at room temperature in dark. Cells were the washed with 200ul of 1x permeabilization buffer and Fc-receptors were blocked with purified anti-mouse CD16/CD32 (1:50 dilution). Cells were then stained with Ki67-PE in addition to anti-mouse CD3-FITC and anti-mouse CD49b-APC for NK cell population (1:50 dilution). After 30-minutes incubation, cells were collected and washed, resuspended in FACS buffer and ready for the flow cytometric analysis. Statistical analysis was conducted by one-way ANOVA with Tukey's post-hoc multiple comparison in GraphPad prism software.

FIG. 12 shows serum concentrations of IL-15 at 1 hour post the last injection of the IL-15 compounds. The IL-15 was measured by a commercial ELISA kit detecting IL-15 following the manufacturer's instruction (R&D systems; Cat# DY247). After 4 daily dosing of the compounds, the cumulative serum concentrations of IL-15 were the highest in mice treated with P-0234, intermediate in mice treated with the benchmark and the lowest in mice treated with rhlL-15 given at the equivalent molar doses (FIG. 12). Comparing the benchmark and P-0234 administered at 0,1 and 0.5 mg/kg, P-0234 consistently demonstrated 6-fold and 4-fold higher serum concentrations than the benchmark. The mean serum concentrations of IL-15 were 3.2 ± 0.6 (ng/ml) for rhlL-15 (0.03 mg/kg dosing), 13 ± 8 and 121 ± 36 (ng/ml) for the benchmark compound (0.1 and 0.5 mg/kg dosing, respectively), and 72 ± 14 and 443 ± 57 (ng/ml) for P-0234 (0.1 and 0.5 mg/kg dosing, respectively). The superior serum exposure suggests that P-0234 may exhibit a longer in vivo half-life and serum retention compared with the benchmark and rhlL-15.

Although there is no difference in gross body weights among the treatment groups, mice treated with the higher dose of the benchmark lost nearly 6% body weight within 4 days of treatment (FIG. 13A & 13B). The effect was statistically significant compared to baseline values at day 0 and to the vehicle group, suggesting a potential dose-limiting toxicity observed with the benchmark compound but not P-0234.

All tested IL-15 compounds increased the percentage of Ki67 positive NK cells in peripheral blood (FIG. 14A), suggesting an enhanced NK cell proliferation. However, a significant increase in the percentage of NK cell numbers in CD3 negative peripheral blood lymphocytes was only observed in mice treated with P-0234 at both tested dose levels (FIG. 14B). Although an increase in the percentage of NK cells was also observed in mice treated with rhIL-15 and the lower dose of benchmark, the effect failed to reach statistical significance (FIG. 14B). Interestingly, a decline in the NK cell numbers in peripheral blood was observed in mice treated with the higher dose benchmark (FIG. 14B). Such a reversed pharmacodynamic dose-response is suggestive of toxicity in agreement with the observed loss of body weight in this group.

The effect of IL-15 compounds on lymphocyte proliferation and expansion was also examined in the lymphoid organ the spleen. Similar to what was observed in peripheral blood, all IL-15 compounds increased the splenic Ki67 positive NK cells compared to vehicle (FIG. 15A). Only the low dose benchmark and P-0234 significantly increased the total number of NK cells in the spleen (FIG. 15B). Likewise, a reversed dose-response on splenic NK cell expansion was observed for the benchmark (FIG. 15B), and this was associated with high and persistent CD69 expression on splenic NK cells measured 4 days post termination (FIG. 15C). Data suggest that the benchmark compound may overstimulate NK cells and lead to cell exhaustion. With the IL-15 non-covalently bound to the IL-15Rα Fc fusion protein, the IL-15 may dissociate from the fusion complex of the benchmark compound and lead to lymphocyte overstimulation, cell exhaustion, toxicity and weight loss.

### Example 13

### Pharmacokinetic and pharmacodynamic effects of IL-15/IL-15Rα Fc fusion proteins in mice following a single injection

A dose-response study with P-0313, a C-terminal bivalent IL-15 S58DIL-15Rα (noncovalent) Fc fusion protein, was conducted in Balb/C mice following a single injection. The effect on peripheral blood lymphocyte proliferation and expansion was monitored over time. In addition, the pharmacokinetics and pharmacodynamics (PK/PD) of P-0313 were compared with those of the benchmark, a N-terminal divalent IL-15(noncovalent)/IL-15Ra Fc fusion protein comprising N72D mutation in IL-15, following a single injection.

Seven-week old female Balb/c mice were received from Charles River Laboratory and acclimated in house for at least 7 days before the study. Vehicle, benchmark (0.3 mg/kg) or P-0313 (0.01, 0.03, 0.1 and 0.3 mg/kg) was administered via intraperitoneal (i.p.) injection to mice at time 0. Blood samples were withdrawn at -24 hr. (pre-dose), and 1, 4, 24, 72, 120, and 192 hours post injection. Body weight was recorded daily prior to and during the treatment. Each group included 5 mice.

Heparin-treated whole blood was used for immune phenotyping and the volume was recorded. After red blood cell lysis using BD pharm lysis buffer, total viable mononuclear blood cells were counted by trypan blue dead cells exclusion and proceeded to Ki67 intracellular staining. Cell pellets were fully resuspended by 200 µl/well of 1x Foxp3 fixation/permeabilization working solution and incubated for 30 minutes at room temperature in the dark. After centrifugation, 200 µl of 1x permeabilization buffer was added to each well for another wash. After blocking Fc-receptors with purified anti-mouse CD16/CD32 (1:50 dilution), cells were stained with anti-mouse CD3-FITC, Ki67-PE, anti-mouse CD49b-APC and anti-mouse CD8-Percpcy5.5 (1:50 dilution). After a 30-minute incubation, cells were collected and washed, resuspended in FACS buffer and analyzed by flow cytometry. Statistical analysis was conducted by one-way ANOVA with Tukey's multiple comparison test in GraphPad prism software.

Serum concentrations of the compounds were measured using two different ELISA assays. An in-house ELISA assay was developed to measure the IL-15 and Fc complex, and the commercial ELISA assay measures IL-15 alone with both capture and detection antibodies reactive to human IL-15. For the in-house ELISA assay, maxisorp plates were coated with anti-IL-15 antibody (R&D systems MAB647) overnight at 4 °C. Plates were blocked with SuperBlock. Standard and samples at various dilutions were applied to the plates and incubated one hour at room temperature. Active compound was detected with anti-human IgG Fc-HRP and signal was developed using Ultra TMB Substrate Solution. Values were calculated using GraphPad Prism interpolation from non-linear regression curve fit.

Both compounds were detectable in the serum at the first 24 hours with comparable serum concentrations. The peak concentrations were observed 4 hours after i.p. administration. At 72 hours, only P-0313 remained measurable and the benchmark became undetectable in all three mice (FIGS. 16A-16B). Similar results were obtained using two different ELISA assays, confirming P-0313 has a superior pharmacokinetic profile than the benchmark. The result corroborated with the previous observation shown in Example 12 that P-0234, an IL-15/IL-15Rα (noncovalent) Fc fusion protein, also demonstrated higher serum exposure than the benchmark. These data strongly support that the IL-15/IL-15Rα (noncovalent) Fc fusion configuration is superior to that of IL-15 (noncovalent) /IL-15Ra Fc fusion protein in prolongation of IL-15 *in vivo* half-life.

No significant changes in body weight were observed in any treatment groups (FIG. 17).

Dose-dependent increases in Ki67 expression were observed in NK and CD8 T cells in mice treated with P-0313 (FIG. 18A & 18B). Effects peaked at 72 hours and persisted to 120 hours for the benchmark and further extended to 192 hours for P-0313 (FIG. 18A), suggesting P-0313 is longer-acting than the benchmark. In addition, P-0313 revealed a similar Ki67 induction at a dose 3-10 folds lower than that of the benchmark, suggesting P-0313 is more efficacious than the benchmark (FIG. 18A & 18B). A significant response on NK cells was observed at 10-fold lower dose than on CD8 T cells, suggesting NK cell is more sensitive than CD8 T cells to P-0313 treatment.

Consistent with the observed increase in cell proliferation marker Ki67, a dose-dependent expansion of NK and CD8+ T cells in blood was observed in P-0313 treated groups (FIG. 19A & 19B). The cell expansion was observed at 72 hours and peaked at 120 hours. P-0313 increased NK cells by 4-, 15-, 50- and 163-fold from the baselines at 0.01, 0.03, 0.1 and 0.3 mg/kg doses, respectively (FIG. 19A), and also increased CD8 T cells by 10- and 50-fold from the baselines at 0.1 and 0.3 mg/kg doses (FIG. 19B). In contrast, the benchmark at 0.3mg/kg dose only expanded peripheral NK cells by 28 folds and CD8 T cells by 12 folds (FIG. 19A & 19B).

In summary, P-0313 demonstrated a superior pharmacokinetic and pharmacodynamic on NK and CD8 T cell proliferation and expansion to the benchmark compound.

### Example 14

### Effect of IL-15/IL-15Rα Fcfusion proteins on inhibition of lung metastasis of mouse colon cancer

To investigate anti-metastatic efficacy and immunological responses of IL-15/IL-15Rα-Fc fusion proteins in tumor model, 1×10⁵ mouse colon carcinoma cells, CT26-WT (ATCC CRL-2638), were intravenously injected into female Balb/C mice (10-12 weeks-old). On the next day, 0.03 or 0.1 mg/kg of P-0313 or 0.3mg/kg of the Benchmark compound were given every five days by intravenous (i.v.) injection (Day 1, 6, 11 post-cell transplantation). Vehicle (PBS) was included as a negative control and every group contained 8 mice. On day 15, blood samples were collected for lymphocyte phenotyping and liver enzyme measurements. On day 16, all mice were sacrificed for tissue harvesting. Lungs were inflated by 15% india ink and destained in Fekete's solution (10% formaldehyde, 5% glacial acetic acid and 60% ethanol). Lung tumor nodules were counted for the entire lung under light microscope, and anti-metastatic effect were represented by different numbers of tumor nodules between treatment groups and vehicle control.

To study immunological response, mouse peripheral blood was collected on day 15 in heparin-treated tubes and the volume of blood used for the assay per mouse was recorded. After red blood cells were lysed by BD pharm lysis buffer, total viable mononuclear blood cells were counted by trypan blue dead cell exclusion and used for intracellular staining as described previously for immune cell phenotyping, and Ki67 proliferation analyses. After cell fixation, permeabilization and antibody staining, cells were collected and washed, resuspended in FACS buffer and analyzed by flow cytometry.

FIG. 20A shows representative photographs of lung from each group to illustrate lung nodules. Lung metastatic lesions were microscopically counted and quantified (FIG. 20B). As shown in FIG. 20, the benchmark molecule given at 0.3 mg/kg inhibited lung metastasis with 84% reduction in lung nodule counts, confirming that activating IL-15 pathway is effective to prevent the formation and growth of lung metastases. Strikingly, administration of IL-15/IL-15Rα-Fc complex P-0313 at a 3-fold lower dose (0.1 mg/kg) resulted in complete inhibition of the development of lung metastasis with zero nodule observed in all 8 mice treated (FIG. 20A & 20B). The superiority of P-0313 to the benchmark in suppressing the formation and growth of lung metastases is consistent with the enhanced pharmacokinetic and pharmacodynamic effects demonstrated previously (Example 12 & 13). With its IL-15 moiety covalently linked to the Fc domain, P-0313 demonstrated a marked improvement of IL-15 serum half-life over the benchmark, which contains an IL-15 that is non-covalently linked to Fc chain via IL-15RaSushi domain (Example 13). P-0313 at 0.03 mg/kg dosing also reduced lung metastasis with an inhibitory effect of ~35% (FIGS. 20A & 20B). This observation further emphasized P-0313 is efficacious at much lower doses than the benchmark and underscores the importance of biodistribution and bioavailability of the IL-15/IL-15Rα-Fc complex on its anti-cancer effect in vivo.

After three repeated Q5D dosing, the expansion of both NK and CD8+ T cells in peripheral blood remained significantly elevated in P-0313 treated mice (FIGS. 21A & 21B), which correlated with the significant increases in spleen weights of this treating groups (FIG. 22). In contrast, for the group treated with 0.3 mg/kg benchmark, only very modest expansion of CD8+ T cells was observed; no increase in the circulating NK cell numbers versus the control group was observed (FIG. 21A & 21B). However, the spleen weights were significantly increased in the benchmark treated group (FIG. 22). Data suggest after repeated dosing, the expanded lymphocytes may migrate to the lymphatic tissues for storage or cell exhaustion may also occur. As all the three treating groups showed anti-tumor effect, the data suggested that either CD8+ T cells or NK cells can be the effector subset involved in the antitumoral effects. However, complete eradication of lung metastasis seen in the group treated with 0.1 mg/kg of P-0313 suggested that an action engaging both NK cells and CD8+ T cells induced the strongest inhibition of tumor growth.

Liver weight and serum concentrations of alanine aminotransferase (ALT) & aspartate aminotransferase (AST) were measured to assess hepatotoxicity associated with the treatment. As depicted in FIGS. 23A-23C, there was no increase in either liver weights, ALT or AST levels for any treatment groups in comparison to the vehicle group. The data suggested that the strong antitumor effect of IL-15/IL-15Rα Fc fusion proteins was not associated with hepatotoxicity.

### Example 15

### Effect of an IL-15/IL-15Rα Fc fusion protein on established CT26 solid tumor growth in mice

To further investigate anti-tumor efficacy and immunological responses of IL-15/IL-15Ra Fc fusion protein in established tumor model, female Balb/C Mice (10-12 weeks old) were injected with 1 × 10⁵ CT26 cells subcutaneously in the right flank. On day 11, when the average tumor volume was ~70 mm³, mice were randomized into three groups (n = 10/group) and received intraperitoneal injection of vehicle (PBS), or P-0313 (0.1 mg/kg or 0.05 mg/kg) on the same day of randomization. One additional intraperitoneal injections of the respective testing agents were performed on day 16 (a total of 2 doses). Tumors were measured three times weekly using calipers, and the tumor volume was calculated as: volume = 0.5 × (width)² × (length). To study immunological response, non-terminal peripheral blood was collected in heparin-treated tubes on day 19. On day 21, all mice were sacrificed for tissue harvesting.

As shown in FIG. 24A, the PBS-treated mice rapidly developed large subcutaneous tumors. Treatment of mice with P-0313 at either 0.1 mg/kg or 0.05 mg/kg were approximately equipotent in delaying tumor growth (FIG. 24A). The tumor growth curve for each individual mouse was plotted for all three treatment groups (FIGS. 25A-25C). It is apparent that mice responded well to the treatment of P-0313 and showed delayed and synchronized inhibition in tumor growth particularly at the initial phase for two tested dose groups (FIGS. 25A-25C). On day 21 post-tumor inoculation, the mean tumor volume in the PBS-treated mice was- 820 mm³ versus 410 mm³ in mice treated with P-0313 at both doses (FIG. 25A ** *P* < 0.01; 1-way ANOVA with Tukey's post-test). It is worth noting that P-0313 at the higher dose (0.1 mg/kg) showed a greater decrease of tumor load than the lower-dosing group initially, but the difference tapered off as the treatment proceeded.

P-0313-treated mice demonstrated similar body weight gain as the PBS-treated mice over the course of 21 days study (FIG. 24B), suggesting P-0313 is well tolerated and not associated with significant toxicity at the two tested doses.

Next, we examined the effect of P-0313 on CD8 T cell and NK cell populations in the peripheral blood and spleen. Administration of P-0313 to tumor-bearing mice induced strong NK cell and CD8 T cell proliferation (FIGS. 26A & 26B) and dose-dependent expansion of NK and CD8 T cells (FIGS. 26C and 26D), a similar degree of immune cell responses as observed in non-tumor-bearing mice (Example 13). Among these two lymphocyte populations, the higher fold change was observed in NK cells (~100 fold for 0.1 mg/kg dosing group, and ~38 fold for 0.05 mg/kg dosing group). CD8+ T cells expanded by ~5.4 fold at 0.1 mg/kg dose, and ~2.7 fold for the 0.05 mg/kg dosing group.

P-0313 also enhanced the expansion of both NK and CD8+T cells in the spleens as those in peripheral blood (FIGS. 27A and 27B), but the magnitude/fold changes in the spleens were less profound. The higher fold change was observed in NK cells (~10 fold for 0.1 mg/kg dosing group, and ~8 fold for 0.05 mg/kg dosing group). CD8+ T cells expanded by ~2.7 fold at 0.1 mg/kg dose, and only marginally expanded for the 0.05 mg/kg dosing group.

Taken together, these data demonstrated that P-0313 treatment was capable of significantly delaying and inhibiting solid tumor growth and this anti-tumor effect was correlated with the proliferation and expansion of cytotoxic NK and CD8 T cells in tumor-bearing mice, which are consistent with the overall immunomodulatory property of IL-15. Since P-0313 bears an Fc region devoid of effector functions, the anti-tumor activity of P-0313 *in vivo* is not due to direct killing of tumor cells, but rather due to the robust activation of cytotoxic CD8+ T cells and NK cells for potent immune responses against tumor cells.

### Example 16

### Effect of an IL-15/IL-15Rα Fc fusion protein on non-established CT26 solid tumor growth in Balb/C mice

A similar study was conducted in non-established CT26 tumor model to confirm anti-tumor efficacy of P-0313. Three days after tumor cell subcutaneous engraftment with 1 × 10⁵ CT26 cells, mice received intraperitoneal injection of vehicle (PBS) or P-0313 (0.1 mg/kg) every 5 days for a total of 5 injections. Mice were terminated at Day 25 and tumors were measured two to three times a week

Similar as seen in established CT26 tumor model (Example 15), P-0313 demonstrated marked inhibition of tumor growth (FIG. 28A) and significant reduction in solid tumor mass (FIG. 28B). With 5 repeated doses, mice treated with P-0313 showed moderate increase in spleen weight (FIG. 29A) and no significant reduction in body weight gain (FIG. 29B), suggesting P-0313 is well tolerated.

Overall, these data further demonstrated that P-0313 is an effective immunotherapeutic against solid and liquid tumor as well as tumor cell metastasis with a well-tolerated safety profile.

### Example 17

### Construction of IL-15 antagonist variant/IL-15Ra Fc fusion proteins

As disclosed in Example 8 and associated Tables 5-7, a significant number of IL-15 variants, including amino acid substitution, insertion, and deletion resulted in reduced binding to IL-15Rβ in ELISA assay. In this example, additional IL-15Rβγ-disrupting substitutions were introduced to the IL-15 moiety in IL-15/IL-15Rα Fc fusion protein format aiming to achieve optimally attenuated potency. The present inventors reasoned that lower potency may prevent over-activation of the pathway and reduce unwanted target sink; and as such the IL-15 variant /IL-15Rα Fc fusion proteins with attenuated activity can result in potentially reduced toxicity and improved pharmacokinetics.

Three IL-15 residues at the interface with IL-15Rβ, S58, V63, and I68, and one γc-interacting residue Q108, were targeted for mutagenesis aiming for potency attenuation. Additionally, IL-15 variants with amino acid deletion of various lengths at the N-terminus was also constructed similarly.

Amino acid substitutions and deletions and their corresponding IL-15 variant/IL-15Rα Fc fusion proteins are listed in Table 8.

**Table 8**

| Exemplary IL-15 antagonist variant/IL-15Rα Fc fusion proteins. | | |
|---|---|---|
| (All have bivalent IL-15/IL-15Rα (no-covalent) Fc fusion format unless otherwise stated) | | |
| IL-15 amino acid substitutions/deletions | IL-15 variant SEQ ID NO: | Fusion protein ID |
| S58G | 66 | P-0739 |
| S58H | 67 | P-0740 |
| S58R | 68 | P-0741 |
| S58Q | 69 | P-0742 |
| S58I | 70 | P-0743 |
| S58P | 71 | P-0744 |
| V63A | 72 | P-0771 |
| V63F | 26 | P-0184¹ |
| V63K | 73 | P-0769 |
| V63R | 74 | P-0770 |
| I68F | 26 | P-0356 |
| I68D | 30 | P-0357 |
| I68H | 31 | P-0358 |
| I68K | 29 | P-0738 |
| I68Q | 75 | P-0736 |
| I68G | 76 | P-0737 |
| Q108S | 34 | P-0764² |
| Q108A | 32 | P-0793² |
| Q108M | 33 | P-0805² |
| Q108E | 77 | P-0797 |
| Q108K | 78 | P-0796² |
| 1-aa N-terminal deletion | 56 | P-0866 |
| 2-aa N-terminal deletion | 57 | P-0867 |
| 3-aa N-terminal deletion | 58 | P-0868 |
| 4-aa N-terminal deletion | 59 | P-0822 |

| | | |
|---|---|---|
| ¹Monovalent IL-15 (non-covalent)/IL-15Rα Fc fusion format ²Monovalent IL-15/IL-15Rα (no-covalent) Fc fusion format | | |

### Example 18

### Construction of IL-15 antagonist variant/IL-15Rα antibody fusion proteins

Various IL-15 antagonist variant /IL-15RαSushi antibody fusion proteins are prepared and evaluated. It is understood in the art that the use of recombinant antibody-cytokine fusion proteins (immunocytokines) may enhance the therapeutic index of cytokines by targeting them to the site of disease. In addition to tumor-targeting antibodies, the use of immune checkpoint blocking antibodies that bypass the immunosuppressive effects in the tumor microenvironment or immune-stimulatory antibodies to potentiate existing responses can also be used to construct antibody-cytokine fusion proteins. Immune checkpoint blocking antibody-cytokine fusion proteins are expected to further enhance the immune system's activity against tumors. The present inventors propose that the use of IL-15 variants having attenuated activity is likely to facilitate the establishment of stoichiometric balance between the cytokine and antibody arms. Further, cytokine activity attenuation is expected to minimize peripheral activation, mitigate antigen-sink, and promote tumor targeting via the antibody arm.

Following this concept, various IL-15/IL-15RaSushi-antibody fusion proteins were constructed. Exemplary antibodies used in the preparation of the fusion proteins including various PD-1 antagonist antibodies with sequences set forth in SEQ ID NOS: 111-120; the PD-L1 blocking antibody (SEQ ID NOS: 121-122); the anti-CTLA4 antibody (SEQ ID NOS: 123-124) the CD40 agonistic antibody (SEQ ID NOS: 125-126); the L19 antibody directed against the extra-domain of fibronectin (SEQ ID NOS: 127-128); rituximab directed against CD20 (SEQ ID NOS: 129-130); the antibody trastuzumab directed against Her-2 (SEQ ID NOS: 131-132); Cetuximab directed against EGFR (SEQ ID NOS: 133-134); an anti-FAP antibody for tumor-targeting and retention (SEQ ID NOS: 109-110).

To prepare IL-15/IL-15RaSushi -antibody fusion proteins, the CH1-CH2-CH3 (antibody residue 118-447 based on EU numbering) domain of the heavy chains of the above listed antibodies were replaced with an IgG1 sequence set forth in SEQ ID NO: 135 which comprises L234A, L235A, G237A mutations to abolish binding to FcγRs and C1q, but retain FcRn binding or PK. IL-15 variant peptide is fused via a peptide linker with sequences listed in Table 6 to the C-terminus of Fc domain. Alternatively, to express monovalent IL-15 variant, the CH1-CH2-CH3 domain of the heavy chains of the above listed antibodies were replaced with heterodimeric chains set forth in SEQ ID NO: 136-137. IL-15 variant peptide is fused via a peptide linker to the C-terminus of the knob-containing heterodimeric heavy chain engineered using the knob-into-holes technology. Half-life extension mutations, e.g., N434A, can be further incorporated into the homodimeric or heterodimer Fc chains. Yet another approach to generate bivalent IL-15/IL-15RaSushi-antibody fusion proteins is to fuse the IL-15 variant peptide via a peptide linker to the C-terminus of the light chain. In all the cases, IL-15Rα is co-expressed and non-covalently complexed with IL-15 moiety. Exemplary IL-15 variant/IL-15RaSushi- PD-1 antagonist antibody fusion proteins are listed in Table 9 and their cartoon diagrams are illustrated in FIG. 30.

**Table 9**

| Exemplary IL-15 antagonist variant/IL-15Rα-PD-1 antagonist antibody fusion proteins | | | |
|---|---|---|---|
| IL-15 amino acid substitutions/deletions | Antibody fusion SEQ ID NOS: | IL-15/IL-15Rα valency | Protein ID |
| Q108S | 138 + 114 + 5 | Dimer | P-0820 |
| Q108S | 139 + 144 + 114 + 5 | Monomer | P-0801 |
| Q108A | 140 + 144 + 114 + 5 | Monomer | P-0821 |
| I68G | 142 + 144 + 114 + 5 | Monomer | P-0856 |
| V63A/I68G | 143 + 144 + 114 + 5 | Monomer | P-0857 |
| V63A/I68G | 145 + 114 + 5 | Dimer | P-0858 |
| V63A/I68H | 141 + 114 + 5 | Dimer | P-0870 |
| 2-aa N-terminal deletion | 146 + 114 + 5 | Dimer | P-0886 |
| 4-aa N-terminal deletion | 147 + 114 + 5 | Dimer | P-0887 |
| 2-aa N-terminal deletion + V63A | 148 + 114 + 5 | Dimer | P-0888 |

Gene synthesis, expression vector construction, and protein production, purification, and characterization were conducted following the same procedures detailed in Example 1. All IL-15 variant/IL-15Rα-PD-1 antagonist antibody fusion proteins expressed with decent productivity comparable to parent antibody with low aggregation propensity (data not shown).

### Example 19

### IL-15 variant /IL-15Rα fusion proteins demonstrate various levels of potency modulation in ex vivo functional activity

Exemplary IL-15 variant/IL-15Rα Fc fusion proteins (monomer or dimer) were screened for their ability to stimulate cell proliferation by measuring Ki67 expression in CD8 T cells. Human PBMC were treated with increasing doses of IL-15 fusion molecules and Ki67 expression was determined by intracellular FACS analysis gated on CD8+ T cell population as previously described. P-0313, a bivalent IL-15/IL-15Rα sushi (non-covalent) Fc fusion containing S58D substitution with enhanced binding and functional activity, was used as the positive control as it has been extensively characterized.

As illustrated in FIG. 31A, replacing IL-15 residue S58 with amino acids of various sizes, solubility, and ionization properties only marginally modulated the potency in stimulating CD8 T cell proliferation. Table 10A summarizes the EC₅₀ values of the exemplary variants in stimulating CD8 T cell proliferation. Compared to P-0313, all variants display slightly reduced potency; reduction ranges from 1.7-fold to 7-fold.

**Table 10A**

| Impact of amino acid changes at S58 on CD8 T cell proliferation potency | | | |
|---|---|---|---|
| Protein ID | Substitution | EC50 (pM) | Fold change vs. P-0313 |
| P-0313 | S58D | 20 | 1 |
| P-0739 | S58G | 41.5 | 2.1 |
| P-0740 | S58H | 63.3 | 3.2 |
| P-0741 | S58R | 95.8 | 4.8 |
| P-0742 | S58Q | 139 | 7 |
| P-0743 | S58I | 78.6 | 3.9 |
| P-0744 | S58P | 34.3 | 1.7 |

Likewise, exemplary IL-15 variant/IL-15Rα Fc fusion comprising substitutions at position V63 only resulted in moderate attenuation of CD8 T cell attenuation as illustrated in FIG. 31B. EC₅₀ value of the variants in stimulating CD8 T cell proliferation and fold reduction in comparison to P-0313 are summarized in Table 10B.

**Table 10B**

| Impact of amino acid changes at V63 on CD8 T cell proliferation potency | | | |
|---|---|---|---|
| Protein ID | Substitution | EC50 (pM) | Fold change vs. P-0313 |
| P-0313 | S58D | 32 | 1 |
| P-0739 | V63K | 359.8 | 11.3 |
| P-0740 | V63R | 184.4 | 5.8 |
| P-0741 | V63A | 131.7 | 4.2 |

In contrast to residues S58 and V63, amino acid substitutions at position I68, which forms Van der Waals interaction with multiple IL-15Rβ residues, yielded variants with a wide spectrum of potency in proliferating CD8 T cells with EC₅₀ ranging from 0.4 nM to ~300 nM, an extent of 700-fold difference. Compared to P-0313, the levels of activity attenuation span from 10-fold to ~7000-fold. The data are illustrated in FIG. 31C and summarized in Table 10C.

**Table 10C**

| Impact of amino acid changes at !68 on CD8 T cell proliferation potency | | | |
|---|---|---|---|
| Protein ID | Substitution | EC50 (pM) | Fold change vs. P-0313 |
| P-0313 | S58D | 44 | 1 |
| P-0358 | I68H | 419 | 9.5 |
| P-0356 | I68F | 1251 | 29 |
| P-0736 | I68Q | 4473 | 102 |
| P-0737 | I68G | 14466 | 330 |
| P-0738 | I68K | 24842 | 564 |
| P-0357 | I68D | -296000 | -6727 |

Yet another residue targeted for attenuate IL-15 functional activity is Q108, a critical hotspot residue that interacts with multiple residues in γc. Both exemplary IL-15 Q108 variant/IL-15Rα Fc fusion proteins, P-0763 and P-0793 harboring Q108S and Q108A mutations, respectively, displayed significantly reduction (>1000-fold) in CD8 T cell proliferation (FIG. 31D and Table 10D)

**Table 10D**

| Impact of amino acid changes at Q108 on CD8 T cell proliferation potency | | | |
|---|---|---|---|
| Protein ID | Substitution | EC50 (pM) | Fold change vs. P-0313 |
| P-0313 | S58D | 32 | 1 |
| P-0764 | Q108S | 62051 | 1956 |
| P-0793 | Q108A | 68817 | 2169 |

In addition to substituting IL-15 residues at the interface with the IL-15Rβ and/or γc receptor subunits to achieve desired activity attenuation, N-terminal deletion offers another avenue to fulfill the same goal. The N-terminus of IL-15 is part of the alpha helix, which contains important residues, e.g. Asp8 and Lys10, that are interacting with IL-15Rβ and γc. Bivalent IL-15 variant/IL-15Ra (non-covalent) Fc fusion proteins, P-0866, P-0867, P-0868, and P-822, comprising 1, 2, 3, and 4 amino acid deletion at the IL-15 N-terminus, respectively, were assayed in human PBMC assay. As illustrated in FIG. 32, 1-amino acid deletion did not impact the potency of P-0866 in stimulating CD8 T cell proliferation when compared to P-0234, its wild-type IL-15 counterpart. In contrast, 2-amino acid deletion in P-0867 resulted in about 100-fold potency reduction (EC₅₀ of 24.8 nM vs 289 pM for P-0234). One additional amino acid deletion in P-0868 deletion did not further reduce the functional. However, one extra amino acid deletion or a total of four amino acid deletion essentially abrogated the biological activity of the fusion protein P-0822.

Further, combination of IL-15 amino acid substitutions disrupting IL-15Rβ and/or γc interactions were deemed to result in various levels of potency attenuation. Exemplary combination mutants incorporating V63A and one of the preferred mutations at !68 described in Table 8, including V68H, I68Q, and I68G, were constructed and assessed for CD8 T cell proliferation in human PBMC. Combination of IL-15Rβ-disrupting substitutions resulted in incremental attenuation of CD8 T cell proliferation potency (Table 11). Compared to P-0313, there was a 4.2-fold potency reduction due to the V63A substitution, and V63A consistently resulted in 2.1-fold, 4.3-fold, and 3.8-fold attenuation in CD8 T cell proliferation potency in the mutational context of I68H, I68Q, and I68G, respectively. The data are also illustrated in FIG 33A. Likewise, adding V63A amino acid substitution in the context of N-terminal amino acid deletion also resulted in a 3-fold attenuation in CD8 T cell proliferation potency, which was exemplified by P-0886 and P-0888 and illustrated in FIG. 33B. Both compounds are IL-15 variant/IL-15Ra PD-1 antibody fusion protein comprising two-amino acid deletion at the N-terminus of IL-15. P-0888 contains one additional V63A mutation.

**Table 11**

| Impact of exemplary combinations of IL-15Rβ-disrupting amino acid substitutions on CD8 T cell proliferation potency | | | | | |
|---|---|---|---|---|---|
| Substitutions | SEQ ID NO: | Fusion protein ID | EC50 (pM) | Fold change vs P-0313 | Impact of V63A (fold change) |
| S58D | 24 | P-0313 | 31.7 | 1 | N/A |
| V63A | 72 | P-0771 | 132 | 4.2 | 4.2 |
| I68H | 31 | P-0358 | 683 | 21.5 | N/A |
| V63A/I68H | 79 | P-0773 | 1418 | 45 | 2.1 |
| I68Q | 75 | P-0736 | 3614 | 114 | N/A |
| V63A/I68Q | 80 | P-0772 | 15379 | 485 | 4.3 |
| I68G | 76 | P-0737 | 12783 | 403 | N/A |
| V63A/I68G | 81 | P-0768 | 48015 | 1513 | 3.8 |

As can be appreciated by skilled artisan, additional mutants with various levels of potency attenuation can be obtained by combining the preferred mutations described in Table 8, e.g., V63K/Q108M, I68H/Q108K. Any further combination mutants come with the spirit and scope of the present invention.

Representative IL-15 variant /IL-15RαSushi Fc fusion proteins, P-0736, P-0772, P-0737, P-768, P-0793, and P-0764, with CD8 T cell Ki67 EC₅₀ ranging from 3.6 nM to 69 nM were further tested for their ability to stimulate NK cell proliferation by measuring Ki67 expression as previously described. The data are illustrated in FIGS. 34A and 34B and EC₅₀ values were summarized in Table 12.

**Table 12**

| CD8 T and NK cell proliferation potency of representative IL-15 variant /IL-15RαSushi fusion proteins | | | | | |
|---|---|---|---|---|---|
| Protein ID | Substitution | CD8 T cells | | NK cells | |
| | | EC50 (pM) | Fold change vs P-0313 | EC50 (pM) | Fold change vs P-0313 |
| P-0313 | S58D | 31.7 | 1 | 13 | 1 |
| P-0736 | I68Q | 3614 | 114 | 416 | 32 |
| P-0772 | V63A/I68Q | 15379 | 485 | 1110 | 85 |
| P-0737 | I68G | 12783 | 403 | 2064 | 159 |
| P-0768 | V63A/I68G | 48015 | 1515 | 5852 | 450 |
| P-0793 | Q108A | 68817 | 2171 | 4125 | 317 |
| P-0764 | Q108S | 62051 | 1957 | 9746 | 750 |

Similarly, as observed in CD8 T cells, the representative IL-15 variant /IL-15RαSushi Fc fusion proteins also demonstrated a wide potency range in stimulating NK cells proliferation with EC₅₀ ranging from 0.42 nM to 9.7 nM. Compared to P-0313, the potency of each molecule was pronouncedly attenuated; the level of potency reduction spans from 100-fold to 2000-fold for CD8 T cells and ranges from 30-fold to 750-fold for NK cells. While each molecule followed the similar potency ranking in proliferating CD8 T cells, all compounds are more responsive to NK cells.

Finally, it is clearly illustrated in FIG. 35 that the IL-15/IL-15Rα complex in either Fc fusion format or antibody fusion format demonstrated identical biological activity. Both P-0773 and P-0870 in FIG. 35A comprise V63A/I68H amino acid substitution and are IL-15/IL-15Rα Fc fusion and antibody fusion, respectively. P-0867 and P-0886 in FIG. 35B are a similar pair comprising 2-amino acid deletion at the N-terminus of IL-15.

In summary and importantly, introduction of IL-15Rβγc-disrupting amino acid substitutions or deletions individually or in combination into the IL-15 moiety resulted in IL-15 variants with a wide spectrum of potency in stimulating cytotoxic lymphocytes, represented by CD8 T cells and NK cells. The level of activity attenuation by different mutations or mutation combinations was the same in Fc fusion and antibody fusion proteins.

All of the articles and methods disclosed and claimed herein can be made and executed without undue experimentation in light of the present disclosure. While the articles and methods of this disclosure have been described in terms of preferred embodiments, it will be apparent to those of skill in the art that variations may be applied to the articles and methods without departing from the spirit and scope of the disclosure. All such variations and equivalents apparent to those skilled in the art, whether now existing or later developed, are deemed to be within the spirit and scope of the disclosure as defined by the appended claims. All patents, patent applications, and publications mentioned in the specification are indicative of the levels of those of ordinary skill in the art to which the disclosure pertains. All patents, patent applications, and publications are herein incorporated by reference in their entirety for all purposes and to the same extent as if each individual publication was specifically and individually indicated to be incorporated by reference in its entirety for any and all purposes. The disclosure illustratively described herein suitably may be practiced in the absence of any element(s) not specifically disclosed herein. Thus, it should be understood that although the present disclosure has been specifically disclosed by preferred embodiments and optional features, modification and variation of the concepts herein disclosed may be resorted to by those skilled in the art, and that such modifications and variations are considered to be within the scope of this disclosure as defined by the appended claims.

### Sequence Listings

The amino acid sequences listed in the accompanying sequence listing are shown using standard letter abbreviations for amino acids, as defined in 37 C.F.R. 1.822.
SEQ ID NO: 1 is a human IL-15 precursor amino acid sequence.
SEQ ID NO: 2 is a human IL-15 mature form amino acid sequence.
SEQ ID NO: 3 is a human IL-15Rα amino acid sequence.
SEQ ID NO: 4 is a human IL-15R*α*, extracellular domain amino acid sequence.
SEQ ID NO: 5 is a human IL-15R*α*, sushi domain+ amino acid sequence.
SEQ ID NO: 6 is a human IgG1-Fc amino acid sequence.
SEQ ID NO: 7 is a Knob-Fc amino acid sequence.
SEQ ID NO: 8 is a Hole-Fc amino acid sequence.
SEQ ID NOS: 9-12 are the amino acid sequences of various peptide linkers.
SEQ ID NO: 13 is the amino acid sequence of a Hole-Fc-Linker 1-IL-15 chain.
SEQ ID NO: 14 is the amino acid sequence of a Knob-Fc -Linker 1-IL-15R*α*-Sushi+ chain.
SEQ ID NO: 15 is the amino acid sequence of an IL-15-Linker 4-Hole-Fc chain.
SEQ ID NO: 16 is the amino acid sequence of an IL-15R*α*-Sushi+-Linker 4-Knob-Fc chain.
SEQ ID NO: 17 is the amino acid sequence of a Knob-Fc-linker 2-IL-15R*α*-Sushi+ chain.
SEQ ID NO: 18 is the amino acid sequence of a Hole-Fc-Linker 2-IL-15 chain.
SEQ ID NO: 19 is the amino acid sequence of an IL-15-Linker 3-Hole-Fc chain.
SEQ ID NO: 20 is the amino acid sequence of a Fc-Linker 3-IL-15 chain.
SEQ ID NO: 21 is the amino acid sequence of an IL-15-Linker 3-Fc chain.
SEQ ID NO: 22 is the amino acid sequence of a Knob-Fc-Linker2-IL-15R*α*-Sushi+ chain.
SEQ ID NO: 23 is the amino acid sequence of a Fc-Linker2-IL-15R*α*-Sushi+ chain.
SEQ ID NOS: 24-45 are the amino acid sequences of various IL-15 variant polypeptides.
SEQ ID NO: 46 is the amino acid sequence of a Fc-Linker 3-IL-15 S58D chain.
SEQ ID NO: 47 is the amino acid sequence of a peptide linker.
SEQ ID NO: 48 is the amino acid sequence of a Hole-Fc-Linker 3-IL-15-S58D chain
SEQ ID NO: 49 is the amino acid sequence of an IL-15-S58D-Linker 3-Hole-Fc chain.
SEQ ID NO: 50 is the amino acid sequence of an IL-15-S58D-Linker 3-Fc chain.
SEQ ID NO: 51 is the amino acid sequence of an IL-15R*α*-Sushi-Linker 2-Knob-Fc chain.
SEQ ID NO: 52 is the amino acid sequence of an IL-15R*α*-Sushi-Linker 2-Fc chain.
SEQ ID NO: 53 is the amino acid sequence of a Hole-Fc-Linker 1-IL-15-S58D chain.
SEQ ID NO: 54 is the amino acid sequence of a Hole-Fc-Linker 3-IL-15 chain
SEQ ID NO: 55 is the amino acid sequence of a Knob-Fc-Linker 1-IL-15 chain.
SEQ ID NOS: 56-63 are the amino acid sequences of various IL-15 variant polypeptides comprising N-terminal deletions
SEQ ID NOS 64 and 65 are the amino acid sequences of the two polypeptide chains of the benchmark.
SEQ ID NOS: 66-81 are the amino acid sequences of various IL-15 variant polypeptides.
SEQ ID NOS: 82-104 are the amino acid sequences of various IL-15 variant Fc fusion constructs.
SEQ ID NOS: 105-106 are human IgG1-Fc amino acid sequences with reduced/abolished effector function and extended half-life sequence.
SEQ ID NO: 107 is a Knob-Fc with extended half-life amino acid sequence.
SEQ ID NO: 108 is a Hole-Fc with extended half-life amino acid sequence.
SEQ ID NOS: 109-110 are the amino acid sequences of the heavy chain and light chain of a humanized anti-FAP antibody.
SEQ ID NOS: 111-112 are the amino acid sequences of the heavy chain and light chain of a humanized PD-1 antagonist antibody.
SEQ ID NOS: 113-114 are the amino acid sequences of the heavy chain and light chain of a human PD-1 antagonist antibody.
SEQ ID NOS: 115-116 are the amino acid sequences of the heavy chain and light chain of a PD-1 antagonist antibody.
SEQ ID NOS: 117-118 are the amino acid sequences of the heavy chain and light chain of a PD-1 antagonist antibody.
SEQ ID NOS: 119-120 are the amino acid sequences of the heavy chain and light chain of a PD-1 antagonist antibody.
SEQ ID NOS: 121-122 are the amino acid sequences of the heavy chain and light chain of a PD-L1 antagonist antibody.
SEQ ID NOS: 123-124 are the amino acid sequences of the heavy chain and light chain of a CTLA-4 antagonist antibody.
SEQ ID NOS: 125-126 are the amino acid sequences of the heavy chain and light chain of a CD40 agonist antibody.
SEQ ID NOS: 127-128 are the amino acid sequences of the heavy chain and light chain of an anti-fibronectin antibody.
SEQ ID NOS: 129-130 are the amino acid sequences of the heavy chain and light chain of CD20 antagonist antibody.
SEQ ID NOS: 131-132 are the amino acid sequences of the heavy chain and light chain of a Her-2/neu antagonist antibody.
SEQ ID NOS: 133-134 are the amino acid sequences of the heavy chain and light chain of an EGFR antagonist antibody.
SEQ ID NO: 135 is the amino acid sequence of a human IgG1 CH1CH2CH3 domain sequence with reduced/abolished Fc effector function.
SEQ ID NO: 136 is the amino acid sequence of a human IgG1 CH1CH2CH3 domain knob chain sequence with reduced/abolished Fc effector function.
SEQ ID NO: 137 is the amino acid sequence of a human IgG1 CH1CH2CH3 domain hole chain sequence with reduced/abolished Fc effector function.
SEQ ID NOS: 138-148 are the amino acid sequences of various exemplary IL-5/IL-15RαSushi-antibody fusion proteins.
SEQ ID NOS: 149-152 are the amino acid sequences of various IL-15 deletion variant Fc fusion constructs.
SEQ ID NOS: 153-154 are the amino acid sequences of various peptide linkers.

### SEQUENCE LISTINGS

Human IL-15 precursor sequence 
MRISKPHLRSISIQCYLCLLLNSHFLTEAGIHVFILGCFSAGLPKTEANWVNVISDLKKIEDLIQSM
HIDATLYTESDVHPSCKVTAMKCFLLELQVISLESGDASIHDTVENLIILANNSLSSNGNVTESGC
KECEELEEKNIKEFLQSFVHIVQMFINTS (SEQ ID NO: 1)
Human IL-15 mature form sequence 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIVQMFINTS (SEQ ID NO: 2)
Human IL-15Rα sequence 
MAPRRARGCRTLGLPALLLLLLLRPPATRGITCPPPMSVEHADIWVKSYSLYSRERYICNSGFK
RKAGTSSLTECVLNKATNVAHWTTPSLKCIRDPALVHQRPAPPSTVTTAGVTPQPESLSPSGK
EPAASSPSSNNTAATTAAIVPGSQLMPSKSPSTGTTEISSHESSHGTPSQTTAKNWELTASASH
QPPGVYPQGHSDTTVAISTSTVLLCGLSAVSLLACYLKSRQTPPLASVE
MEAMEALPVTWGTSSRDEDLENCSHHL (SEQ ID NO: 3)
Human IL-15R*α*, extracellular domain 
ITCPPPMSVEHADIWVKSYSLYSRERYICNSGFKRKAGTSSLTECVLNKATNVAHWTTPSLKCI
RDPALVHQRPAPPSTVTTAGVTPQPESLSPSGKEPAASSPSSNNTAATTAAIVPGSQLMPSKS
PSTGTTEISSHESSHGTPSQTTAKNWELTASASHQPPGVYPQGHSDTT (SEQ ID NO: 4)
Human IL-15R*α*, sushi domain+ 
ITCPPPMSVEHADIWVKSYSLYSRERYICNSGFKRKAGTSSLTECVLNKATNVAHWTTPSLKCI
RDPALVHQRPAPP (SEQ ID NO: 5)
Human IgG1-Fc with reduced effector function 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPG (SEQ ID NO: 6)
Knob-Fc 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVCTLPPSREEMTKNQVSLWCLVKGFYPSDIAVEWESNGQPENNYKTTPPVLDSDGSFFLYS
KLTVDKSRWQQGNVFSCSVMHEALHNHYTQKSLSLSPG (SEQ ID NO: 7)
Hole-Fc 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPCREEMTKNQVSLSCAVKGFYPSDIAVEWESNGQPENNYKTTPPVLDSDGSFFLVSK
LTVDKSRWQQGNVFSCSVMHEALHNHYTQKSLSLSPG (SEQ ID NO: 8)
Peptide Linker Sequence
   EPKSSDKTHTSPPSP (SEQ ID NO: 9)
Peptide Linker Sequence
   GGGGSGGGGS (SEQ ID NO: 10)
Peptide Linker Sequence
   GGGGSGGGGSGGGGS (SEQ ID NO: 11)
Peptide Linker Sequence
   GSGS (SEQ ID NO: 12)
Hole-Fc-Linker 1-IL-15 chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPCREEMTKNQVSLSCAVKGFYPSDIAVEWESNGQPENNYKTTPPVLDSDGSFFLVSK
LTVDKSRWQQGNVFSCSVMHEALHNHYTQKSLSLSPGEPKSSDKTHTSPPSPNWVNVISDLK
KIEDLIQSMHIDATLYTESDVHPSCKVTAMKCFLLELQVISLESGDASIHDTVENLIILANNSLSSN
GNVTESGCKECEELEEKNIKEFLQSFVHIVQMFINTS (SEQ ID NO: 13)
Knob-Fc-! L-Linker1-IL-15R*α*-Sushi+ chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVCTLPPSREEMTKNQVSLWCLVKGFYPSDIAVEWESNGQPENNYKTTPPVLDSDGSFFLYS
KLTVDKSRWQQGNVFSCSVMHEALHNHYTQKSLSLSPGEPKSSDKTHTSPPSPITCPPPMSV
EHADIWVKSYSLYSRERYICNSGFKRKAGTSSLTECVLNKATNVAHWTTPSLKCIRDPALVHQR
PAPP (SEQ ID NO: 14)
IL-15-Linker 4-Hole-Fc chain 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIVQMFINTSGCPPCPAPEAAGAPSVFL
FPPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPCREEMTKNQVSLSCA
VKGFYPSDIAVEWESNGQPENNYKTTPPVLDSDGSFFLVSKLTVDKSRWQQGNVFSCSVMHE
ALHNHYTQKSLSLSPG (SEQ ID NO: 15)
IL-15R*α*-Sushi+-Linker 4-Knob-Fc chain 
ITCPPPMSVEHADIWVKSYSLYSRERYICNSGFKRKAGTSSLTECVLNKATNVAHWTTPSLKCI
RDPALVHQRPAPPGCPPCPAPEAAGAPSVFLFPPKPKDTLMISRTPEVTCVVVDVSHEDPEVK
 
FNWYVDGVEVHNAKTKPREEQYNSTYRVVSVLTVLHQDWLNGKEYKCKVSNKALPAPIEKTIS
KAKGQPREPQVCTLPPSREEMTKNQVSLWCLVKGFYPSDIAVEWESNGQPENNYKTTPPVLD
SDGSFFLYSKLTVDKSRWQQGNVFSCSVMHEALHNHYTQKSLSLSPG (SEQ ID NO: 16)
Knob-Fc-linker 2-IL-15R*α*-+ chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVCTLPPSREEMTKNQVSLWCLVKGFYPSDIAVEWESNGQPENNYKTTPPVLDSDGSFFLYS
KLTVDKSRWQQGNVFSCSVMHEALHNHYTQKSLSLSPGGGGGSGGGGSITCPPPMSVEHAD
IWVKSYSLYSRERYICNSGFKRKAGTSSLTECVLNKATNVAHWTTPSLKCIRDPALVHQRPAPP
(SEQ ID NO: 17)
Hole-Fc-Linker 2-IL-15 chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPCREEMTKNQVSLSCAVKGFYPSDIAVEWESNGQPENNYKTTPPVLDSDGSFFLVSK
LTVDKSRWQQGNVFSCSVMHEALHNHYTQKSLSLSPGGGGGSGGGGSNWVNVISDLKKIED
LIQSMHIDATLYTESDVHPSCKVTAMKCFLLELQVISLESGDASIHDTVENLIILANNSLSSNGNV
TESGCKECEELEEKNIKEFLQSFVHIVQMFINTS (SEQ ID NO: 18)
IL-15-Linker 3-Hole-Fc chain 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIVQMFINTSGGGGSGGGGSGGGGSC
PPCPAPEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLP
PCREEMTKNQVSLSCAVKGFYPSDIAVEWESNGQPENNYKTTPPVLDSDGSFFLVSKLTVDKS
RWQQGNVFSCSVMHEALHNHYTQKSLSLSPG (SEQ ID NO: 19)
Fc-Linker 3-IL-15 chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTVENLIILANNSLS
SNGNVTESGCKECEELEEKNIKEFLQSFVHIVQMFINTS (SEQ ID NO: 20)
IL-15-Linker 3-Fc chain 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIVQMFINTSGGGGSGGGGSGGGGSC
PPCPAPEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLP
PSRDELTKNQVSLTCAVKGFYPSDIAVEWESNGQPENNYKTTPPVLDSDGSFFLVSKLTVDKS
RWQQGNVFSCSVMHEALHNHYTQKSLSLSPG (SEQ ID NO: 21)
Knob-Fc-Linker2-IL-15R*α*-Sushi+ chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVCTLPPSREEMTKNQVSLWCLVKGFYPSDIAVEWESNGQPENNYKTTPPVLDSDGSFFLYS
KLTVDKSRWQQGNVFSCSVMHEALHNHYTQKSLSLSPGGGGGSGGGGSITCPPPMSVEHAD
IWVKSYSLYSRERYICNSGFKRKAGTSSLTECVLNKATNVAHWTTPSLKCIRDPALVHQRPAPP
(SEQ ID NO: 22)
Fc-Linker 2-IL-15R*α*-Sushi+ chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSITCPPPMSVEHADI
WVKSYSLYSRERYICNSGFKRKAGTSSLTECVLNKATNVAHWTTPSLKCIRDPALVHQRPAPP
(SEQ ID NO: 23)
Human IL-15 S58D Variant Polypeptide 
NWVNVISDLKKIEDLIQSMHIDATLYTESDVHPSCKVTAMKCFLLELQVISLESGDADIHDTVENL
IILANNSLSSNGNVTESGCKECEELEEKNIKEFLQSFVHIVQMFINTS (SEQ ID NO: 24)
Human IL-15 T62D Variant Polypeptide 
NWVNVISDLKKIEDLIQSMHIDATLYTESDVHPSCKVTAMKCFLLELQVISLESGDASIHDDVENL
IILANNSLSSNGNVTESGCKECEELEEKNIKEFLQSFVHIVQMFINTS (SEQ ID NO: 25)
Human IL-15 V63F Variant Polypeptide 
NWVNVISDLKKIEDLIQSMHIDATLYTESDVHPSCKVTAMKCFLLELQVISLESGDASIHDTFENLI
ILANNSLSSNGNVTESGCKECEELEEKNIKEFLQSFVHIVQMFINTS (SEQ ID NO: 26)
Human IL-15 I67V Variant Polypeptide 
NWVNVISDLKKIEDLIQSMHIDATLYTESDVHPSCKVTAMKCFLLELQVISLESGDASIHDTVENL
VILANNSLSSNGNVTESGCKECEELEEKNIKEFLQSFVHIVQMFINTS (SEQ ID NO: 27)
Human IL-15 I68F Variant Polypeptide 
NWVNVISDLKKIEDLIQSMHIDATLYTESDVHPSCKVTAMKCFLLELQVISLESGDASIHDTVENLI
FLANNSLSSNGNVTESGCKECEELEEKNIKEFLQSFVHIVQMFINTS (SEQ ID NO: 28)
Human IL-15 I68K Variant Polypeptide 
NWVNVISDLKKIEDLIQSMHIDATLYTESDVHPSCKVTAMKCFLLELQVISLESGDASIHDTVENLI
KLANNSLSSNGNVTESGCKECEELEEKNIKEFLQSFVHIVQMFINTS (SEQ ID NO: 29)
Human IL-15 I68D Variant Polypeptide 
NWVNVISDLKKIEDLIQSMHIDATLYTESDVHPSCKVTAMKCFLLELQVISLESGDASIHDTVENLI
DLANNSLSSNGNVTESGCKECEELEEKNIKEFLQSFVHIVQMFINTS (SEQ ID NO: 30)
Human IL-15 I68H Variant Polypeptide 
NWVNVISDLKKIEDLIQSMHIDATLYTESDVHPSCKVTAMKCFLLELQVISLESGDASIHDTVENLI
HLANNSLSSNGNVTESGCKECEELEEKNIKEFLQSFVHIVQMFINTS (SEQ ID NO: 31)
Human IL-15 Q108A Variant Polypeptide 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIVAMFINTS (SEQ ID NO: 32)
Human IL-15 Q108M Variant Polypeptide 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIVMMFINTS (SEQ ID NO: 33)
Human IL-15 Q108S Variant Polypeptide 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIVSMFINTS (SEQ ID NO: 34)
Human IL-15 Q108S/D30T Variant Polypeptide 
NWVNVISDLKKIEDLIQSMHIDATLYTESTVHPSCKVTAMKCFLLELQVISLESGDASIHDTVENLI
ILANNSLSSNGNVTESGCKECEELEEKNIKEFLQSFVHIVSMFINTS (SEQ ID NO: 35)
Human IL-15 Q108S/V31Y Variant Polypeptide 
NWVNVISDLKKIEDLIQSMHIDATLYTESDYHPSCKVTAMKCFLLELQVISLESGDASIHDTVENLI
ILANNSLSSNGNVTESGCKECEELEEKNIKEFLQSFVHIVSMFINTS (SEQ ID NO: 36)
Human IL-15 Q108S/H32E Variant Polypeptide 
NWVNVISDLKKIEDLIQSMHIDATLYTESDVEPSCKVTAMKCFLLELQVISLESGDASIHDTVENLI
ILANNSLSSNGNVTESGCKECEELEEKNIKEFLQSFVHIVSMFINTS (SEQ ID NO: 37)
Human IL-15 Q108S/D30T/V31Y/H32E Variant Polypeptide 
NWVNVISDLKKIEDLIQSMHIDATLYTESTYEPSCKVTAMKCFLLELQVISLESGDASIHDTVENLI
ILANNSLSSNGNVTESGCKECEELEEKNIKEFLQSFVHIVSMFINTS (SEQ ID NO: 38)
Human IL-15 deletion 111-114 Variant Polypeptide 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIVQMF (SEQ ID NO: 39)
Human IL-15 deletion 109-114 Variant Polypeptide 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIVQ (SEQ ID NO: 40)
Human IL-15 deletion 108-114 Variant Polypeptide 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIV (SEQ ID NO: 41)
Human IL-15 deletion 105-114 Variant Polypeptide 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFV (SEQ ID NO: 42)
Human IL-15 Insertion 'GS' after N95 Variant Polypeptide 
NWVNVISDLKKIEDLIQSMHIDATLYTESDVHPSCKVTAMKCFLLELQVISLESGDASIHDTVENLI
ILANNSLSSNGNVTESGCKECEELEEKNGSIKEFLQSFVHIVQMFINTS (SEQ ID NO: 43)
Human IL-15 Insertion `GGSGG' after N95 Variant Polypeptide 
NWVNVISDLKKIEDLIQSMHIDATLYTESDVHPSCKVTAMKCFLLELQVISLESGDASIHDTVENLI
ILANNSLSSNGNVTESGCKECEELEEKNGGSGGIKEFLQSFVHIVQMFINTS (SEQ ID NO: 44)
Human IL-15 Insertion `GSSGGSGGS' after N95 Variant Polypeptide 
NWVNVISDLKKIEDLIQSMHIDATLYTESDVHPSCKVTAMKCFLLELQVISLESGDASIHDTVENLI
ILANNSLSSNGNVTESGCKECEELEEKNGSSGSSGGSIKEFLQSFVHIVQMFINTS
(SEQ ID NO: 45)
Fc-Linker 3-IL-15 S58D chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
 
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDADIHDTVENLIILANNSLS
SNGNVTESGCKECEELEEKNIKEFLQSFVHIVQMFINTS (SEQ ID NO: 46)
Peptide Linker Sequence
   GGGGSGGGG (SEQ ID NO: 47)
Hole-Fc-Linker 3-IL-15 S58D chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPCREEMTKNQVSLSCAVKGFYPSDIAVEWESNGQPENNYKTTPPVLDSDGSFFLVSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDADIHDTVENLIILANNSLS
SNGNVTESGCKECEELEEKNIKEFLQSFVHIVQMFINTS (SEQ ID NO: 48)
IL-15 S58D-Linker 3-Hole-Fc chain 
NWVNVISDLKKIEDLIQSMHIDATLYTESDVHPSCKVTAMKCFLLELQVISLESGDADIHDTVENL
IILANNSLSSNGNVTESGCKECEELEEKNIKEFLQSFVHIVQMFINTSGGGGSGGGGSGGGGSC
PPCPAPEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLP
PCREEMTKNQVSLSCAVKGFYPSDIAVEWESNGQPENNYKTTPPVLDSDGSFFLVSKLTVDKS
RWQQGNVFSCSVMHEALHNHYTQKSLSLSPG (SEQ ID NO: 49)
IL-15 S58D-Linker 3-Fc chain 
NWVNVISDLKKIEDLIQSMHIDATLYTESDVHPSCKVTAMKCFLLELQVISLESGDADIHDTVENL
IILANNSLSSNGNVTESGCKECEELEEKNIKEFLQSFVHIVQMFINTSGGGGSGGGGSGGGGSC
PPCPAPEAAGAPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVYTLP
PSRDELTKNQVSLTCAVKGFYPSDIAVEWESNGQPENNYKTTPPVLDSDGSFFLVSKLTVDKS
RWQQGNVFSCSVMHEALHNHYTQKSLSLSPG (SEQ ID NO: 50)
IL-15R*α*-Sushi-Linker 2-Knob-Fc chain 
ITCPPPMSVEHADIWVKSYSLYSRERYICNSGFKRKAGTSSLTECVLNKATNVAHWTTPSLKCI
RDPALVHQRPAPPGGGGSGGGGSCPPCPAPEAAGAPSVFLFPPKPKDTLMISRTPEVTCVVV
DVSHEDPEVKFNWYVDGVEVHNAKTKPREEQYNSTYRVVSVLTVLHQDWLNGKEYKCKVSN
KALPAPIEKTISKAKGQPREPQVCTLPPSREEMTKNQVSLWCLVKGFYPSDIAVEWESNGQPE
NNYKTTPPVLDSDGSFFLYSKLTVDKSRWQQGNVFSCSVMHEALHNHYTQKSLSLSPG
(SEQ ID NO: 51)
IL-15R*α*-Sushi-Linker 2-Fc chain 
ITCPPPMSVEHADIWVKSYSLYSRERYICNSGFKRKAGTSSLTECVLNKATNVAHWTTPSLKCI
RDPALVHQRPAPPGGGGSGGGGSCPPCPAPEAAGAPSVFLFPPKPKDTLMISRTPEVTCVVV
DVSHEDPEVKFNWYVDGVEVHNAKTKPREEQYNSTYRVVSVLTVLHQDWLNGKEYKCKVSN
KALPAPIEKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSPG
(SEQ ID NO: 52)
Hole-Fc-Linker 1-IL-15-S58D chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPCREEMTKNQVSLSCAVKGFYPSDIAVEWESNGQPENNYKTTPPVLDSDGSFFLVSK
LTVDKSRWQQGNVFSCSVMHEALHNHYTQKSLSLSPGEPKSSDKTHTSPPSPNWVNVISDLK
KIEDLIQSMHIDATLYTESDVHPSCKVTAMKCFLLELQVISLESGDADIHDTVENLIILANNSLSSN
GNVTESGCKECEELEEKNIKEFLQSFVHIVQMFINTS (SEQ ID NO: 53)
Hole-Fc-Linker 3-IL-15 chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPCREEMTKNQVSLSCAVKGFYPSDIAVEWESNGQPENNYKTTPPVLDSDGSFFLVSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTVENLIILANNSLS
SNGNVTESGCKECEELEEKNIKEFLQSFVHIVQMFINTS (SEQ ID NO: 54)
Knob-Fc-Linker 1-IL-15 chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVCTLPPSREEMTKNQVSLWCLVKGFYPSDIAVEWESNGQPENNYKTTPPVLDSDGSFFLYS
KLTVDKSRWQQGNVFSCSVMHEALHNHYTQKSLSLSPGEPKSSDKTHTSPPSPNWVNVISDL
KKIEDLIQSMHIDATLYTESDVHPSCKVTAMKCFLLELQVISLESGDASIHDTVENLIILANNSLSS
NGNVTESGCKECEELEEKNIKEFLQSFVHIVQMFINTS (SEQ ID NO: 55)
Human IL-15 variant with 1 amino acid deletion at the N-terminus 
WVNVISDLKKIEDLIQSMHIDATLYTESDVHPSCKVTAMKCFLLELQVISLESGDASIHDTVENLII
LANNSLSSNGNVTESGCKECEELEEKNIKEFLQSFVHIVQMFINTS (SEQ ID NO: 56)
Human IL-15 variant with 2 amino acid deletion at the N-terminus 
VNVISDLKKIEDLIQSMHIDATLYTESDVHPSCKVTAMKCFLLELQVISLESGDASIHDTVENLIILA
NNSLSSNGNVTESGCKECEELEEKNIKEFLQSFVHIVQMFINTS (SEQ ID NO: 57)
Human IL-15 variant with 3 amino acid deletion at the N-terminus 
NVISDLKKIEDLIQSMHIDATLYTESDVHPSCKVTAMKCFLLELQVISLESGDASIHDTVENLIILAN
NSLSSNGNVTESGCKECEELEEKNIKEFLQSFVHIVQMFINTS (SEQ ID NO: 58)
Human IL-15 variant with 4 amino acid deletion at the N-terminus 
VISDLKKIEDLIQSMHIDATLYTESDVHPSCKVTAMKCFLLELQVISLESGDASIHDTVENLIILANN
SLSSNGNVTESGCKECEELEEKNIKEFLQSFVHIVQMFINTS (SEQ ID NO: 59)
Human IL-15 variant with 5 amino acid deletion at the N-terminus 
ISDLKKIEDLIQSMHIDATLYTESDVHPSCKVTAMKCFLLELQVISLESGDASIHDTVENLIILANNS
LSSNGNVTESGCKECEELEEKNIKEFLQSFVHIVQMFINTS (SEQ ID NO: 60)
Human IL-15 variant with 6 amino acid deletion at the N-terminus 
SDLKKIEDLIQSMHIDATLYTESDVHPSCKVTAMKCFLLELQVISLESGDASIHDTVENLIILANNS
LSSNGNVTESGCKECEELEEKNIKEFLQSFVHIVQMFINTS (SEQ ID NO: 61)
Human IL-15 V63A variant with 2 amino acid deletion at the N-terminus 
VNVISDLKKIEDLIQSMHIDATLYTESDVHPSCKVTAMKCFLLELQVISLESGDASIHDTAENLIILA
NNSLSSNGNVTESGCKECEELEEKNIKEFLQSFVHIVQMFINTS (SEQ ID NO: 62)
Human IL-15 I68H variant with 2 amino acid deletion at the N-terminus 
VNVISDLKKIEDLIQSMHIDATLYTESDVHPSCKVTAMKCFLLELQVISLESGDASIHDTVENLIHL
ANNSLSSNGNVTESGCKECEELEEKNIKEFLQSFVHIVQMFINTS (SEQ ID NO: 63)
Benchmark Chain 1 
ITCPPPMSVEHADIWVKSYSLYSRERYICNSGFKRKAGTSSLTECVLNKATNVAHWTTPSLKCI
REPKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVLDSDGS
FFLYSKLTVDKSRWQQGNVFSCSVMHEALHNHYTQKSLSLSPG (SEQ ID NO: 64)
Benchmark Chain 2 
NWVNVISDLKKIEDLIQSMHIDATLYTESDVHPSCKVTAMKCFLLELQVISLESGDASIHDTVENLI
ILANDSLSSNGNVTESGCKECEELEEKNIKEFLQSFVHIVQMFINTS (SEQ ID NO: 65)
Human IL-15 S58G Variant Polypeptide 
NWVNVISDLKKIEDLIQSMHIDATLYTESDVHPSCKVTAMKCFLLELQVISLESGDAGIHDTVENL
IILANNSLSSNGNVTESGCKECEELEEKNIKEFLQSFVHIVQMFINTS (SEQ ID NO: 66)
Human IL-15 S58H Variant Polypeptide 
NWVNVISDLKKIEDLIQSMHIDATLYTESDVHPSCKVTAMKCFLLELQVISLESGDAHIHDTVENL
IILANNSLSSNGNVTESGCKECEELEEKNIKEFLQSFVHIVQMFINTS (SEQ ID NO: 67)
Human IL-15 S58R Variant Polypeptide 
NWVNVISDLKKIEDLIQSMHIDATLYTESDVHPSCKVTAMKCFLLELQVISLESGDARIHDTVENL
IILANNSLSSNGNVTESGCKECEELEEKNIKEFLQSFVHIVQMFINTS (SEQ ID NO: 68)
Human IL-15 S58Q Variant Polypeptide 
NWVNVISDLKKIEDLIQSMHIDATLYTESDVHPSCKVTAMKCFLLELQVISLESGDAQIHDTVENL
IILANNSLSSNGNVTESGCKECEELEEKNIKEFLQSFVHIVQMFINTS (SEQ ID NO: 69)
Human IL-15 S58I Variant Polypeptide 
NWVNVISDLKKIEDLIQSMHIDATLYTESDVHPSCKVTAMKCFLLELQVISLESGDAIIHDTVENLII
LANNSLSSNGNVTESGCKECEELEEKNIKEFLQSFVHIVQMFINTS (SEQ ID NO: 70)
Human IL-15 S58P Variant Polypeptide 
NWVNVISDLKKIEDLIQSMHIDATLYTESDVHPSCKVTAMKCFLLELQVISLESGDAPIHDTVENLI
ILANNSLSSNGNVTESGCKECEELEEKNIKEFLQSFVHIVQMFINTS (SEQ ID NO: 71)
Human IL-15 V63A Variant Polypeptide 
NWVNVISDLKKIEDLIQSMHIDATLYTESDVHPSCKVTAMKCFLLELQVISLESGDASIHDTAENLI
ILANNSLSSNGNVTESGCKECEELEEKNIKEFLQSFVHIVQMFINTS (SEQ ID NO: 72)
Human IL-15 V63K Variant Polypeptide 
NWVNVISDLKKIEDLIQSMHIDATLYTESDVHPSCKVTAMKCFLLELQVISLESGDASIHDTKENLI
ILANNSLSSNGNVTESGCKECEELEEKNIKEFLQSFVHIVQMFINTS (SEQ ID NO: 73)
Human IL-15 V63R Variant Polypeptide 
NWVNVISDLKKIEDLIQSMHIDATLYTESDVHPSCKVTAMKCFLLELQVISLESGDASIHDTRENL
IILANNSLSSNGNVTESGCKECEELEEKNIKEFLQSFVHIVQMFINTS (SEQ ID NO: 74)
Human IL-15 I68Q Variant Polypeptide 
NWVNVISDLKKIEDLIQSMHIDATLYTESDVHPSCKVTAMKCFLLELQVISLESGDASIHDTVENLI
QLANNSLSSNGNVTESGCKECEELEEKNIKEFLQSFVHIVQMFINTS (SEQ ID NO: 75)
Human IL-15 I68G Variant Polypeptide 
NWVNVISDLKKIEDLIQSMHIDATLYTESDVHPSCKVTAMKCFLLELQVISLESGDASIHDTVENLI
GLANNSLSSNGNVTESGCKECEELEEKNIKEFLQSFVHIVQMFINTS (SEQ ID NO: 76)
Human IL-15 Q108E Variant Polypeptide 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIVEMFINTS (SEQ ID NO: 77)
Human IL-15 Q108K Variant Polypeptide 
NWVNVISDLKKIEDLIQSMHIDATLYTESDVHPSCKVTAMKCFLLELQVISLESGDASIHDTVENLI
ILANNSLSSNGNVTESGCKECEELEEKNIKEFLQSFVHIVKMFINTS (SEQ ID NO: 78)
Human IL-15 V63A/I68H Variant Polypeptide 
NWVNVISDLKKIEDLIQSMHIDATLYTESDVHPSCKVTAMKCFLLELQVISLESGDASIHDTAENLI
HLANNSLSSNGNVTESGCKECEELEEKNIKEFLQSFVHIVQMFINTS (SEQ ID NO: 79)
Human IL-15 V63A/I68Q Variant Polypeptide 
NWVNVISDLKKIEDLIQSMHIDATLYTESDVHPSCKVTAMKCFLLELQVISLESGDASIHDTAENLI
QLANNSLSSNGNVTESGCKECEELEEKNIKEFLQSFVHIVQMFINTS (SEQ ID NO: 80)
Human IL-15 V63A/I68G Variant Polypeptide 
NWVNVISDLKKIEDLIQSMHIDATLYTESDVHPSCKVTAMKCFLLELQVISLESGDASIHDTAENLI
GLANNSLSSNGNVTESGCKECEELEEKNIKEFLQSFVHIVQMFINTS (SEQ ID NO: 81)
Fc-Linker 3-IL-15 S58G chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDAGIHDTVENLIILANNSLS
SNGNVTESGCKECEELEEKNIKEFLQSFVHIVQMFINTS (SEQ ID NO: 82)
Fc-Linker 3-IL-15 S58H chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDAHIHDTVENLIILANNSLS
SNGNVTESGCKECEELEEKNIKEFLQSFVHIVQMFINTS (SEQ ID NO: 83)
Fc-Linker 3-IL-15 S58R chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDARIHDTVENLIILANNSLS
SNGNVTESGCKECEELEEKNIKEFLQSFVHIVQMFINTS (SEQ ID NO: 84)
Fc-Linker 3-IL-15 S58Q chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDAQIHDTVENLIILANNSLS
SNGNVTESGCKECEELEEKNIKEFLQSFVHIVQMFINTS (SEQ ID NO: 85)
Fc-Linker 3-IL-15 S58I chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDAIIHDTVENLIILANNSLSS
NGNVTESGCKECEELEEKNIKEFLQSFVHIVQMFINTS (SEQ ID NO: 86)
Fc-Linker 3-IL-15 S58P chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDAPIHDTVENLIILANNSLS
SNGNVTESGCKECEELEEKNIKEFLQSFVHIVQMFINTS (SEQ ID NO: 87)
Fc-Linker 3-IL-15 V63A chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTAENLIILANNSLS
SNGNVTESGCKECEELEEKNIKEFLQSFVHIVQMFINTS (SEQ ID NO: 88)
Fc-Linker 3-IL-15 V63K chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTKENLIILANNSLS
SNGNVTESGCKECEELEEKNIKEFLQSFVHIVQMFINTS (SEQ ID NO: 89)
Fc-Linker 3-IL-15 V63R chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTRENLIILANNSLS
SNGNVTESGCKECEELEEKNIKEFLQSFVHIVQMFINTS (SEQ ID NO: 90)
Fc-Linker 3-IL-15 I68F chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTVENLIFLANNSLS
SNGNVTESGCKECEELEEKNIKEFLQSFVHIVQMFINTS (SEQ ID NO: 91)
Fc-Linker 3-IL-15 I68D chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTVENLIDLANNSLS
SNGNVTESGCKECEELEEKNIKEFLQSFVHIVQMFINTS (SEQ ID NO: 92)
Fc-Linker 3-IL-15 I68H chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTVENLIHLANNSLS
SNGNVTESGCKECEELEEKNIKEFLQSFVHIVQMFINTS (SEQ ID NO: 93)
Fc-Linker 3-IL-15 I68K chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTVENLIKLANNSLS
SNGNVTESGCKECEELEEKNIKEFLQSFVHIVQMFINTS (SEQ ID NO: 94)
Fc-Linker 3-IL-15 I68Q chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTVENLIQLANNSLS
SNGNVTESGCKECEELEEKNIKEFLQSFVHIVQMFINTS (SEQ ID NO: 95)
Fc-Linker 3-IL-15 I68G chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTVENLIGLANNSLS
SNGNVTESGCKECEELEEKNIKEFLQSFVHIVQMFINTS (SEQ ID NO: 96)
Hole Fc-Linker 1-IL-15 Q108S chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPCREEMTKNQVSLSCAVKGFYPSDIAVEWESNGQPENNYKTTPPVLDSDGSFFLVSK
 
LTVDKSRWQQGNVFSCSVMHEALHNHYTQKSLSLSPGEPKSSDKTHTSPPSPNWVNVISDLK
KIEDLIQSMHIDATLYTESDVHPSCKVTAMKCFLLELQVISLESGDASIHDTVENLIILANNSLSSN
GNVTESGCKECEELEEKNIKEFLQSFVHIVSMFINTS (SEQ ID NO: 97)
Hole Fc-Linker 1-IL-15 Q108A chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPCREEMTKNQVSLSCAVKGFYPSDIAVEWESNGQPENNYKTTPPVLDSDGSFFLVSK
LTVDKSRWQQGNVFSCSVMHEALHNHYTQKSLSLSPGEPKSSDKTHTSPPSPNWVNVISDLK
KIEDLIQSMHIDATLYTESDVHPSCKVTAMKCFLLELQVISLESGDASIHDTVENLIILANNSLSSN
GNVTESGCKECEELEEKNIKEFLQSFVHIVAMFINTS (SEQ ID NO: 98)
Knob Fc-Linker 3-IL-15 Q108M chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVCTLPPSREEMTKNQVSLWCLVKGFYPSDIAVEWESNGQPENNYKTTPPVLDSDGSFFLYS
KLTVDKSRWQQGNVFSCSVMHEALHNHYTQKSLSLSPGGGGGSGGGGSGGGGSNWVNVIS
DLKKIEDLIQSMHIDATLYTESDVHPSCKVTAMKCFLLELQVISLESGDASIHDTVENLIILANNSL
SSNGNVTESGCKECEELEEKNIKEFLQSFVHIVMMFINTS (SEQ ID NO: 99)
Knob Fc-Linker 3-IL-15 Q108E chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVCTLPPSREEMTKNQVSLWCLVKGFYPSDIAVEWESNGQPENNYKTTPPVLDSDGSFFLYS
KLTVDKSRWQQGNVFSCSVMHEALHNHYTQKSLSLSPGGGGGSGGGGSGGGGSNWVNVIS
DLKKIEDLIQSMHIDATLYTESDVHPSCKVTAMKCFLLELQVISLESGDASIHDTVENLIILANNSL
SSNGNVTESGCKECEELEEKNIKEFLQSFVHIVEMFINTS (SEQ ID NO: 100)
Knob Fc-Linker 3-IL-15 Q108K chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVCTLPPSREEMTKNQVSLWCLVKGFYPSDIAVEWESNGQPENNYKTTPPVLDSDGSFFLYS
KLTVDKSRWQQGNVFSCSVMHEALHNHYTQKSLSLSPGGGGGSGGGGSGGGGSNWVNVIS
DLKKIEDLIQSMHIDATLYTESDVHPSCKVTAMKCFLLELQVISLESGDASIHDTVENLIILANNSL
SSNGNVTESGCKECEELEEKNIKEFLQSFVHIVKMFINTS (SEQ ID NO: 101)
Fc-Linker 3-IL-15 V63A/I68H chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTAENLIHLANNSLS
SNGNVTESGCKECEELEEKNIKEFLQSFVHIVQMFINTS (SEQ ID NO: 102)
Fc-Linker 3-IL-15 V63A/I68Q chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTAENLIQLANNSLS
SNGNVTESGCKECEELEEKNIKEFLQSFVHIVQMFINTS (SEQ ID NO: 103)
Fc-Linker 3-IL-15 V63A/I68G chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNWVNVISD
LKKIEDLIQSMHIDATLYTESDVHPSCKVTAMKCFLLELQVISLESGDASIHDTAENLIGLANNSLS
SNGNVTESGCKECEELEEKNIKEFLQSFVHIVQMFINTS (SEQ ID NO: 104)
Human IgG1-Fc with reduced/abolished effector function and extended half-life 
DKTHTCPPCPAPEAAGGPSVFLFPPKPKDTLYITREPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPG (SEQ ID NO: 105)
Human IgG1-Fc with reduced/abolished effector function and extended in vivo half-life 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHAHYTQKSLSLSPG (SEQ ID NO: 106)
Knob-Fc with extended in vivo half-life 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVCTLPPSREEMTKNQVSLWCLVKGFYPSDIAVEWESNGQPENNYKTTPPVLDSDGSFFLYS
KLTVDKSRWQQGNVFSCSVMHEALHAHYTQKSLSLSPG (SEQ ID NO: 107)
Hole-Fc with extended in vivo half-life 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPCREEMTKNQVSLSCAVKGFYPSDIAVEWESNGQPENNYKTTPPVLDSDGSFFLVSK
LTVDKSRWQQGNVFSCSVMHEALHAHYTQKSLSLSPG (SEQ ID NO: 108)
Humanized anti-FAP antibody heavy chain 
QVQLVQSGAEVKKPGASVKVSCKASGYTFTENIIHWVRQAPGQGLEWMGWFHPGSGSIKYA
QKFQGRVTMTADKSTSTVYMELSSLRSEDTAVYYCARHGGTGRGAMDYWGQGTLVTVSSAS
TKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPEAAGAPSVFLFPP
KPKDTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNVFSCSVMHEALHN
HYTQKSLSLSPG (SEQ ID NO: 109)
Humanized anti-FAP antibody kappa light chain 
DIQMTQSPSSLSASVGDRVTITCRASRSISTSAYSYMHWYQQKPGKAPKLLIYLASNLESGVPS
RFSGSGSGTDFTLTISSLQPEDFATYYCQHSRELPYTFGQGTKVEIKRTVAAPSVFIFPPSDEQL
KSGTASVVCLLNNFYPREAKVQWKVDNALQSGNSQESVTEQDSKDSTYSLSSTLTLSKADYEK
HKVYACEVTHQGLSSPVTKSFNRGEC (SEQ ID NO: 110)
Human PD-1 antagonist antibody heavy chain 
EVQLVQSGAEVKKPGASVKVSCKASGYRFTSYGISWVRQAPGQGLEWMGWISAYNGNTNYA
QKLQGRVTMTTDTSTNTAYMELRSLRSDDTAVYYCARDADYSSGSGYWGQGTLVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPEAAGAPSVFLFPPKP
KDTLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVMHEALHNHY
TQKSLSLSPG (SEQ ID NO: 111)
Human PD-1 antagonist antibody Lλ 
SYELTQPPSVSVSPGQTARITCSGDALPKQYAYWYQQKPGQAPVMVIYKDTERPSGIPERFSG
SSSGTKVTLTISGVQAEDEADYYCQSADNSITYRVFGGGTKVTVLGQPKAAPSVTLFPPSSEEL
QANKATLVCLISDFYPGAVTVAWKADSSPVKAGVETTTPSKQSNNKYAASSYLSLTPEQWKSH
RSYSCQVTHEGSTVEKTVAPTECS (SEQ ID NO: 112)
Humanized PD-1 antagonist antibody-HC 
EVQLVESGGGLVKPGGSLRLSCAASGFTFSSYDMSWVRQAPGKGLEWVATISGGGSYTYYP
DSVKGRFTISRDNAKNSLYLQMNSLRAEDTAVYYCASPDSSGVAYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPEAAGAPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALHNHYTQ
KSLSLSPG (SEQ ID NO: 113)
Humanized PD-1 antagonist antibody-Lκ 
DIVMTQSPLSLPVTPGEPASITCKASQDVETVVAWYLQKPGQSPRLLIYWASTRHTGVPDRFS
GSGSGTDFTLKISRVEAEDVGVYYCQQYSRYPWTFGQGTKLEIKRTVAAPSVFIFPPSDEQLKS
GTASVVCLLNNFYPREAKVQWKVDNALQSGNSQESVTEQDSKDSTYSLSSTLTLSKADYEKH
KVYACEVTHQGLSSPVTKSFNRGEC (SEQ ID NO: 114)
Humanized PD-1 antagonist antibody-HC 
QGQLVQSGAEVKKPGASVKVSCKASGYTFTDYEMHWVRQAPGQGLEWMGVIESETGGTAYN
QKFKGRAKITADKSTSTAYMELSSLRSEDTAVYYCTREGITTVATTYYWYFDVWGQGTTVTVS
SASTKGPSVFPLAPCSRSTSESTAALGCLVKDYFPEPVTVSWNSGALTSGVHTFPAVLQSSGL
YSLSSVVTVPSSSLGTKTYTCNVDHKPSNTKVDKRVESKYGPPCPPCPAPEFLGGPSVFLFPP
KPKDTLMISRTPEVTCVVVDVSQEDPEVQFNWYVDGVEVHNAKTKPREEQFNSTYRVVSVLTV
LHQDWLNGKEYKCKVSNKGLPSSIEKTISKAKGQPREPQVYTLPPSQEEMTKNQVSLTCLVKG
FYPSDIAVEWESNGQPENNYKTTPPVLDSDGSFFLYSRLTVDKSRWQEGNVFSCSVMHEALH
NHYTQKSLSLSLGK (SEQ ID NO: 115)
Humanized PD-1 antagonist antibody-Lκ 
DVVMTQSPLSLPVTLGQPASISCRSSQSIVHSNGNTYLEWYLQKPGQSPQLLIYKVSNRFSGVP
DRFSGSGSGTDFTLKISRVEAEDVGVYYCFQGSHVPLTFGQGTKLEIKRTVAAPSVFIFPPSDE
QLKSGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO: 116)
Humanized PD-1 antagonist antibody-HC 
QVQLVQSGVEVKKPGASVKVSCKASGYTFTNYYMYWVRQAPGQGLEWMGGINPSNGGTNF
NEKFKNRVTLTTDSSTTTAYMELKSLQFDDTAVYYCARRDYRFDMGFDYWGQGTTVTVSSAS
TKGPSVFPLAPCSRSTSESTAALGCLVKDYFPEPVTVSWNSGALTSGVHTFPAVLQSSGLYSL
 
SSVVTVPSSSLGTKTYTCNVDHKPSNTKVDKRVESKYGPPCPPCPAPEFLGGPSVFLFPPKPK
DTLMISRTPEVTCVVVDVSQEDPEVQFNWYVDGVEVHNAKTKPREEQFNSTYRVVSVLTVLH
QDWLNGKEYKCKVSNKGLPSSIEKTISKAKGQPREPQVYTLPPSQEEMTKNQVSLTCLVKGFY
PSDIAVEWESNGQPENNYKTTPPVLDSDGSFFLYSRLTVDKSRWQEGNVFSCSVMHEALHNH
YTQKSLSLSLGK (SEQ ID NO: 117)
Humanized PD-1 antagonist antibody- Lκ 
EIVLTQSPATLSLSPGERATLSCRASKGVSTSGYSYLHWYQQKPGQAPRLLIYLASYLESGVPA
RFSGSGSGTDFTLTISSLEPEDFAVYYCQHSRDLPLTFGGGTKVEIKRTVAAPSVFIFPPSDEQL
KSGTASVVCLLNNFYPREAKVQWKVDNALQSGNSQESVTEQDSKDSTYSLSSTLTLSKADYEK
HKVYACEVTHQGLSSPVTKSFNRGEC (SEQ ID NO: 118)
Human PD-1 antagonist antibody-HC 
QVQLVESGGGVVQPGRSLRLDCKASGITFSNSGMHWVRQAPGKGLEWVAVIWYDGSKRYYA
DSVKGRFTISRDNSKNTLFLQMNSLRAEDTAVYYCATNDDYWGQGTLVTVSSASTKGPSVFPL
APCSRSTSESTAALGCLVKDYFPEPVTVSWNSGALTSGVHTFPAVLQSSGLYSLSSVVTVPSS
SLGTKTYTCNVDHKPSNTKVDKRVESKYGPPCPPCPAPEFLGGPSVFLFPPKPKDTLMISRTP
EVTCVVVDVSQEDPEVQFNWYVDGVEVHNAKTKPREEQFNSTYRVVSVLTVLHQDWLNGKE
YKCKVSNKGLPSSIEKTISKAKGQPREPQVYTLPPSQEEMTKNQVSLTCLVKGFYPSDIAVEWE
SNGQPENNYKTTPPVLDSDGSFFLYSRLTVDKSRWQEGNVFSCSVMHEALHNHYTQKSLSLS
LGK (SEQ ID NO: 119)
Human PD-1 antagonist antibod-Lκ 
EIVLTQSPATLSLSPGERATLSCRASQSVSSYLAWYQQKPGQAPRLLIYDASNRATGIPARFSG
SGSGTDFTLTISSLEPEDFAVYYCQQSSNWPRTFGQGTKVEIKRTVAAPSVFIFPPSDEQLKSG
TASVVCLLNNFYPREAKVQWKVDNALQSGNSQESVTEQDSKDSTYSLSSTLTLSKADYEKHKV
YACEVTHQGLSSPVTKSFNRGEC (SEQ ID NO: 120)
Humanized PD-L1 antagonist antibody-HC 
EVQLVESGGGLVQPGGSLRLSCAASGFTFSDSWIHWVRQAPGKGLEWVAWISPYGGSTYYA
DSVKGRFTISADTSKNTAYLQMNSLRAEDTAVYYCARRHWPGGFDYWGQGTLVTVSSASTKG
PSVFPLAPSSKSTSGGTAALGCLVKDYFPEPVTVSWNSGALTSGVHTFPAVLQSSGLYSLSSV
VTVPSSSLGTQTYICNVNHKPSNTKVDKKVEPKSCDKTHTCPPCPAPELLGGPSVFLFPPKPKD
TLMISRTPEVTCVVVDVSHEDPEVKFNWYVDGVEVHNAKTKPREEQYASTYRVVSVLTVLHQD
WLNGKEYKCKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVMHEALHNHYT
QKSLSLSPGK (SEQ ID NO: 121)
Humanized PD-L1 antagonist antibody-Lκ 
DIQMTQSPSSLSASVGDRVTITCRASQDVSTAVAWYQQKPGKAPKLLIYSASFLYSGVPSRFS
GSGSGTDFTLTISSLQPEDFATYYCQQYLYHPATFGQGTKVEIKRTVAAPSVFIFPPSDEQLKS
GTASVVCLLNNFYPREAKVQWKVDNALQSGNSQESVTEQDSKDSTYSLSSTLTLSKADYEKH
KVYACEVTHQGLSSPVTKSFNRGEC (SEQ ID NO: 122)
Human CTLA-4 antagonist antibody-HC 
QVQLVESGGGVVQPGRSLRLSCAASGFTFSSYTMHWVRQAPGKGLEWVTFISYDGNNKYYA
DSVKGRFTISRDNSKNTLYLQMNSLRAEDTAIYYCARTGWLGPFDYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPELLGGPSVFLFPPKPKDT
LMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVKGFYPSD
 
IAVEWESNGQPENNYKTTPPVLDSDGSFFLYSKLTVDKSRWQQGNVFSCSVMHEALHNHYTQ
KSLSLSPGK (SEQ ID NO: 123)
Human CTLA-4 antagonist antibody-Lκ 
EIVLTQSPGTLSLSPGERATLSCRASQSVGSSYLAWYQQKPGQAPRLLIYGAFSRATGIPDRFS
GSGSGTDFTLTISRLEPEDFAVYYCQQYGSSPWTFGQGTKVEIKRTVAAPSVFIFPPSDEQLKS
GTASVVCLLNNFYPREAKVQWKVDNALQSGNSQESVTEQDSKDSTYSLSSTLTLSKADYEKH
KVYACEVTHQGLSSPVTKSFNRGEC (SEQ ID NO: 124)
Human CD40 agonist antibody-HC 
QVQLVQSGAEVKKPGASVKVSCKASGYTFTGYYMHWVRQAPGQGLEWMGWINPDSGGTNY
AQKFQGRVTMTRDTSISTAYMELNRLRSDDTAVYYCARDQPLGYCTNGVCSYFDYWGQGTLV
TVSSASTKGPSVFPLAPCSRSTSESTAALGCLVKDYFPEPVTVSWNSGALTSGVHTFPAVLQS
SGLYSLSSVVTVPSSNFGTQTYTCNVDHKPSNTKVDKTVERKCCVECPPCPAPPVAGPSVFLF
PPKPKDTLMISRTPEVTCVVVDVSHEDPEVQFNWYVDGVEVHNAKTKPREEQFNSTFRVVSVL
TVVHQDWLNGKEYKCKVSNKGLPAPIEKTISKTKGQPREPQVYTLPPSREEMTKNQVSLTCLV
KGFYPSDISVEWESNGQPENNYKTTPPMLDSDGSFFLYSKLTVDKSRWQQGNVFSCSVMHEA
LHNHYTQKSLSLSPGK (SEQ ID NO: 125)
Human CD40 agonist antibody-Lκ 
DIQMTQSPSSVSASVGDRVTITCRASQGIYSWLAWYQQKPGKAPNLLIYTASTLQSGVPSRFS
GSGSGTDFTLTISSLQPEDFATYYCQQANIFPLTFGGGTKVEIKRTVAAPSVFIFPPSDEQLKSG
TASVVCLLNNFYPREAKVQWKVDNALQSGNSQESVTEQDSKDSTYSLSSTLTLSKADYEKHKV
YACEVTHQGLSSPVTKSFNRGEC (SEQ ID NO: 126)
Humanized anti-fibronectin antibody-HC 
EVQLLESGGGLVQPGGSLRLSCAASGFTFSSFSMSWVRQAPGKGLEWVSSISGSSGTTYYAD
SVKGRFTISRDSKNTLYLQMNSLRAEDTAVYYCAKPFPYFDYWGQGTLVTVSSASTKGPSVFP
LAPSSKSTSGGTAALGCLVKDYFPEPVTVSWNSGALTSGVHTFPAVLQSSGLYSLSSVVTVPS
SSLGTQTYICNVNHKPSNTKVDKRVEPKSCDKTHTCPPCPAPELLGGPSVFLFPPKPKDTLMIS
RTPEVTCVVVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEALHNHYTQKSL
SLSPGK (SEQ ID NO: 127)
Humanized anti-fibronectin antibody-Lκ 
EIVLTQSPGTLSLSPGERATLSCRASQSVSSSFLAWYQQKPGQAPRLLIYYASSRATGIPDRFS
GSGSGTDFTLTISRLEPEDFAVYYCQQTGRIPPTFGQGTKVEIKRTVAAPSVFIFPPSDEQLKSG
TASVVCLLNNFYPREAKVQWKVDNALQSGNSQESVTEQDSKDSTYSLSSTLTLSKADYEKHKV
YACEVTHQGLSSPVTKSFNRGEC (SEQ ID NO: 128)
Chimeric anti-CD20 antibody-HC 
QVQLQQPGAELVKPGASVKMSCKASGYTFTSYNMHWVKQTPGRGLEWIGAIYPGNGDTSYN
QKFKGKATLTADKSSSTAYMQLSSLTSEDSAVYYCARSTYYGGDWYFNVWGAGTTVTVSAAS
TKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKAEPKSCDKTHTCPPCPAPELLGGPSVFLFPPK
PKDTLMISRTPEVTCVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNVFSCSVMHEALHN
HYTQKSLSLSPGK (SEQ ID NO: 129)
Chimeric anti-CD20 antibody-Lκ 
QIVLSQSPAILSASPGEKVTMTCRASSSVSYIHWFQQKPGSSPKPWIYATSNLASGVPVRFSGS
GSGTSYSLTISRVEAEDAATYYCQQWTSNPPTFGGGTKLEIKRTVAAPSVFIFPPSDEQLKSGT
ASVVCLLNNFYPREAKVQWKVDNALQSGNSQESVTEQDSKDSTYSLSSTLTLSKADYEKHKVY
ACEVTHQGLSSPVTKSFNRGEC (SEQ ID NO: 130)
Humanized anti-Her2 antibody-HC 
EVQLVESGGGLVQPGGSLRLSCAASGFNIKDTYIHWVRQAPGKGLEWVARIYPTNGYTRYADS
VKGRFTISADTSKNTAYLQMNSLRAEDTAVYYCSRWGGDGFYAMDYWGQGTLVTVSSASTK
GPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVMHEALHNHY
TQKSLSLSPGK (SEQ ID NO: 131)
Humanized anti-Her2 antibody-Lκ 
DIQMTQSPSSLSASVGDRVTITCRASQDVNTAVAWYQQKPGKAPKLLIYSASFLYSGVPSRFS
GSRSGTDFTLTISSLQPEDFATYYCQQHYTTPPTFGQGTKVEIKRTVAAPSVFIFPPSDEQLKS
GTASVVCLLNNFYPREAKVQWKVDNALQSGNSQESVTEQDSKDSTYSLSSTLTLSKADYEKH
KVYACEVTHQGLSSPVTKSFNRGEC (SEQ ID NO: 132)
Chimeric anti-EGFR antibody-HC 
QVQLKQSGPGLVQPSQSLSITCTVSGFSLTNYGVHWVRQSPGKGLEWLGVIWSGGNTDYNTP
FTSRLSINKDNSKSQVFFKMNSLQSNDTAIYYCARALTYYDYEFAYWGQGTLVTVSAASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALHNHYTQ
KSLSLSPGK (SEQ ID NO: 133)
Chimeric anti-EGFR antibody-Lκ 
DILLTQSPVILSVSPGERVSFSCRASQSIGTNIHWYQQRTNGSPRLLIKYASESISGIPSRFSGSG
SGTDFTLSINSVESEDIADYYCQQNNNWPTTFGAGTKLELKRTVAAPSVFIFPPSDEQLKSGTA
SVVCLLNNFYPREAKVQWKVDNALQSGNSQESVTEQDSKDSTYSLSSTLTLSKADYEKHKVYA
CEVTHQGLSSPVTKSFNRGEC (SEQ ID NO: 134)
Human IgG1 CH1-CH2-CH3 domain with reduced/abolished effector function 
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPEAAGAPSVFLFP
PKPKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSCSVMHEALH
NHYTQKSLSLSPG (SEQ ID NO: 135)
Human IgG1 CH1-CH2-CH3 domain with reduced/abolished effector function knob chain 
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPEAAGAPSVFLFP
PKPKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVCTLPPSREEMTKNQVSLWCLVK
 
GFYPSDIAVEWESNGQPENNYKTTPPVLDSDGSFFLYSKLTVDKSRWQQGNVFSCSVMHEAL
HNHYTQKSLSLSPG (SEQ ID NO: 136)
Human lgG1 CH1-CH2-CH3 domain with reduced/abolished effector function Hole chain 
ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPEAAGAPSVFLFP
PKPKDTLMISRTPEVTCVVVDVSHEDPEVKFNWYVDGVEVHNAKTKPREEQYNSTYRVVSVLT
VLHQDWLNGKEYKCKVSNKALPAPIEKTISKAKGQPREPQVYTLPPCREEMTKNQVSLSCAVK
GFYPSDIAVEWESNGQPENNYKTTPPVLDSDGSFFLVSKLTVDKSRWQQGNVFSCSVMHEAL
HNHYTQKSLSLSPG (SEQ ID NO: 137)
PD-1 antagonist antibody-HC-IL-15 Q108S chain 
EVQLVESGGGLVKPGGSLRLSCAASGFTFSSYDMSWVRQAPGKGLEWVATISGGGSYTYYP
DSVKGRFTISRDNAKNSLYLQMNSLRAEDTAVYYCASPDSSGVAYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPEAAGAPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALHNHYTQ
KSLSLSPGGGGGSGGGGSGGGGSNWVNVISDLKKIEDLIQSMHIDATLYTESDVHPSCKVTAM
KCFLLELQVISLESGDASIHDTVENLIILANNSLSSNGNVTESGCKECEELEEKNIKEFLQSFVHIV
SMFINTS (SEQ ID NO: 138)
PD-1 antagonist antibody-HC-IL-15 Q108S knob chain 
EVQLVESGGGLVKPGGSLRLSCAASGFTFSSYDMSWVRQAPGKGLEWVATISGGGSYTYYP
DSVKGRFTISRDNAKNSLYLQMNSLRAEDTAVYYCASPDSSGVAYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPEAAGAPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVCTLPPSREEMTKNQVSLWCLVKGFYPS
DIAVEWESNGQPENNYKTTPPVLDSDGSFFLYSKLTVDKSRWQQGNVFSCSVMHEALHNHYT
QKSLSLSPGGGGGSGGGGSGGGGSNWVNVISDLKKIEDLIQSMHIDATLYTESDVHPSCKVTA
MKCFLLELQVISLESGDASIHDTVENLIILANNSLSSNGNVTESGCKECEELEEKNIKEFLQSFVHI
VSMFINTS (SEQ ID NO: 139)
PD-1 antagonist antibody-HC-IL-15 Q108A knob chain 
EVQLVESGGGLVKPGGSLRLSCAASGFTFSSYDMSWVRQAPGKGLEWVATISGGGSYTYYP
DSVKGRFTISRDNAKNSLYLQMNSLRAEDTAVYYCASPDSSGVAYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPEAAGAPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVCTLPPSREEMTKNQVSLWCLVKGFYPS
DIAVEWESNGQPENNYKTTPPVLDSDGSFFLYSKLTVDKSRWQQGNVFSCSVMHEALHNHYT
QKSLSLSPGGGGGSGGGGSGGGGSNWVNVISDLKKIEDLIQSMHIDATLYTESDVHPSCKVTA
MKCFLLELQVISLESGDASIHDTVENLIILANNSLSSNGNVTESGCKECEELEEKNIKEFLQSFVHI
VAMFINTS (SEQ ID NO: 140)
PD-1 antagonist antibody HC-IL-15 V63A/I68H chain 
EVQLVESGGGLVKPGGSLRLSCAASGFTFSSYDMSWVRQAPGKGLEWVATISGGGSYTYYP
DSVKGRFTISRDNAKNSLYLQMNSLRAEDTAVYYCASPDSSGVAYWGQGTLVTVSSASTKGP
SVFPLAPSSKSTSGGTAALGCLVKDYFPEPVTVSWNSGALTSGVHTFPAVLQSSGLYSLSSVV
 
TVPSSSLGTQTYICNVNHKPSNTKVDKKVEPKSCDKTHTCPPCPAPEAAGAPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALHNHYTQ
KSLSLSPGGGGGSGGGGSGGGGSNWVNVISDLKKIEDLIQSMHIDATLYTESDVHPSCKVTAM
KCFLLELQVISLESGDASIHDTAENLIHLANNSLSSNGNVTESGCKECEELEEKNIKEFLQSFVHI
VQMFINTS (SEQ ID NO: 141)
PD-1 antagonist antibody-HC-IL-15 I68G knob chain 
EVQLVESGGGLVKPGGSLRLSCAASGFTFSSYDMSWVRQAPGKGLEWVATISGGGSYTYYP
DSVKGRFTISRDNAKNSLYLQMNSLRAEDTAVYYCASPDSSGVAYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPEAAGAPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVCTLPPSREEMTKNQVSLWCLVKGFYPS
DIAVEWESNGQPENNYKTTPPVLDSDGSFFLYSKLTVDKSRWQQGNVFSCSVMHEALHNHYT
QKSLSLSPGGGGGSGGGGSGGGGSNWVNVISDLKKIEDLIQSMHIDATLYTESDVHPSCKVTA
MKCFLLELQVISLESGDASIHDTVENLIGLANNSLSSNGNVTESGCKECEELEEKNIKEFLQSFV
HIVQMFINTS (SEQ ID NO: 142)
PD-1 antagonist antibody-HC-IL-15 V63A/I68G knob chain 
EVQLVESGGGLVKPGGSLRLSCAASGFTFSSYDMSWVRQAPGKGLEWVATISGGGSYTYYP
DSVKGRFTISRDNAKNSLYLQMNSLRAEDTAVYYCASPDSSGVAYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPEAAGAPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVCTLPPSREEMTKNQVSLWCLVKGFYPS
DIAVEWESNGQPENNYKTTPPVLDSDGSFFLYSKLTVDKSRWQQGNVFSCSVMHEALHNHYT
QKSLSLSPGGGGGSGGGGSGGGGSNWVNVISDLKKIEDLIQSMHIDATLYTESDVHPSCKVTA
MKCFLLELQVISLESGDASIHDTAENLIGLANNSLSSNGNVTESGCKECEELEEKNIKEFLQSFV
HIVQMFINTS (SEQ ID NO: 143)
PD-1 antagonist antibody-HC hole chain 
EVQLVESGGGLVKPGGSLRLSCAASGFTFSSYDMSWVRQAPGKGLEWVATISGGGSYTYYP
DSVKGRFTISRDNAKNSLYLQMNSLRAEDTAVYYCASPDSSGVAYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPEAAGAPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPCREEMTKNQVSLSCAVKGFYPS
DIAVEWESNGQPENNYKTTPPVLDSDGSFFLVSKLTVDKSRWQQGNVFSCSVMHEALHNHYT
QKSLSLSPG (SEQ ID NO: 144)
PD-1 antagonist antibody-HC-IL-15 V63A/I68G chain 
EVQLVESGGGLVKPGGSLRLSCAASGFTFSSYDMSWVRQAPGKGLEWVATISGGGSYTYYP
DSVKGRFTISRDNAKNSLYLQMNSLRAEDTAVYYCASPDSSGVAYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPEAAGAPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALHNHYTQ
KSLSLSPGGGGGSGGGGSGGGGSNWVNVISDLKKIEDLIQSMHIDATLYTESDVHPSCKVTAM
 
KCFLLELQVISLESGDASIHDTAENLIGLANNSLSSNGNVTESGCKECEELEEKNIKEFLQSFVHI
VQMFINTS (SEQ ID NO: 145)
PD-1 antagonist antibody-HC-IL-15 N-terminal 2-aa deletion chain 
EVQLVESGGGLVKPGGSLRLSCAASGFTFSSYDMSWVRQAPGKGLEWVATISGGGSYTYYP
DSVKGRFTISRDNAKNSLYLQMNSLRAEDTAVYYCASPDSSGVAYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPEAAGAPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALHNHYTQ
KSLSLSPGGGGGSGGGGSGGGGSVNVISDLKKIEDLIQSMHIDATLYTESDVHPSCKVTAMKC
FLLELQVISLESGDASIHDTVENLIILANNSLSSNGNVTESGCKECEELEEKNIKEFLQSFVHIVQ
MFINTS (SEQ ID NO: 146)
PD-1 antagonist antibody-HC-IL-15 N-terminal 4-aa deletion chain 
EVQLVESGGGLVKPGGSLRLSCAASGFTFSSYDMSWVRQAPGKGLEWVATISGGGSYTYYP
DSVKGRFTISRDNAKNSLYLQMNSLRAEDTAVYYCASPDSSGVAYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPEAAGAPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALHNHYTQ
KSLSLSPGGGGGSGGGGSGGGGSVISDLKKIEDLIQSMHIDATLYTESDVHPSCKVTAMKCFLL
ELQVISLESGDASIHDTVENLIILANNSLSSNGNVTESGCKECEELEEKNIKEFLQSFVHIVQMFIN
TS (SEQ ID NO: 147)
PD-1 antagonist antibody-HC-IL-15 V63A & N-terminal 2-aa deletion chain 
EVQLVESGGGLVKPGGSLRLSCAASGFTFSSYDMSWVRQAPGKGLEWVATISGGGSYTYYP
DSVKGRFTISRDNAKNSLYLQMNSLRAEDTAVYYCASPDSSGVAYWGQGTLVTVSSASTKGP
SVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPEAAGAPSVFLFPPKPKD
TLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLTCLVKGFYPSD
IAVEWESNGQPENNYKTTPPVLDSDGSFFLYSKLTVDKSRWQQGNVFSCSVMHEALHNHYTQ
KSLSLSPGGGGGSGGGGSGGGGSVNVISDLKKIEDLIQSMHIDATLYTESDVHPSCKVTAMKC
FLLELQVISLESGDASIHDTAENLIILANNSLSSNGNVTESGCKECEELEEKNIKEFLQSFVHIVQ
MFINTS (SEQ ID NO: 148)
Fc-Linker 3-IL-15 N-terminal 1-aa deletion chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSWVNVISDL
KKIEDLIQSMHIDATLYTESDVHPSCKVTAMKCFLLELQVISLESGDASIHDTVENLIILANNSLSS
NGNVTESGCKECEELEEKNIKEFLQSFVHIVQMFINTS (SEQ ID NO: 149)
Fc-Linker 3-IL-15 N-terminal 2-aa deletion chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
 
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSVNVISDLKK
IEDLIQSMHIDATLYTESDVHPSCKVTAMKCFLLELQVISLESGDASIHDTVENLIILANNSLSSNG
NVTESGCKECEELEEKNIKEFLQSFVHIVQMFINTS (SEQ ID NO: 150)
Fc-Linker 3-IL-15 N-terminal 3-aa deletion chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSNVISDLKKI
EDLIQSMHIDATLYTESDVHPSCKVTAMKCFLLELQVISLESGDASIHDTVENLIILANNSLSSNG
NVTESGCKECEELEEKNIKEFLQSFVHIVQMFINTS (SEQ ID NO: 151)
Fc-Linker 3-IL-15 N-terminal 4-aa deletion chain 
DKTHTCPPCPAPEAAGAPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGGGGGSGGGGSGGGGSVISDLKKIE
DLIQSMHIDATLYTESDVHPSCKVTAMKCFLLELQVISLESGDASIHDTVENLIILANNSLSSNGN
VTESGCKECEELEEKNIKEFLQSFVHIVQMFINTS (SEQ ID NO: 152)
Peptide Linker Sequence
   GGSGG (SEQ ID NO: 153)
Peptide Linker Sequence
   GSSGGSGGS (SEQ ID NO: 154)

### ADDITIONAL EMBODIMENTS

1. An isolated Interleukin-15 (IL-15) fusion protein complex comprising: (1) an IL-15 polypeptide (or variant thereof) linked to a heterologous protein; and (2) an IL-15 Receptor alpha ("IL-15Rα") domain noncovalently linked to the IL-15 polypeptide to form an IL-15/IL-15Rα-heterologous protein fusion protein, wherein the IL-15 variant polypeptide is selected from the group of polypeptides having the amino acid sequence set forth in SEQ ID NOS: 56-63 and 66-81.
2. The IL-15/IL-15Rα-heterologous protein fusion protein according to embodiment 1, wherein the IL-15 polypeptide is linked to the C-terminus of the heterologous protein.
3. The IL-15/IL-15Rα-heterologous protein fusion protein according to embodiment 1, wherein the IL-15 polypeptide is linked to the N-terminus of the heterologous protein.
4. An isolated IL-15/IL-15Rα-heterologous protein fusion protein comprising: (1) an IL-15Rα domain linked to a heterologous protein; and (2) an IL-15 polypeptide (or variant thereof) noncovalently linked to the IL-15Rα domain.
5. The IL-15/IL-15Rα-heterologous protein fusion protein according to embodiment 4, wherein the IL-15Rα domain is linked to the C-terminus of the heterologous protein.
6. The IL-15/IL-15Rα-heterologous protein fusion protein according to embodiment 4, wherein the IL-15Rα domain is linked to the N-terminus of the heterologous protein.
7. The IL-15/IL-15Rα-heterologous protein fusion protein according to any one of embodiments 1 to 6, wherein the IL-15Rα domain comprises the amino acid sequence set forth in SEQ ID NO: 4 or any functional fragment thereof.
8. The IL-15/IL-15Rα-heterologous protein fusion protein according to any one of embodiments 1 to 7, wherein the IL-15Rα domain is an IL-15 Receptor alpha Sushi ("IL-15RαSushi") domain, wherein the IL-15RαSushi domain comprises an amino acid sequence that is at least 90% homologous to the sequence set forth in SEQ ID NO: 5.
9. The IL-15/IL-15Rα-heterologous protein fusion protein according to embodiment 8, wherein the IL-15RαSushi domain comprises an amino acid sequence that is at least 90% homologous to the sequence set forth in SEQ ID NO: 5.
10. The IL-15/IL-15Rα-heterologous protein fusion protein complex according to any one of embodiments 1 to 9, wherein the heterologous protein is an Fc domain, and wherein each IL-15 polypeptide is covalently attached to an Fc domain by a peptide linker.
11. The IL-15/IL-15Rα-Fc fusion protein complex according to embodiment 10, wherein the peptide linker is selected from the group of sequences set forth in SEQ ID NOs: 9-12, SEQ ID NO: 47 and SEQ IS NOS: 153-154.
12. The IL-15/IL-15Rα-heterologous protein fusion protein according to any one of embodiments 1 to 11, wherein the heterologous protein is selected from the group consisting of an antibody, an antibody fragment, a ligand or its variant, a receptor or its variant capable of binding to a tumor associated antigen (TAA) or a tissue-specific antigen or target, a cell surface molecule or extracellular matrix protein, protease(s) and any post-translational modification residue(s).
13. The construct according to embodiment 12, wherein the antibody, or an antibody fragment is selected from the group consisting of: PD-1 antagonistic antibodies; PD-L1 antagonistic antibodies; CTLA-4 antagonistic antibodies; CD20 antagonistic antibodies; Her-2/neu antagonistic antibodies; EGFR antagonistic antibodies; FAP antagonistic antibodies; antiinflammatory antibodies against integrin α₄β₇; TNFα antagonistic antibodies; and agonistic CD40 antibodies.
14. The construct according to embodiment 13, wherein the antibody is an antagonistic Fibroblast Activation Protein (FAP) antibody or antibody fragment.
15. The construct according to embodiment 14, wherein the antibody is a humanized anti-FAP antibody comprising the heavy chain and light chain amino acid sequences set forth in SEQ ID NOS: 109 and 110.
16. The construct according to embodiment 13, wherein the D1 domain is an antibody or an antibody fragment to an immune checkpoint modulator.
17. The construct according to embodiment 16, wherein the antibody is an antagonistic Programmed Death-1 (PD-1) antibody or antibody fragment.
18. The construct according to embodiment 17, wherein the antibody is an antagonistic PD-1 antibody selected from the antibody comprising the heavy chain and light chain amino acid sequences set forth in SEQ ID NOS: 111 and 112; the heavy chain and light chain amino acid sequences set forth in SEQ ID NOS: 113 and 114; the heavy chain and light chain amino acid sequences set forth in SEQ ID NOS: 115 and 116; the heavy chain and light chain amino acid sequences set forth in SEQ ID NOS: 117 and 118; and the heavy chain and light chain amino acid sequences set forth in SEQ ID NOS: 119 and 120.
19. The IL-15/IL-15Rα-heterologous protein fusion protein according to any one of embodiments 1 to 11, wherein the heterologous protein is selected from the group consisting of a full-length null antibody or antibody fragment which provides for half-life extension.
20. The IL-15/IL-15Rα-heterologous protein fusion protein according to any one of embodiments 1 to 11, wherein the heterologous protein is selected from the group consisting of: a full-length IgG or bispecific diabody which provides an additive or synergistic effect with the IL-15/IL-15Rα complex.
21. An isolated IL-15 fusion protein complex comprising: (1) two IL-15 polypeptides (or variants thereof) linked to two heterologous proteins; and (2) two IL-15Rα domains noncovalently linked to each IL-15 polypeptide to form a dimeric IL-15/IL-15Rα-heterologous protein fusion protein complex, wherein the IL-15 variant polypeptide is selected from the group of polypeptides having the amino acid sequence set forth in SEQ ID NOS: 56-63 and 66-81.
22. The IL-15/IL-15Rα-heterologous protein fusion protein complex according to embodiment 21, wherein the two IL-15 polypeptides are linked to the C-terminus of the two heterologous proteins.
23. The IL-15/IL-15Rα-heterologous protein fusion protein complex according to embodiment 21, wherein the two IL-15 polypeptides are linked to the N-terminus of the two heterologous proteins.
24. The IL-15/IL-15Rα-heterologous protein fusion protein complex according to any one of embodiments 21 to 23, wherein the IL-15Rα domain comprises the amino acid sequence set forth in SEQ ID NO: 4 or any functional fragment thereof.
25. The IL-15/IL-15Rα-heterologous protein fusion protein complex according to any one of embodiments 21 to 23, wherein the IL-15Rα domain is an IL-15 Receptor alpha Sushi ("IL-15RαSushi") domain, wherein the IL-15RαSushi domain comprises an amino acid sequence that is at least 90% homologous to the sequence set forth in SEQ ID NO: 5.
26. The IL-15/IL-15Rα-heterologous protein fusion protein complex according to embodiment 25, wherein the IL-15RαSushi domain comprises the amino acid sequence set forth in SEQ ID NO: 5.
27. The IL-15/IL-15Rα-heterologous protein fusion protein complex according to any one of embodiments 21 to 26, wherein each IL-15 polypeptide is covalently attached to an heterologous protein domain by a peptide linker.
28. The IL-15/IL-15Rα-heterologous protein fusion protein complex according to embodiment 27, wherein the peptide linker is selected from the group of sequences set forth in SEQ ID NOs: 9-12, SEQ ID NO: 47, and SEQ ID NOS: 153-154.
29. An isolated IL-15 fusion protein complex comprising: (1) two IL-15Rα domains linked to two heterologous protein domains; and (2) two IL-15 polypeptides (or variants thereof) noncovalently linked to the IL-15Rα domain to form a dimeric IL-15/IL-15Rα-heterologous protein fusion protein complex, wherein the IL-15 variant polypeptide is selected from the group of polypeptides having the amino acid sequence set forth in SEQ ID NOS: 56-63 and 66-81.
30. The IL-15/IL-15Rα-heterologous protein fusion protein complex according to embodiment 29, wherein the two IL-15Rα domains are linked to the C-terminus of the two heterologous protein domains.
31. The IL-15/IL-15Rα-heterologous protein fusion protein complex according to embodiment 29, wherein the IL-15Rα domain is linked to the N-terminus of the two heterologous protein domains.
32. The IL-15/IL-15Rα-heterologous protein fusion protein complex according to any one of embodiments 29 to 31, wherein the IL-15Rα domain comprises the amino acid sequence set forth in SEQ ID NO: 4 or any functional fragment thereof.
33. The IL-15/IL-15Rα-heterologous protein fusion protein complex according to any one of embodiments 29 to 31, wherein the IL-15Rα domain is an IL-15 receptor alpha Sushi ("IL-15RαSushi") domain, wherein the IL-15RαSushi domain comprises an amino acid sequence that is at least 90% homologous to the sequence set forth in SEQ ID NO: 5.
34. The IL-15/IL-15Rα-heterologous protein fusion protein complex according to embodiment 33, wherein the IL-15RαSushi domain comprises the amino acid sequence set forth in SEQ ID NO: 5.
35. The IL-15/IL-15Rα-heterologous protein fusion protein complex according to any one of embodiments 29 to 34, wherein each IL-15 polypeptide is covalently attached to an heterologous protein domain by a peptide linker.
36. The IL-15/IL-15Rα-heterologous protein fusion protein complex according to embodiment 35, wherein the peptide linker is selected from the group of sequences set forth in SEQ ID NOs: 9-12, SEQ ID NO: 47, and SEQ ID NOS: 153-154.
37. A pharmaceutical composition comprising an IL-15/IL-15Rα-heterologous protein fusion protein according to any one of embodiments 1 to 36 in admixture with a pharmaceutically acceptable carrier.
38. A method of treating cancer or cancer metastasis in a subject, comprising administering to the subject a therapeutically effective amount of a pharmaceutical composition according to embodiment 37.
39. The method according to embodiment 38, wherein the cancer is selected from the group consisting of pancreatic cancer, gastric cancer, ovarian cancer, colorectal cancer, melanoma, leukemia, myelodysplastic syndrome, lung cancer, liver cancer, breast cancer, prostate cancer, brain cancer, bladder cancer, head-neck cancer, or rhabdomyosarcoma.
40. The method according to any one of embodiments 38 to 39, wherein the method further comprises a second therapy capable of treating cancer or cancer metastasis; wherein the combination therapy provides increased effector cell killing of tumor cells.
41. A method of treating a viral infection in a subject, comprising administering to the subject a therapeutically effective amount of the pharmaceutical composition according to embodiment 37.
42. A method to expand and renew NK cells and T cells in vitro and in vivo and in combination with adoptive transfer NK and T cell therapy or CAR-NK and CAR-T therapy to sustain cell survival and half-life, including but not limited to cell therapies using dendric cells, tumor infiltrating lymphocytes (TILs), NK cells, TCR-T cells; CAR-NK cells, iPS induced-NK cells, iPS induced TCR-T cells, iPS induced CAR-T cells or iPS induced CAR-NK cells; comprising administering to the subject a therapeutically effective amount of the pharmaceutical composition according to embodiment 37.
43. A method of making an IL-15/IL-15Rα fusion protein complex, the method comprising: a) co-expressing a IL-15Rα domain and an IL-15 polypeptide attached an heterologous protein domain in a host cell; b) culturing the host cell in media under conditions sufficient to express the IL-15Rα domain and the IL-15-heterologous protein fusion protein; and c) purifying the IL-15/IL-15Rα-heterologous protein fusion protein complex from the host cell or media.
44. A method of improving IL-15 fusion protein expression by co-expression with IL-15Ra domain leading to reduced aggregation and increased expression.
45. A nucleic acid molecule encoding an IL-15-heterologous protein domain fusion protein according to any one of embodiments 1 to 36.
46. A nucleic acid molecule encoding an IL-15Rα domain according to any one of embodiments 1 to 36.
47. An expression vector comprising the nucleic acid molecule of embodiment 45.
48. An expression vector comprising the nucleic acid molecule of embodiment 46.
49. An expression vector of claim 66 further comprising the nucleic acid molecule of embodiment 46.
50. A host cell comprising one or more of the expression vectors according to any one of embodiments 47 to 49.
51. A method of producing an IL-15/IL-15Rα fusion protein complex according to any one of embodiments 1 to 56 comprising culturing the host cell of claim 1 to 49 under conditions promoting the expression of the IL-15/IL-15Rα fusion protein complex and recovering the IL-15/IL-15Rα fusion protein complex.
52. An isolated IL-15/IL-15Rα fusion protein complex produced by the method of embodiment 51.

## Claims

1. An isolated Interleukin-15 (IL-15) fusion protein complex comprising: (1) an IL-15 variant polypeptide linked to an Fc domain; and (2) an IL-15 Receptor alpha ("IL-15Rα") domain noncovalently linked to the IL-15 variant polypeptide to form an Fc-IL-15/IL-15Rα fusion protein complex,
wherein the IL-15 variant polypeptide is linked to the C-terminus of the Fc domain, or
wherein the IL-15 variant polypeptide is linked to the N-terminus of the Fc domain, and
wherein the IL-15 variant polypeptide comprises the amino acid sequence of SEQ ID NO: 2 having one or more of amino acid residues position V63, I68 or Q108 substituted with another amino acid, wherein the amino acid substitution is selected from the group consisting of: the substitution of V63A, V63K, V63R, I68Q, I68G, I68H, Q108E and Q108K, or any combination of these substitutions, and
wherein the IL-15Rα domain is an IL-15 Receptor alpha Sushi ("IL-15RαSushi") domain which comprises an amino acid sequence that is at least 90% homologous to the sequence set forth in SEQ ID NO: 5.

2. The Fc-IL-15/IL-15Rα fusion protein complex according to claim 1, wherein the Fc domain is an Fc domain having an amino acid sequence selected from the group consisting of the amino acid sequences set forth in SEQ ID NO: 6, SEQ ID NO: 7 and SEQ ID NO: 8, and wherein each IL-15 polypeptide is covalently attached to an Fc domain by a peptide linker.

3. The Fc-IL-15/IL-15Rα fusion protein complex according to any one of claims 1 and 2, wherein said IL-15 variant polypeptide comprises two amino acid substitutions at amino acid residues position V63 and I68 of SEQ ID NO: 2.

4. The Fc-IL-15/IL-15Rα fusion protein complex according to any preceding claim, wherein the complex is in either dimeric or monomeric format.

5. An isolated Interleukin-15 (IL-15) fusion protein complex comprising: (1) an IL-15 variant polypeptide linked to an antibody (Ab) or antibody fragment; and (2) an IL-15 Receptor alpha ("IL-15Rα") domain noncovalently linked to the IL-15 variant polypeptide to form an Ab-IL-15/IL-15Rα fusion protein complex, wherein the IL-15 variant polypeptide is linked to the C-terminus of the antibody, or
wherein the IL-15 variant polypeptide is linked to the N-terminus of the antibody, and
wherein the IL-15 variant polypeptide comprises the amino acid sequence of SEQ ID NO: 2 having one or more of amino acid residues position V63, I68 or Q108 substituted with another amino acid, wherein the amino acid substitution is selected from the group consisting of: the substitution of V63A, V63K, V63R, I68Q, I68G, I68H, Q108E and Q108K, or any combination of these substitutions, and
wherein the antibody is an antagonistic Programmed Death-1 (PD-1) antibody or antibody fragment, and
wherein the IL-15Rα domain is an IL-15 Receptor alpha Sushi ("IL-15RαSushi") domain which comprises an amino acid sequence that is at least 90% homologous to the sequence set forth in SEQ ID NO: 5.

6. The Ab-IL-15/IL-15Rα fusion protein complex according to claim 5, wherein the antibody is an antagonistic PD-1 antibody selected from the antibody comprising the heavy chain and light chain amino acid sequences set forth in SEQ ID NOS: 111 and 112; the heavy chain and light chain amino acid sequences set forth in SEQ ID NOS: 113 and 114; the heavy chain and light chain amino acid sequences set forth in SEQ ID NOS: 115 and 116; the heavy chain and light chain amino acid sequences set forth in SEQ ID NOS: 117 and 118; and the heavy chain and light chain amino acid sequences set forth in SEQ ID NOS: 119 and 120.

7. The Ab-IL-15/IL-15Rα fusion protein complex according to any one of claims 5 and 6, wherein said IL-15 variant polypeptide comprises two amino acid substitutions at amino acid residues position V63 and I68 of SEQ ID NO: 2.

8. The Ab-IL-15/IL-15Rα fusion protein complex according to any one of claims 5 to 7, wherein the fusion protein complex comprises SEQ ID NO: 114, SEQ ID NO: 141 and SEQ ID NO: 5.

9. The Ab-IL-15/IL-15Rα fusion protein complex according to any one of claims 5 to 8, wherein the complex is in either dimeric or monomeric format.

10. A pharmaceutical composition comprising an Fc-IL-15/IL-15Rα fusion protein complex according to any one of claims 1 to 4 in admixture with a pharmaceutically acceptable carrier.

11. A pharmaceutical composition comprising an Ab-IL-15/IL-15Rα fusion protein complex according to any one of claims 5 - 9 in admixture with a pharmaceutically acceptable carrier.
